# EUROPEAN PATENT SPECIFICATION

(11) **EP 1 994 158 B1**
(45) Date of publication and mention of the grant of the patent: **11.05.2011**
(21) Application number: 07717738.4
(22) Date of filing: 11.01.2007
(51) Int. Cl.: C12N 15/82, C12N 15/54, C12N 9/10, A01H 5/10

(54) **EPSP SYNTHASE ENZYME DOMAINS CONFERRING GLYPHOSATE RESISTANCE**
EPSP-SYNTHASE-ENZYMDOMÄNEN FÜR GLYPHOSATRESISTENZ
DOMAINES D'ENZYMES EPSP SYNTHÉTASES CONFÉRANT UNE RÉSISTANCE AU GLYPHOSATE

(30) Priority: 12.01.2006 US 758320 P
(43) Date of publication of application: 26.11.2008
(60) Divisional application: 11158889.3
(73) Proprietor: Athenix Corporation, Research Triangle Park, NC 27709 (US)
(72) Inventor: CARR, Brian, Raleigh, NC 27613 (US); HAMMER, Philip, E., Cary, NC 27513 (US); HINSON, Todd, K., Rougemont, NC 27572 (US); VANDE BERG, Brian, Durham, NC 27713 (US)
(74) Representative: Power, David
(86) International application number: PCT/US2007/060396
(87) International publication number: WO 2007/082269

(56) References cited:
- EP-A1- 1 510 581
- WO-A2-2004/056179
- US-A- 5 627 061
- US-A1- 2003 233 675
- DATABASE EMBL [Online] 4 April 2002 (2002-04-04), "Methanopyrus kandleri AV19 section 55 of 157 of the complete genome." XP002455066 retrieved from EBI accession no. EMBL:AE010356 Database accession no. AE010356

## Description

### FIELD OF THE INVENTION

This invention relates to plant molecular biology, particularly to a method of producing genetically transformed plants, as well as a method for selectively controlling weeds in a field.

### BACKGROUND OF THE INVENTION

N-phosphonomethylglycine, commonly referred to as glyphosate, is an important agronomic chemical. Glyphosate inhibits the enzyme that converts phosphoenolpyruvic acid (PEP) and 3-phosphoshikimic acid (S3P) to 5-enolpyruvyl-3-phosphoshikimic acid. Inhibition of this enzyme (5-enolpyruvylshikimate-3-phosphate synthase; referred to herein as "EPSP synthase", or "EPSPS") kills plant cells by shutting down the shikimate pathway, thereby inhibiting aromatic amino acid biosynthesis.

Since glyphosate-class herbicides inhibit aromatic amino acid biosynthesis, they not only kill plant cells, but are also toxic to bacterial cells. Glyphosate inhibits many bacterial EPSP synthases, and thus is toxic to these bacteria. However, certain bacterial EPSP synthases have a high tolerance to glyphosate.

Plant cells resistant to glyphosate toxicity can be produced by transforming plant cells to express glyphosate-resistant bacterial EPSP synthases. Notably, the bacterial gene from *Agrobacterium tumefaciens* strain CP4 has been used to confer herbicide resistance on plant cells following expression in plants. A mutated EPSP synthase from *Salmonella typhimurium* strain CT7 confers glyphosate resistance in bacterial cells, and confers glyphosate resistance on plant cells (U.S. Patent Nos. 4,535,060; 4,769,061; and 5,094,945).

U.S. patent 6,040,497 reports mutant maize EPSP synthase enzymes having substitutions of threonine to isoleucine at position 102 and proline to serine at position 106 (the "TIPS" mutation). Such alterations confer glyphosate resistance upon the maize enzyme. A mutated EPSP synthase from *Salmonella typhimurium* strain CT7 confers glyphosate resistance in bacterial cells, and is reported to confer glyphosate resistance upon plant cells (U.S. Patent Nos. 4,535,060; 4,769,061; and 5,094,945). He et al. ((2001) Biochim et Biophysica Acta 1568:1-6) have developed EPSP synthases with increased glyphosate tolerance by mutagenesis and recombination between the *E. coli* and *Salmonella typhimurium* EPSP synthase genes, and suggest that mutations at position 42 (T42M) and position 230 (Q230K) are likely responsible for the observed resistance. Subsequent work (He et al. (2003) Biosci. Biotech. Biochem. 67:1405-1409) shows that the T42M mutation (threonine to methionine) is sufficient to improve tolerance of both the *E. coli* and *Salmonella typhimurium* enzymes.

Due to the many advantages herbicide resistance plants provide, methods for identifying herbicide resistance genes with glyphosate resistance activity are desirable.

### SUMMARY OF INVENTION

The present invention provides a method of producing genetically transformed plants which are tolerant toward glyphosate herbicide, comprising the steps of:
a) inserting into the genome of a plant cell a polynucleotide encoding an EPSP synthase polypeptide having a Q-loop comprising D-C-X₁-X₂-S-G (SEQ ID NO:29), X₁ denoting glycine, serine, alanine or asparagine, X₂ denoting asparagine or glutamic acid, wherein said polypeptide has at least 95% sequence identity to SEQ ID NO:10, and wherein said polypeptide is resistant to glyphosate;
b) obtaining a transformed plant cell; and,
c) regenerating from the transformed plant cell a genetically transformed plant which has increased tolerance to glyphosate herbicide.

The present invention additionally provides a glyphosphate tolerant plant cell comprising a heterologous polynucleotide encoding an EPSP synthase polypeptide having a Q-loop comprising D-C-X₁-X₂-S-G (SEQ ID NO:29), X₁ denoting glycine, serine, alanine or asparagine, X₂ denoting asparagine or glutamic acid, wherein said polypeptide is resistant to glyphosate and wherein said polypeptide has at least 95% sequence identity to SEQ ID NO:10.

The present invention further provides a method for selectively controlling weeds in a field containing a plant having planted seeds or plants comprising the steps of:
a) planting the seeds or plants which are glyphosate tolerant as a result of a polynucleotide encoding a glyphosate resistance EPSP synthase polypeptide having a Q loop comprising D-C-X₁-X₂-S-G (SEQ ID NO:29) being inserted into the seed or plant, X₁ denoting glycine, serine, alanine or asparagine, X₂ denoting asparagine or glutamic acid, wherein said polypeptide has at least 95% sequence identity to SEQ ID NO:10; and,
b) applying to the plants and weeds in a field an effective concentration of glyphosate herbicide to control weeds without significantly affecting the plants.

The EPSP synthase enzyme employed in the invention comprises the following domain:
D-C-X₁-X₂-S-G (SEQ ID NO:29), where X₁ denotes glycine, serine, alanine or asparagine, and X₂ denotes asparagine or glutamic acid.

Also referred to are the domains:
D-A-X₁-X₂-S-G (SEQ ID NO:30), where X₁ denotes alanine or arginine, and X₂ denotes asparagine or glutamic acid;
K-L-K-X₁-S-A (SEQ ID NO:31), where X₁ denotes glycine, asparagine or glutamic acid; and,
W-C-E-D-A-G (SEQ ID NO:32).

The nucleotide sequences referred to can be used in DNA constructs or expression cassettes for transformation and expression in organisms, including microorganisms and plants. Compositions also comprise transformed bacteria, plants, plant cells, tissues, and seeds that are glyphosate resistant by the introduction of the compositions into the genome of the organism. Where the organism is a plant, the introduction of the sequence allows for glyphosate containing herbicides to be applied to plants to selectively kill glyphosate sensitive weeds or other untransformed plants, but not the transformed organism.

### DESCRIPTION OF FIGURES

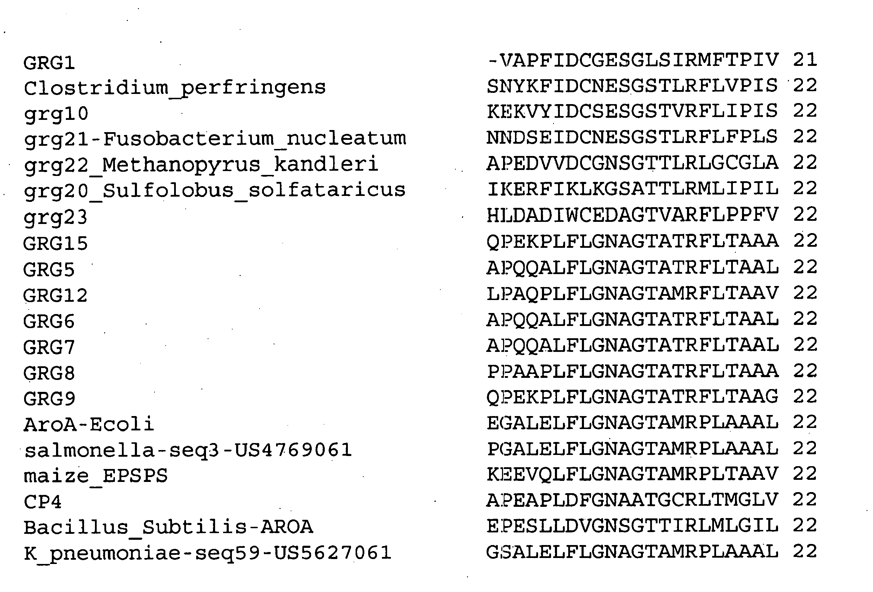

Figure 1 shows an alignment of the amino acid region corresponding to the Q-loop region described herein. The alignment shows GRG1 (amino acid residues 80-100 of SEQ ID NO:2); *Clostridium perfringens* EPSPS (amino acid residues 80-100 of SEQ ID NO:3); GRG10 (amino acid residues 80-100 of SEQ ID NO:6); GRG21 (amino acid residues 80-100 of SEQ ID NO:8); GRG22 (amino acid residues 80-100 of SEQ ID NO:10); GRG20 (amino acid residues 80-100 of SEQ ID NO:12); GRG23 (amino acid residues 80-100 of SEQ ID NO:14); GRG15 (amino acid residues 80-100 of SEQ ID NO:15); GRG5 (amino acid residues 80-100 of SEQ ID NO:16); GRG12 (amino acid residues 80-100 of SEQ ID NO:17); GRG6 (amino acid residues 80-100 of SEQ ID NO:18); GRG7 (amino acid residues 80-100 of SEQ ID NO:19); GRG8 (amino acid residues 80-100 of SEQ ID NO:20); GRG9 (amino acid residues 80-100 of SEQ ID NO:21); *E. coli* AroA (amino acid residues 85-106 of SEQ ID NO:22); *Salmonella typhimurium* EPSPS (amino acid residues 85-106 of SEQ ID NO:23); Zea *mays* EPSPS (amino acid residues 85-106 of SEQ ID NO:24); *Agrobacterium tumefaciens* strain CP4 EPSPS (amino acid residues 85-106 of SEQ ID NO:25); *Bacillus subtilis* AroA (amino acid residues 85-106 of SEQ ID NO:26); and *Kleibsella pneumoniae* EPSPS (amino acid residues 85-106 of SEQ ID NO:27).

### DETAILED DESCRIPTION OF THE INVENTION

### I. Compositions

Compositions and methods for conferring herbicide resistance or tolerance, particularly glyphosate resistance or tolerance, in organisms are referred to. The methods involve transforming organisms with nucleotide sequences encoding a glyphosate tolerance gene wherein said gene encodes a polypeptide having a Q-loop comprising an amino acid sequence with increased polarity. The region of the Q-loop can be identified by aligning amino acid sequences with the conserved arginine in the amino acid region corresponding to positions 90-105 of SEQ ID NO:22. As used herein, the phrase "corresponding to" or "corresponds to" when referring to amino acid (or nucleotide) position numbers means that one or more amino acid (or nucleotide) sequences aligns with the reference sequence at the position numbers specified in the reference sequence. For example, to identify a Q-loop region in an amino acid sequence that corresponds to amino acids 90-105 of SEQ ID NO:22, one could align the amino acid sequence in question with the amino acid sequence of SEQ ID NO:22 using alignment methods discussed elsewhere herein, and identify the region of the amino acid sequence in question that aligns with amino acid residues 90-105 of SEQ ID NO:22. It is recognized that the amino acid number may vary by about plus or minus 20, 15, 10, 9, 8, 7, 6, 5, 4, 3, 2 or 1 amino acid(s) on either side of the Q-loop. The region is believed to be involved in the recognition of the substrate PEP. In particular, a class of enzymes that confers glyphosate resistance or tolerance, and nucleotide sequences encoding such enzyme Such enzymes may also be identified by having at least one sequence domain is referred to selected from the following:
D-C-X₁-X₂-S-G (SEQ ID NO:29), where X₁ denotes glycine, serine, alanine or asparagine, and X₂ denotes asparagine or glutamic acid; or,
D-A-X₁-X₂-S-G (SEQ ID NO:30), where X₁ denotes alanine or arginine, and X₂ denotes asparagine or glutamic acid; or,
K-L-K-X₁-S-A (SEQ ID NO:31), where X₁ denotes glycine, asparagine or glutamic acid; or,

The sequence domain may further comprises a serine or threonine at the amino acid position corresponding to residue 98 of SEQ ID NO:22. By "increased polarity of the Q-loop region" is intended that one or more of the amino acids within the Q-loop have an increased polarity when compared to the same region of an EPSP synthase not containing a sequence domain referred to herein. The sequences find use in preparing plants that show increased resistance to the herbicide glyphosate. Thus, transformed bacteria, plants, plant cells, plant tissues and seeds are referred to.

### A. EPSP synthase

In the present invention, the class of enzyme that confers glyphosate resistance is an EPSP synthase. The term "EPSP synthase" as used herein refers to both a native EPSP synthase or a variant or fragment thereof. EPSP synthase is involved in the penultimate step in the shikimic acid pathway for the biosynthesis of aromatic amino acids and many secondary metabolites, including tetrahydrofolate, ubiquinone and vitamin K (Gruys et al. (1999) Inhibitors of Tryptophan, Pherryalanine, and Tyrosine Biosynthesis as Herbicides (Dekker, New York)). EPSP synthase converts phosphoenolpyruvic acid (PEP) and 3-phosphoshikimic acid (S3P) to 5-enolpyruvyl-3-phosphoshikimic acid (Amrhein et al. (1980) Plant Physiol. 66:830-834). The monomeric EPSP synthase is one of two enzymes in the class of enolpyruvyltransferases. This class of polypeptides shares a unique structure containing two globular domains composed of beta sheets and alpha helices which form something like an inverse alpha/beta barrel. The two domains are connected by two strands which act like a hinge to bring the upper and lower domains together, sandwiching the substrates in the active site. Ligand binding converts the enzyme from an open state to a tightly-packed closed state, following the pattern of an induced-fit mechanism (Schönbrunn et al. (2001) Proc. Natl. Acad. Sci. USA 90:1376-1380, Stauffer et al. (2001) Biochemistry 40:3951-3957).

EPSP synthase has been isolated from plants, bacteria and fungi, including *E. coli* (Duncan et al. (1984) FEBS Lett. 170:59-63), *Staphylococcus aureus* (Horsburgh et al. (1996) Microbiology 142(Part 10):2943-2950), *Streptococcus pneumoniae* (Du et al. (2000) Eur. J. Biochem. 267(1):222-227) and *Salmonella typhi* (Chatfield et al. (1990) Nucleic Acids Res. 18(20):6133). Variants of the wild-type EPSP synthase enzyme have been isolated which are glyphosate tolerant as a result of alterations in the EPSP synthase amino acid coding sequence (Kishore and Shah (1988) Annu. Rev. Biochem. 57:627-63; Wang et al. (2003) J. Plant Res. 116:455-60; Eschenburg et al. (2002) Planta 216:129-35).

EPSP synthase sequences have been characterized and residues frequently conserved in this class of polypeptides have been identified. For example, Lys-22, Arg-124, Asp-313, Arg-344, Art-386, and Lys-411, are conserved residues of the EPSP synthase from *E. coli* (Schönbrunn et al. (2001) Proc. Natl. Acad. Sci. USA 98:1376-1380). Additional residues that influence EPSP synthase activity also include Arg-100, Asp-242, and Asp-384 (Selvapandiyan et al. (1995) FEBS Letters 374:253-256). Arg-27 has been shown to bind to S3P (Shuttleworth et al. (1999) Biochemistry 38:296-302).

### B. Glyphosate-resistant EPSP synthase

EPSP synthase is the target of the broad-spectrum herbicide glyphosate. By "glyphosate" is intended any herbicidal form of N-phosphonomethylglycine (including any salt thereof) and active derivatives thereof that result in the production of the glyphosate anion. Inhibition of EPSP synthase by glyphosate has been shown to proceed through the formation of an EPSP synthase-S3P-glyphosate ternary complex and the binding is ordered with glyphosate binding to the enzyme only after the formation of a binary EPSP synthase-S3P complex. Binding of glyphosate to EPSP synthase has been shown to be competitive with PEP and noncompetitive with respect to S3P (Kishore et al. (1988) Annu. Rev. Biochem. 57:627-663). By binding to EPSP synthase, glyphosate shuts down the shikimic acid pathway, thereby leading to a depletion of aromatic amino acid biosynthesis and death or severe growth reduction of the plant.

Glyphosate-resistant EPSP synthase polypeptides have been identified and used to increase glyphosate tolerance in plants. A "glyphosate resistance polypeptide" or "glyphosate tolerance polypeptide" includes a polypeptide that confers upon a cell the ability to tolerate a higher concentration of glyphosate than cells that do not express the polypeptide, or to tolerate a certain concentration of glyphosate for a longer period of time than cells that do not express the polypeptide. By "tolerate" or "tolerance" is intended either to survive, or to carry out essential cellular functions such as protein synthesis and respiration in a manner that is not readily discernable from untreated cells. An example of a naturally-occurring glyphosate-resistant EPSP synthase includes the bacterial gene from *Agrobacterium tumefacians* strain CP4 which has been used to confer herbicide resistance on plant cells following expression in plants. Mutated EPSP synthase polypeptides have been identified through random mutagenesis and selection for herbicide resistance, including a mutated EPSP synthase from *Salmonella typhimurium* strain CT7 that confers glyphosate resistance in bacterial cells, and confers glyphosate resistance on plant cells (U.S. Patent Nos. 4,535,060; 4,769,061; and 5,094,945 and U.S. Appl. Nos. 60/669,686 and 20040177399). These enzymes contain amino acid substitutions in their active sites that prevent the binding of glyphosate without affecting binding by PEP or S3P. Mutations that occur in the hinge region between the two globular domains of EPSP synthase have been shown to alter the binding affinity of glyphosate but not PEP (He et al. (2003) Biosci. Biotechnol. Biochem. 67(6):1405-1409). Therefore, such enzymes have high catalytic activity, even in the presence of glyphosate.

The EPSP synthase enzymes employed in the present invention is characterized as having a Q-loop region with increased polarity. The enzyme is characterized by having the domain:
D-C-X₁-X₂-S-G (SEQ ID NO:29), where X₁ denotes glycine, serine, alanine or asparagine, and X₂ denotes asparagine or glutamic acid.

### C. Activity of EPSP synthase

A variety of methods can be used to measure EPSP synthase activity. For example, Lewendon et al. ((1983) Biochem J. 213:187-191) describes two assays which couple the EPSP synthase reaction with other enzymes which produced detectable products. In the forward direction, EPSP synthase can be coupled with chorismate synthase, the enzyme in the shikimate acid pathway that converts EPSP to chorismate; as EPSP synthase produces EPSP, chorismate synthase can convert EPSP to chorismate which can be detected at 275 nm. Since EPSP synthase can also proceed in the reverse direction, activity can also be assayed with coupling to pyruvate kinase and lactate dehydrogenase which oxidize NADH in the breakdown of pyruvate, allowing the detection of NADH loss at 340 nm which corresponds to pyruvate evolution by EPSP synthase. EPSP synthase activity can also be assayed by measuring an increase in resistance of a plant to glyphosate when glyphosate-resistant EPSP synthase is present, or by measuring an increase in plant yield when glyphosate-sensitive and/or -tolerant EPSP synthase is expressed.

### D. Isolated polynucleotides, and variants and fragments thereof

An "isolated" or "purified" polynucleotide or polypeptide, or biologically active portion thereof, is substantially free of other cellular material, or culture medium when produced by recombinant techniques, or substantially free of chemical precursors or other chemicals when chemically synthesized. By "biologically active" is intended to possess the desired biological activity of the native polypeptide, that is, retain herbicide resistance or tolerance activity. An "isolated" polynucleotide may be free of sequences (for example, protein encoding sequences) that naturally flank the nucleic acid (i.e., sequences located at the 5' and 3' ends of the nucleic acid) in the genomic DNA of the organism from which the polynucleotide is derived. For purposes of the invention, "isolated" when used to refer to polynucleotides excludes isolated chromosomes. For example, the isolated glyphosate resistance-encoding polynucleotide can contain less than about 5 kb, 4 kb, 3 kb, 2 kb, 1 kb, 0.5 kb, or 0.1 kb of nucleotide sequence that naturally flanks the polynucleotide in genomic DNA of the cell from which the polynucleotide is derived.

Polynucleotides regerred to herein include those encoding polypeptides characterized by having a Q-loop with increased polarity or at least one domain referred to herein The information used in identifying these domains includes sequence alignments of EPSP synthase enzymes as described elsewhere herein. The sequence alignments are used to identify regions of homology between the sequences and to identify the domains that are characteristic of these EPSP synthase enzymes.

Aslo referred to are variants and fragments of the polynucleotides described herein. A "fragment" of a polynucleotide may encode a biologically active portion of a polypeptide, or it may be a fragment that can be used as a hybridization probe or PCR primer using methods disclosed elsewhere herein. Polynucleotides that are fragments of a polynucleotide comprise at least about 15, 20, 50, 75, 100, 200, 300, 350, 400, 450, 500, 550, 600, 650, 700, 750, 800, 850, 900, 950, 1000, 1050, 1100, 1150, 1200, 1250, 1300, 1350, 1400, 1454, 1500, 1550, 1600, 1650, 1700, 1750, 1800, 1850, 1900, 1950 contiguous nucleotides, or up to the number of nucleotides present in a full-length polynucleotide disclosed herein depending upon the intended use. By "contiguous" nucleotides is intended nucleotide residues that are immediately adjacent to one another.

Fragments of the polynucleotides generally will encode polypeptide fragments that retain the biological activity of the full-length glyphosate resistance protein; i.e., herbicide-resistance activity. By "retains herbicide resistance activity" is intended that the fragment will have at least about 30%, at least about 50%, at least about 70%, or at least about 80% of the herbicide resistance activity of the full-length glyphosate resistance protein disclosed herein as SEQ ID NO:1. Methods for measuring herbicide resistance activity are well known in the art. See, for example, U.S. Patent Nos. 4,535,060, and 5,188,642.

A fragment of a polynucleotide that encodes a biologically active portion of a polypeptide will encode at least about 15, 25, 30, 50, 75, 100, 125, 150, 175, 200, 250, 300, 350, 400 contiguous amino acids, or up to the total number of amino acids present in a full-length polypeptide.

Also referred to are variant polynucleotides. "Variants" of the polynucleotide include those sequences that encode the polypeptides disclosed herein but that differ conservatively because of the degeneracy of the genetic code, as well as those that are sufficiently identical. The term "sufficiently identical" is intended a polypeptide or polynucleotide sequence that has at least about 95%, 96%, 97%, 98% or 99% sequence identity compared to a reference sequence using one of the alignment programs using standard parameters. One of skill in the art will recognize that these values can be appropriately adjusted to determine corresponding identity of polypeptides encoded by two polynucleotides by taking into account codon degeneracy, amino acid similarity, reading frame positioning, and the like.

To determine the percent identity of two amino acid sequences or of two polynucleotides, the sequences are aligned for optimal comparison purpose. The percent identity between the two sequences is a function of the number of identical positions shared by the sequences (i.e., percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain polynucleotides homologous to herbicide resistance-encoding polynucleotides used in methods of the invention. BLAST polypeptide searches can be performed with the BLASTX program, score = 50, wordlength = 3, to obtain amino acid sequences homologous to polypeptide molecules expressed using the methods of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. See Altschul *et al. (1997) supra.* When utilizing BLAST, Gapped BLAST, and PSI-Blast programs, the default parameters of the respective programs (e.g., BLASTX and BLASTN) can be used. See www.ncbi.nlm.nih.gov. Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with Clustal W, the percent amino acid identity can be assessed. A non-limiting example of a software program useful for analysis of Clustal W alignments is GeneDoc™. Genedoc™ (Karl Nicholas) allows assessment of amino acid (or DNA) similarity and identity between multiple polypeptides. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM 120 weight residue table, a gap length penalty of 12, and a gap penalty of 4 can be used.

Unless otherwise stated, GAP Version 10, which uses the algorithm of Needleman and Wunsch (1970) *supra,* will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

Naturally occurring allelic variants can be identified with the use of well-known molecular biology techniques, such as polymerase chain reaction (PCR) and hybridization techniques as outlined below. Variant polynucleotides also include synthetically derived polynucleotides that have been generated, for example, by using site-directed mutagenesis but which still encode the polypeptide having the desired biological activity.

The skilled artisan will further appreciate that changes can be introduced by mutation into the polynucleotides thereby leading to changes in the amino acid sequence of the encoded polypeptides, without altering the biological activity of the polypeptides. Thus, variant isolated polynucleotides can be created by introducing one or more nucleotide substitutions, additions, or deletions into the corresponding polynucleotide disclosed herein, such that one or more amino acid substitutions, additions or deletions are introduced into the encoded polypeptide. Mutations can be introduced by standard techniques, such as site-directed mutagenesis and PCR-mediated mutagenesis, or gene shuffling techniques. Such variant polynucleotides are also referred to.

Variant polynucleotides can be made by introducing mutations randomly aiong all or part of the coding sequence, such as by saturation mutagenesis, and the resultant mutants can be screened for the ability to confer herbicide resistance activity to identify mutants that retain activity. Following mutagenesis, the encoded polypeptide can be expressed recombinantly, and the activity of the polypeptide can be determined using standard assay techniques.

Gene shuffling or sexual PCR procedures (for example, Smith (1994) Nature 370:324-325; U.S. Pat. Nos. 5,837,458; 5,830,721; 5,811,238; and 5,733,731) can be used to identify additional polynucleotides that encode polypeptides that perform similar functions as those described herein (for example, polypeptides that confer glyphosate resistance). Gene shuffling involves random fragmentation of several mutant DNAs followed by their reassembly by PCR into full length molecules. Examples of various gene shuffling procedures include, but are not limited to, assembly following DNase treatment, the staggered extension process (STEP), and random priming *in vitro* recombination. In the DNase mediated method, DNA segments isolated from a pool of positive mutants are cleaved into random fragments with DNaseI and subjected to multiple rounds of PCR with no added primer. The lengths of random fragments approach that of the uncleaved segment as the PCR cycles proceed, resulting in mutations in different clones becoming mixed and accumulating in some of the resulting sequences. Multiple cycles of selection and shuffling have led to the functional enhancement of several enzymes (Stemmer (1994) Nature 370:389-391; Stemmer (1994) Proc. Natl. Acad. Sci. USA 91:10747-10751; Crameri et al. (1996) Nat. Biotechnol. 14:315-319; Zhang et al. (1997) Proc. Natl. Acad. Sci. USA 94:4504-4509; and Crameri et al. (1997) Nat. Biotechnol. 15:436-438). Such procedures could be performed, for example, on polynucleotides encoding EPSP synthase enzymes having a Q-loop region with increased polarity or polypeptides comprising domains referred to herein to generate polypeptides that confer glyphosate resistance.

Using methods such as PCR, hybridization, and the like corresponding herbicide resistance sequences can be identified by looking for the conserved domains referred to herein. See, for example, Sambrook and Russell (2001) Molecular Cloning: A Laboratory Manual (Cold Spring Harbor Laboratory Press, Cold Spring Harbor, NY) and Innis et al. (1990) PCR Protocols: A Guide to Methods and Applications (Academic Press, NY).

In a hybridization method, all or part of the herbicide resistance polynucleotide sequence or a sequence encoding a domain referred to herein can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known herbicide resistance-encoding nucleotide sequence described herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, at least about 25, at least about 50, 75, 100, 125, 150, 175, 200, 250, 300, 350, 400, 500, 600, 700, 800, 900, 1000, 1200, 1400, 1600, or 1800 consecutive nucleotides of the herbicide resistance-encoding polynucleotide or a fragment or variant thereof. Methods for the preparation of probes for hybridization are generally known in the art and are disclosed in Sambrook and Russell (2001) *supra,* and Sambrook et al. (1989) Molecular Cloning: A Laboratory Manual (2d ed., Cold Spring Harbor Laboratory Press, Cold Spring Harbor, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, or less than about 500 nucleotides in length,

Stringent conditions will be those in which the salt concentration is less than about 1.5 M Na ion, or about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulfate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the polynucleotide sequence, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash conditions, and desired Tₘ, those of ordinary skill in the art will understand that variations in the stringency of hybridization and/or wash solutions are inherently described. If the desired degree of mismatching results in a Tₘ of less than 45°C (aqueous solution) or 32°C (formamide solution), the SSC concentration can be increased so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, NY).

### E. Isolated Proteins and Variants and Fragments Thereof

An "isolated" or "purified" herbicide resistance polypeptide that is substantially free of cellular material includes preparations of polypeptides having less than about 30%, 20%, 10%, or 5% (by dry weight) of non-herbicide resistance polypeptide (also referred to herein as a "contaminating protein"). In the present invention, "herbicide resistance protein" is intended an EPSP synthase enzyme having a Q-loop region with increased polarity, with at least one domain comprising SEQ ID No: 29, the synthase having at least 95% sequence identity to SEQ ID No: 10. Fragments, biologically active portions, and variants thereof are also referred to and may be used to practice the methods.

"Fragments" or "biologically active portions" include polypeptide fragments comprising a portion of an amino acid sequence encoding an herbicide resistance protein and that retains herbicide resistance activity. A biologically active portion of an herbicide resistance protein can be a polypeptide that is, for example, 10, 25, 50, 100 or more amino acids in length. Such biologically active portions can be prepared by recombinant techniques and evaluated for herbicide resistance activity.

By "variants" is intended proteins or polypeptides having an amino acid sequence that is at least about 95%, 96%, 97%, 98% or 99% identical to the EPSP synthase polypeptide of SEQ ID No: 10. One of skill in the art will recognize that these values can be appropriately adjusted to determine corresponding identity of polypeptides encoded by two polynucleotides by taking into account codon degeneracy, amino acid similarity, reading frame positioning, and the like.

For example, conservative amino acid substitutions may be made at one or more nonessential amino acid residues. A "nonessential" amino acid residue is a residue that can be altered from the wild-type sequence of a polypeptide without substantially altering the biological activity of the resulting peptide, whereas an "essential" amino acid residue a residue that cannot be substituted without substantially affecting biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Amino acid substitutions may be made in nonconserved regions that retain function. In general, such substitutions would not be made for conserved amino acid residues, or for amino acid residues residing within a conserved motif, where such residues are essential for polypeptide activity. However, one of skill in the art would understand that functional variants may have minor conserved or nonconserved alterations in the conserved residues.

Amino acid substitutions that are made to increase the polarity and/or bulkiness of the EPSP synthase binding pocket for PEP and glyphosate (herein referred to as the "Q-loop") are also referred to. This loop forms a portion of the binding pocket for PEP and glyphosate, and contains an invariant arginine that is known to hydrogen bond directly with the phosphate of PEP (Shuttleworth et al. (1999) Biochemistry 38:296-302).

An increase in the polarity of this region refers to an increase in the number or relative percent composition of polar and/or charged amino acids in a given polypeptide sequence relative to the polypeptide sequence in this region of *E*. *coli* AroA (SEQ ID NO:22), which is an example of an EPSP synthase enzyme not having a domain referred to herein. For example, the substitution of an aspartic acid residue for a phenylalanine residue at position 1 of SEQ ID NO:33 and 34 (which corresponds to an example sequence in the Q-loop region) may, while not being bound by any mechanism of action, result in charge repulsion between the loop and the negatively charged phosphonate residue of glyphosate. Methods and algorithms for estimating the net charge and/or net polarity of a particular amino acid composition are known in the art.

An increase in bulk (for example, by the substitution of the more bulky lysine residues at positions 1 and 3, respectively, of SEQ ID NO:31, in place of the less bulky phenylalanine and glycine residues present in other EPSP synthases) in this loop may, while not bound by any mechanism of action, result in steric effects resulting in a downward displacement of this loop further into the binding pocket, reducing the size of the active site pocket. Polypeptides (as well as the polynucleotides encoding them) in which an increase in bulk in the Q-loop has been introduced by substitution of one or more residues in the Q-loop for a more bulky residue are also referred to.

Variants also include polypeptides encoded by a polynucleotide that hybridizes to the polynucleotide encoding an enzyme having a Q-loop region with increased polarity, or a complement thereof, under stringent conditions. Variants include polypeptides that differ in amino acid sequence due to mutagenesis. Variant proteins employed in the present invention are biologically active, that is they continue to possess the desired biological activity of the native protein, that is, retain herbicide resistance activity. Methods for measuring herbicide resistance activity are well known in the art. See, for example, U.S. Patent Nos. 4,535,060, and 5,188,642.

Bacterial genes quite often possess multiple methionine initiation codons in proximity to the start of the open reading frame. Often, translation initiation at one or more of these start codons will lead to generation of a functional protein. These start codons can include ATG codons. However, bacteria such as *Bacillus* sp. also recognize the codon GTG as a start codon, and proteins that initiate translation at GTG codons contain a methionine at the first amino acid. Furthermore, it is not often determined *a priori* which of these codons are used naturally in the bacterium. Thus, it is understood that use of one of the alternate methionine codons may lead to generation of variants that confer herbicide resistance. These herbicide resistance proteins may be used in the methods

### F. Polynucleotide Constructs

The polynucleotides employed in the methods of the invention may be modified to obtain or enhance expression in plant cells. The polynucleotides encoding the domains used in the invention may be provided in expression cassettes for expression in the plant of interest. A "plant expression cassette" includes a DNA construct that is capable of resulting in the expression of a polynucleotide in a plant cell. The cassette can include in the 5'-3' direction of transcription, a transcriptional initiation region (i.e., promoter) operably-linked to one or more polynucleotides of interest, and a translation and transcriptional termination region (i.e., termination region) functional in plants. The cassette may additionally contain at least one additional polynucleotide to be introduced into the organism, such as a selectable marker gene. Alternatively, the additional polynucleotide(s) can be provided on multiple expression cassettes. Such an expression cassette is provided with a plurality of restriction sites for insertion of the polynucleotide(s) to be under the transcriptional regulation of the regulatory regions.

"heterologous" generally refers to the polynucleotide or polypeptide that is not endogenous to the cell or is not endogenous to the location in the native genome in which it is present, and has been added to the cell by infection, transfection, microinjection, electroporation, microprojection, or the like. By "operably linked" is intended a functional linkage between two polynucleotides. For example, when a promoter is operably linked to a DNA sequence, the promoter sequence initiates and mediates transcription of the DNA sequence. It is recognized that operably linked polynucleotides may or may not be contiguous and, where used to reference the joining of two polypeptide coding regions, the polypeptides are expressed in the same reading frame.

The promoter may be any polynucleotide sequence which shows transcriptional activity in the chosen plant cells, plant parts, or plants. The promoter may be native or homologous, or foreign or heterologous, to the plant host and/or to the DNA sequence of the invention. Where the promoter is "native" or "homologous" to the plant host, it is intended that the promoter is found in the native plant into which the promoter is introduced. Where the promoter is "foreign" or "heterologous" to the DNA sequence, it is intended that the promoter is not the native or naturally occurring promoter for the operably linked DNA sequence of the invention. The promoter may be inducible or constitutive. It may be naturally-occurring, may be composed of portions of various naturally-occurring promoters, or may be partially or totally synthetic. Guidance for the design of promoters is provided by studies of promoter structure, such as that of Harley and Reynolds (1987) Nucleic Acids Res. 15:2343-2361. Also, the location of the promoter relative to the transcription start may be optimized. See, e.g., Roberts et al. (1979) Proc. Natl. Acad. Sci. USA, 76:760-764. Many suitable promoters for use in plants are well known in the art.

For instance, suitable constitutive promoters for use in plants include: the promoters from plant viruses, such as the peanut chlorotic streak caulimovirus (PCISV) promoter (U.S. Pat. No. 5,850,019); the 35S promoter from cauliflower mosaic virus (CaMV) (Odell et al. (1985) Nature 313:810-812); promoters of Chlorella virus methyltransferase genes (U.S. Pat. No. 5,563,328) and the full-length transcript promoter from figwort mosaic virus (FMV) (U.S. Pat. No. 5,378,619); the promoters from such genes as rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); maize H3 histone (Lepetit et al. (1992) Mol. Gen. Genet. 231:276-285 and Atanassova et al. (1992) Plant J. 2(3):291-300); Brassica napus ALS3 (PCT application WO 97/41228); and promoters of various *Agrobacterium* genes (see U.S. Pat. Nos. 4,771,002; 5,102,796; 5,182,200; and 5,428,147).

Suitable inducible promoters for use in plants include: the promoter from the ACE1 system which responds to copper (Mett et al. (1993) PNAS 90:4567-4571); the promoter of the maize In2 gene which responds to benzenesulfonamide herbicide safeners (Hershey et al. (1991) Mol. Gen. Genetics 227:229-237 and Gatz et al. (1994) Mol. Gen. Genetics 243:32-38); and the promoter of the Tet repressor from *Tn10 (*Gatz et al. (1991) Mol. Gen. Genet. 227:229-237). Another inducible promoter for use in plants is one that responds to an inducing agent to which plants do not normally respond. An exemplary inducible promoter of this type is the inducible promoter from a steroid hormone gene, the transcriptional activity of which is induced by a glucocorticosteroid hormone (Schena et al. (1991) Proc. Natl. Acad. Sci. USA 88:10421) or the recent application of a chimeric transcription activator, XVE, for use in an estrogen receptor-based inducible plant expression system activated by estradiol (Zuo et al. (2000) Plant J., 24:265-273). Other inducible promoters for use in plants are described in EP 332104, PCT WO 93/21334 and PCT WO 97/06269.

Promoters composed of portions of other promoters and partially or totally synthetic promoters can also be used. See, e.g., Ni et al. (1995) Plant J. 7:661-676 and PCT WO 95/14098 describing such promoters for use in plants.

The promoter may include, or be modified to include, one or more enhancer elements. The promoter may include a plurality of enhancer elements. Promoters containing enhancer elements provide for higher levels of transcription as compared to promoters that do not include them. Suitable enhancer elements for use in plants include the PC1SV enhancer element (U.S. Pat. No. 5,850,019), the CaMV 35S enhancer element (U.S. Pat. Nos. 5,106,739 and 5,164,316) and the FMV enhancer element (Maiti et al. (1997) Transgenic Res. 6:143-156). See also PCT WO 96/23898.

Often, such constructs can contain 5' and 3' untranslated regions. Such constructs may contain a "signal sequence" or "leader sequence" to facilitate co-translational or post-translational transport of the peptide of interest to certain intracellular structures such as the chloroplast (or other plastid), endoplasmic reticulum, or Golgi apparatus, or to be secreted. For example, the construct can be engineered to contain a signal peptide to facilitate transfer of the peptide to the endoplasmic reticulum. By "signal sequence" is intended a sequence that is known or suspected to result in cotranslational or post-translational peptide transport across the cell membrane. In eukaryotes, this typically involves secretion into the Golgi apparatus, with some resulting glycosylation. By "leader sequence" is intended any sequence that, when translated, results in an amino acid sequence sufficient to trigger co-translational transport of the peptide chain to a sub-cellular organelle. Thus, this includes leader sequences targeting transport and/or glycosylation by passage into the endoplasmic reticulum, passage to vacuoles, plastids including chloroplasts, mitochondria, and the like. It may also be preferable to engineer the plant expression cassette to contain an intron, such that mRNA processing of the intron is required for expression.

By "3' untranslated region" is intended a polynucleotide located downstream of a coding sequence. Polyadenylation signal sequences and other sequences encoding regulatory signals capable of affecting the addition of polyadenylic acid tracts to the 3' end of the mRNA precursor are 3' untranslated regions. By "5' untranslated region" is intended a polynucleotide located upstream of a coding sequence.

Other upstream or downstream untranslated elements include enhancers. Enhancers are polynucleotides that act to increase the expression of a promoter region. Enhancers are well known in the art and include, but are not limited to, the SV40 enhancer region and the 35S enhancer element.

The termination region may be native with the transcriptional initiation region, may be native with the sequence referred to herein, or may be derived from another source. Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the polynucleotide(s) encoding the polypeptide domains may be optimized for increased expression in the transformed host cell. That is, the sequences can be synthesized using host cell-preferred codons for improved expression, or may be synthesized using codons at a host-preferred codon usage frequency. Generally, the GC content of the polynucleotide will be increased. See, for example, Campbell and Gowri (1990) Plant Physiol. 92:1-11 for a discussion of host-preferred codon usage. Methods are known in the art for synthesizing host-preferred polynucleotides. See, for example, U.S. Patent Nos. 6,320,100; 6,075,185; 5,380,831; and 5,436,391, U.S. Published Application Nos. 20040005600 and 20010003849, and Murray et al. (1989) Nucleic Acids Res. 17:477-498.

The polynucleotides of interest can be targeted to the chloroplast for expression. In this manner, where the polynucleotide of interest is not directly inserted into the chloroplast, the expression cassette will additionally contain a polynucleotide encoding a transit peptide to direct the nucleotide of interest to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The polynucleotides of interest to be targeted to the chloroplast may be optimized for expression in the chloroplast to account for differences in codon usage between the plant nucleus and this organelle. In this manner, the polynucleotides of interest may be synthesized using chloroplast-preferred codons. See, for example, U.S. Patent No. 5,380,831.

This plant expression cassette can be inserted into a plant transformation vector. By "transformation vector" is intended a DNA molecule that allows for the transformation of a cell. Such a molecule may consist of one or more expression cassettes, and may be organized into more than one vector DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a polynucleotide construct designed for transfer between different host cells. "Expression vector" refers to a vector that has the ability to incorporate, integrate and express heterologous DNA sequences or fragments in a foreign cell.

The plant transformation vector comprises one or more DNA vectors for achieving plant transformation. For example, it is a common practice in the art to utilize plant transformation vectors that comprise more than one contiguous DNA segment. These vectors are often referred to in the art as binary vectors. Binary vectors as well as vectors with helper plasmids are most often used for *Agrobacterium-*mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "polynucleotide of interest" (a polynucleotide engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker sequence and the sequence of interest are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science, 5:446-451). Several types of *Agrobacterium* strains (e.g., LBA4404, GV3101, EHA101, EHA105, etc.) can be used for plant transformation. The second plasmid vector is not necessary for introduction of polynucleotides into plants by other methods such as microprojection, microinjection, electroporation, polyethylene glycol, etc.

### G. Plants and Plant Parts

By "plant" is intended whole plants, plant organs (e.g., leaves, stems, roots, etc.), seeds, plant cells, propagules, embryos and progeny of the same. Plant cells can be differentiated or undifferentiated (e.g., callus, suspension culture cells, protoplasts, leaf cells, root cells, phloem cells, pollen). The present invention may be used for introduction of polynucleotides into any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Crop plants are also of interest, including, for example, maize, sorghum, wheat, sunflower, tomato, crucifers, peppers, potato, cotton, rice, soybean, sugarbeet, sugarcane, tobacco, barley, oilseed rape, etc.

This invention is suitable for any member of the monocot plant family including, but not limited to, maize, rice, barley, oats, wheat, sorghum, rye, sugarcane, pineapple, yams, onion, banana, coconut, and dates.

### II. Methods

### A. Plant transformation

The present invention provides a method of producing genetically transformed plants which are tolerant toward glyphosate herbicide, comprising the steps of:
a) inserting into the genome of a plant cell a polynucleotide encoding an EPSP synthase polypeptide having a Q-loop comprising D-C-X₁-X₂-S-G (SEQ ID NO:29), X₁ denoting glycine, serine, alanine or asparagine, X₂ denoting asparagine or glutamic acid, wherein said polypeptide has at least 95% sequence identity to SEQ ID NO:10, and wherein said polypeptide is resistant to glyphosate;
b) obtaining a transformed plant cell; and,
c) regenerating from the transformed plant cell a genetically transformed plant which has increased tolerance to glyphosate herbicide

Hence the invention involves introducing one or more such polynucleotides into a plant. By "introducing" is intended to present to the plant the polynucleotide in such a manner that the polynucleotide gains access to the interior of a cell of the plant. The methods of the invention do not require that a particular method for introducing a polynucleotide into a plant be used, only that the polynucleotide gains access to the interior of at least one cell of the plant.

Introduction of a polynucleotide into plant cells is accomplished by one of several techniques known in the art, including but not limited to electroporation or chemical transformation (See, for example, Ausubel, ed. (1994) Current Protocols in Molecular Biology (John Wiley and Sons, Inc., Indianapolis, IN). Markers conferring resistance to toxic substances are useful in identifying transformed cells (having taken up and expressed the test polynucleotide sequence) from non-transformed cells (those not containing or not expressing the test polynucleotide sequence). In one aspect genes are useful as a marker to assess introduction of DNA into plant cells. "Transgenic plants" or "transformed plants" or "stably transformed" plants, cells, tissues or seed refer to plants that have incorporated or integrated exogenous polynucleotides into the plant cell. By "stable transformation" is intended that the polynucleotide construct introduced into a plant integrates into the genome of the plant and is capable of being inherited by progeny thereof.

In general, plant transformation methods involve transferring heterologous DNA into target plant cells (e.g., immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent (i.e., temperature and/or herbicide). The shoots are then transferred to a selective rooting medium for recovering rooted shoot or plantlet. The transgenic plantlet then grow into mature plants and produce fertile seeds (e.g., Hiei et al. (1994) Plant J. 6:271-282; Ishida et al. (1996) Nat. Biotechnol. 14:745-750). A general description of the techniques and methods for generating transgenic plants is found in Ayres and Park (1994) CRG Crit. Rev. Plant Sci. 13:219-239 and Bommineni and Jauhar (1997) Maydica 42:107-120. Since the transformed material contains many cells, both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants. Molecular and biochemical methods may be used to confirm the presence of the integrated polynucleotide(s) of interest in the genome of transgenic plant.

Generation of transgenic plants may be performed by one of several methods, including but not limited to introduction of heterologous DNA by *Agrobacterium* into plant cells (*Agrobcaclerium-mediated* transformation), bombardment of plant cells with heterologous foreign DNA adhered to particles, and various other non-particle direct-mediated methods (e.g., Hiei et al. (1994) Plant J. 6:271-282; Ishida et al. (1996) Nat. Biotechnol. 14:745-750; Ayres and Park (1994) CRC Crit. Rev. Plant Sci. 13:219-239; Bommineni and Jauhar (1997) Maydica 42:107-120) to transfer DNA.

There are three common methods of transforming plant cells with *Agrobacterium.* The first method is co-cultivation of *Agrobacterium* with cultured isolated protoplasts. This method requires an established culture system that allows culturing protoplasts and plant regeneration from cultured protoplasts. The second method is transformation of cells or tissues with *Agrobacterium.* This method requires (a) that the plant cells or tissues can be transformed by *Agrobacterium* and (b) that the transformed cells or tissues can be induced to regenerate into whole plants. The third method is transformation of seeds, apices or meristems with *Agrobacterium.* This method requires micropropagation.

The efficiency of transformation by *Agrobacterium* may be enhanced by using a number of methods known in the art. For example, the inclusion of a natural wound response molecule such as acetosyringone (AS) to the *Agrobacterium* culture has been shown to enhance transformation efficiency with *Agrobacterium tumefaciens* (Shahla et al. (1987) Plant Molec. Biol. 8:291-298). Alternatively, transformation efficiency may be enhanced by wounding the target tissue to be transformed. Wounding of plant tissue may be achieved, for example, by punching, maceration, bombardment with microprojectiles, etc. See, for example, Bidney et al. (1992) Plant Molec. Biol. 18:301-313.

The plant cells can be transfected with vectors via particle bombardment (i.e., with a gene gun). Particle mediated gene transfer methods are known in the art, are commercially available, and include, but are not limited to, the gas driven gene delivery instrument described in U.S. Pat. No. 5,584,807.

This method involves coating the polynucleotide sequence of interest onto heavy metal particles, and accelerating the coated particles under the pressure of compressed gas for delivery to the target tissue.

Other particle bombardment methods are also available for the introduction of heterologous polynucleotide sequences into plant cells. Generally, these methods involve depositing the polynucleotide sequence of interest upon the surface of small, dense particles of a material such as gold, platinum, or tungsten. The coated particles are themselves then coated onto either a rigid surface, such as a metal plate, or onto a carrier sheet made of a fragile material such as mylar. The coated sheet is then accelerated toward the target biological tissue. The use of the flat sheet generates a uniform spread of accelerated particles that maximizes the number of cells receiving particles under uniform conditions, resulting in the introduction of the polynucleotide sample into the target tissue.

Specific initiation signals may also be used to achieve more efficient translation of sequences encoding the polypeptide of interest. Such signals include the ATG initiation codon and adjacent sequences. In cases where sequences encoding the polypeptide of interest, its initiation codon, and upstream sequences are inserted into the appropriate expression vector, no additional transcriptional or translational control signals may be needed. However, in cases where only the coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers that are appropriate for the particular cell system that is used, such as those described in the literature (Scharf et al. (1994) Results Probl. Cell Differ. 20:125).

Cells that have been transformed with a polynucleotide in a method of the invention may be grown into plants in accordance with conventional ways. See, for example, McCormick et al. (1986) Plant Cell Rep. 5:81-84. These plants may then be grown, and pollinated with either the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved.

Following introduction of DNA into plant cells, the transformation or integration of the polynucleotide into the plant genome is confirmed by various methods such as analysis of polynucleotides, polypeptides and metabolites associated with the integrated sequence.

PCR analysis is a rapid method to screen cells, tissue or shoots for the presence of incorporated gene at the earlier stage before transplanting into the soil (Sambrook and Russell (2001) Molecular Cloning: A Laboratory Manual (Cold Spring Harbor Laboratory Press, Cold Spring Harbor, NY)). PCR is carried out using oligonucleotide primers specific to the nucleotide of interest or *Agrobacterium* vector background, etc.

Introduction of DNA may be confirmed by Southern blot analysis of genomic DNA (Sambrook and Russell (2001) *supra*)*.* In general, total DNA is extracted from the cell or organism, digested with appropriate restriction enzymes, fractionated in an agarose gel and transferred to a nitrocellulose or nylon membrane. The membrane or "blot" is then probed with, for example, radiolabeled ³²P target DNA fragment to confirm the integration of introduced DNA into the plant genome according to standard techniques (Sambrook and Russell (2001) *supra*)*.*

In Northern analysis, RNA is isolated from specific tissues of the cell or organism, fractionated in a formaldehyde agarose gel and blotted onto a nylon filter according to standard procedures that are routinely used in the art (Sambrook and Russell (2001) *supra*)*.* Expression of RNA encoded by the polynucleotide of the present invention is then tested by hybridizing the filter to a radioactive probe derived from the sequence of interest by methods known in the art (Sambrook and Russell (2001) *supra*)*.*

Western blot, biochemical assays and the like may be carried out on the transgenic plants to determine the presence of a polypeptide(s) encoded by the polynucleotide(s) of interest by standard procedures (Sambrook and Russell (2001) *supra*) using antibodies that bind to one or more epitopes present on the herbicide resistance polypeptide.

### C. Methods for selectively controlling weeds in a crop field

Methods for selectively controlling weeds in a field containing a plant are also provided.

In particular, the present invention provides a method for selectively controlling weeds in a field containing a plant having planted seeds or plants comprising the steps of:
a) planting the seeds or plants which are glyphosate tolerant as a result of a polynucleotide encoding a glyphosate resistance EPSP synthase polypeptide having a Q loop comprising D-C-X₁-X₂-S-G (SEQ ID NO:29) being inserted into the seed or plant, X, denoting glycine, serine, alanine or asparagine, X₂ denoting asparagine or glutamic acid, wherein said polypeptide has at least 95% sequence identity to SEQ ID NO:10; and,
b) applying to the plants and weeds in a field an effective concentration of glyphosate herbicide to control weeds without significantly affecting the plants.

In specific methods, the plant is treated with an effective concentration of an herbicide, where the herbicide application results in a selective control of weeds or other untransformed plants. By "effective concentration" is intended the concentration which controls the growth or spread of weeds or other untransformed plants without significantly affecting the glyphosate-resistant plant or plant seed. Such effective concentrations for herbicides of interest are generally known in the art. The herbicide may be applied either pre- or post emergence in accordance with usual techniques for herbicide application to fields comprising plants or plant seeds which have been rendered resistant to the herbicide.

### D. Predicting Protein Function from Sequence

Additional polypeptides (for example, SEQ ID NO: 8) which confer glyphosate tolerance can be identified. These additional polypeptides can be identified by searching sequence databases containing EPSP synthase sequences, and/or by alignment of polypeptide sequences to search for the presence of domains referred to herein using methods described elsewhere herein. These polypeptides include known polypeptides as well as newly identified polypeptides. It is understood that some modification of these domains are tolerated in nature without disrupting the glyphosate resistance conferring nature of these domains, and are therefore equivalent to the domains listed herein.

In general, there are four levels of protein structure: the primary structure, which consists of the linear chain of amino acids, or the polypeptide sequence; the secondary structure, which is given by the α-helices, β-strands, and turns that the protein folds into; the tertiary structure, which is made up of simple motifs that have combined to form compact globular domains; and the quaternary structure, which can comprise several amino acid chains or subunits. When predicting function from sequence, it is important to identify the functionally important motifs or patterns. Protein domains with similar folds often share the same molecular function (Hegyi and Gentein (1999) J. Mol. Biol. 288:147-164; Moult and Melamud (2000) Curr. Opin. Struct. Biol. 10:384-389; Shakhnovich et al. (2003) J. Mol. Biol. 326:1-9)*.* Identification of domains important to protein function can be done by multiple sequence alignment using, for example, alignment programs described elsewhere herein.

Three-dimensional structure can be predicted by homology modeling, i.e., by using a sequence homolog (>25% sequence identity) with an experimentally determined 3D structure. The three-dimensional structure of, for example, *E. coli* EPSP synthase (AroA) is well known (Shönbrunn et al. (2001) Proc. Natl. Acad. Sci. USA 98:1375-1380). This structure is based on the crystallization of AroA with glyphosate and shikimate 3-phosphate.

The following examples are offered by way of illustration and not by way of limitation.

### EXPERIMENTAL

### Example 1. Identification of glyphosate resistant EPSP synthases.

GRG1 is an EPSP synthase that confers glyphosate resistance upon both bacteria and plants. Comparison of the GRG1 amino acid sequence (SEQ ID NO:2) with the amino acid sequences of other glyphosate resistance EPSP synthase enzymes suggests that GRG1 is significantly different from these enzymes in the region corresponding to amino acids 90-105 of SEQ ID NO:2. This region is known to be involved in recognition of the substrate PEP (Schönbrunn et al. (2001) Proc. Natl. Acad. Sci. USA 90:1376-1380, Stauffer et al. (2001) Biochemistry 40:3951-3957). Notably, GRG1 has a motif of DCxES and a motif of PI in this region that are different from the other known glyphosate-resistant EPSP synthase enzymes. The DNA coding sequence (SEQ ID NO:1) and amino acid sequence of the *grgl* open reading frame (SEQ ID NO:2) are provided in U.S. Patent Application No. 10/739,610, filed December 18, 2003.

Alignment of GRG1 with other EPSP synthase enzymes and analysis of the alignment of amino acids in this Q-loop region identifies a small subset of EPSP synthase enzymes that share significant homology to GRG1 in this region of interest. Notably, the EPSP synthase enzymes from *Clostridium perfringens, Clostridium acetobutylicum, Fusobacterium nucleatum, and Methanopyrus kandleri* (SEQ ID NO: 4, 6, 8, and 10, respectively) are homologous to GRG1 in this region. An alignment of these proteins is provided in Figure 1.

To test the usefulness of this novel domain to predict glyphosate resistance, and to identify novel glyphosate resistant EPSP synthase enzymes, a comparison of the amino acid sequences in this region of GRG1 was performed with a large set of published EPSP synthase amino acid sequences and several other published EPSP synthase enzymes were identified that have amino acid composition in this region similar to GRG1.

### Example 2. Glyphosate resistance of EPSP synthase with homology to GRG1 in the "Q-loop region."

The coding sequence of the *Clostidium acetobutylicum* EPSP synthase gene (SEQ ID NO:5), identified in Genbank accession number NC_003030, was PCR amplified using the following primers:
CAGGGATCCGCCATGAATTGTGTTAAAATAAATCCATG (upper) (SEQ ID NO:42) and CAGGGCGCGCCTTATTCCCCCAAACTCCACTC (lower) (SEQ ID NO:43). The upper primer changed the start codon to ATG from TTG, as it naturally occurs. The resultant 1.3kb product was digested with *BamH* I and *Asc* I, and ligated into the same sites of a modified version of pUC18 and transformed into the *E. coli* strain DH5α. A positive clone containing the EPSP synthase insert was identified by restriction digest and named pAX714. A pAX714 colony was struck onto minimal M63 media containing IPTG, carbenicillin and 0, 20, 50 or 100mM glyphosate, and the plates were incubated at 37°C. The pAX714-containing cells grew very well on all concentrations of glyphosate tested, indicating that the encoded EPSP synthase was glyphosate resistant to at least 100mM. The encoded EPSP synthase (SEQ ID NO:6) was named *grg10.*

### Example 3. Cloning the EPSP synthase gene from Sulfolobus solfataricus

The EPSP synthase coding sequence was PCR-amplified from genomic DNA of *Sulfolobus solfataricus* (ATCC 35092D and SEQ ID NO:11) using the following primers: CAGGGATCCGCCATGATTGTAAAGATTTATCCATC (upper) (SEQ ID NO:44)and CAGGGCGCGCCGGTCTCATTCAATAGAAATCTTCGC (lower) (SEQ ID NO:45). The upper primer changed the start codon to ATG from TTG to facilitate translation in *E*. *coli.* The resultant 1.3 kb PCR product was digested with *Bam*H I *and Asc* I, ligated into modified pUC18 (pAX700 backbone) which had been digested with *Bam*H I and *Asc* I, then transformed into DH5α cells. A positive clone containing the EPSP synthase insert was identified by restriction digest and DNA sequencing, and named pAX716. The encoded EPSP synthase was named *grg20* (SEQ I D NO: 12).

### Example 4. Testing grg10 and grg20 for resistance to glyphosate.

Plasmids pAX714 and pAX716, containing *grg10* and *grg20,* respectively, were transformed into *E*. *coli* cells and streaked onto M63 agar medium containing IPTG, carbenicillin, and various concentrations of glyphosate. Colonies of pAX701 (containing the wild-type *E*. *coli aroA* gene) were used as glyphosate-sensitive controls. The results are presented in the table below and demonstrate that expression of *grg10* or *grg20* confers resistance to high levels of glyphosate.

| Growth of *E*. *coli* expressing *grg10* or *grg20* in the presence of glyphosate. | | | | | |
|---|---|---|---|---|---|
| | | Glyphosate Concentration | | | |
| Plasmid | Gene | 0 mM | 20 mM | 50 mM | 100 mM |
| pAX701 | *E. coli aroA* | ++ | - | - | - |
| pAX714 | *grg10* | ++ | +++ | +++ | +++ |
| pAX716 | *grg20* | ++ | +++ | +++ | +++ |

### Example 5. Molecular Modeling of glyphosate-resistant EPSP synthases.

To further identify the key domains that are predictive of glyphosate resistance, molecular modeling data was analyzed based on the published crystal structure of the *E*. *coli* EPSP synthase. First, the amino acid sequence of GRG1 was fitted to the three dimensional structure of the *E*. *coli* EPSP synthase (AroA) based on its crystallization with glyphosate and shikimate 3-phosphate (Shonbrunn et al. (2001) PNAS98:1375-1380*;* Protein databank code (pdb)1G6T). The results of alteration of each of the domains for an effect on glyphosate binding, or alteration of the substrate binding pocket was analyzed. This analysis revealed a region of interest in the loop that forms a portion of the binding pocket for PEP and its inhibitor glyphosate, and contains an invariant arginine that is known to hydrogen bond directly with phosphate of PEP. This region comprises an amino acid sequence with an increase in polarity and at least one sequence domain selected from the group consisting of:
D-C-X₁-X₂-S-G (SEQ ID NO:29), where X₁ denotes glycine, serine, alanine or asparagine, and X₂ denotes asparagine or glutamic acid; or,
D-A-X₁-X₂-S-G (SEQ ID NO:30), where X₁ denotes alanine or arginine, and X₂ denotes asparagine or glutamic acid; or,
K-L-K-X₁-S-A (SEQ ID NO:31), where X₁ denotes glycine, asparagine or glutamic acid; or,
W-C-E-D-A-G (SEQ ID NO:32).

The domain residues aspartic acid, cysteine, glutamic acid and serine each have the effect of increasing the polarity of this Q-loop region. While not bound by any mechanism of action, the change in polarity in the region of EPSP synthases comprising these domains relative to other classes of EPSP synthase enzymes may result in an increase in the charge repulsion between the loop and the negatively charged phosphonate residue of glyphosate. Likewise, in some examples, the residues in this region appear to increase the bulk of this loop, and may result in steric effects that cause a downward displacement of this loop further into the binding pocket, reducing the size of the active site pocket. This effect may contribute to the reduced affinity for glyphosate observed in EPSP synthase enzymes with one or more domain(s) referred to herein. For example, GRG20 (SEQ ID NO:12) contains a substitution of two lysine residues in this loop. This substitution results in a net increase in polarity, and also results in an increased bulk due to the long side chains of the lysine residues.

Other regions of interest were identified using molecular modeling data. These regions include:
PX (SEQ ID NO:35) where X is isoleucine or leucine.

This region is present in many of the EPSP synthases with one or more domains referred to herein. The insertion of a proline at the top of the alpha helix of the Q-loop region partially unwinds the alpha helix. This insertion is likely to result in a downward displacement or other movement of the loop relative to the binding pocket, thereby affecting binding of glyphosate relative to PEP. D-A-X₁ -X₂-C-P-D-X₃-X₄-P (SEQ ID NO: 36) where X₁ is serine or threonine, X₂ is glutamine or aspartic acid, X₃ is alanine, leucine, methionine, isoleucine or valine, and X₄ is phenylalanine, alanine, leucine, methionine, isoleucine or valine, and where D is a highly conserved residue in all EPSP synthase enzymes.

Both GRG1 and GRG10 have a conserved block of amino acids near a key aspartic acid residue of EPSP synthase. Substitution of these residues onto the *E*. *coli* structure suggests that these residues may affect the distance interaction of this key aspartic acid residue with the carbonyl end of glyphosate.

Comparison of this domain to the amino acid sequence of approximately 169 EPSP synthase enzymes suggests that, while the proline residue corresponding to position 6 of SEQ ID NO:36 is often found in EPSP synthase sequences, the cysteine residue corresponding to position 5 of SEQ ID NO:36 in combination with the proline is unique to GRG1, GRG 10, and *Clostridium perfringens* EPSP synthases. Thus, the presence of this domain also appears to be associated with glyphosate resistance.

### LK (SEQ ID NO:37)

Several glyphosate-resistant EPSP synthase enzymes that contain a Q-loop region with an increased polarity of a domain referred to herein (including, for example, GRG1, GRG 10 and EPSP synthases from *Clostridium perfringens* and *Fusobacterium nucleatum*) also contain a conserved LK domain. Analysis of the location of this sequence by fitting on the *E*. *coli* crystal structure shows that this sequence is exposed to the exterior surface of the molecule. Since this sequence is not close to any known key regions of EPSP synthases, and does not seem to be directly involved in binding of PEP, glyphosate, or shikimate 3-phosphate, the contribution of this sequence to glyphosate resistance is not yet known. Further, since this domain is found in many EPSP synthase enzymes other than those containing domains referred to herein this sequence may have little or no effect on glyphosate resistance in the absence of a Q-loop region having an increased polarity or of a presently described domain. It may however, affect other properties of the protein.

### Example 6. Prediction of additional glyphosate-resistant enzymes comprising domains referred to herein.

Given the discovery of these key domains, we were able to predict the existence of several glyphosate resistant EPSP synthase enzymes.

The EPSP synthase from *Fusobacterium nucleatum* and *Methanopyrus kandleri* are highly homologous to both GRG1 and GRG10 in the Q-loop region, and thus were predicted to confer glyphosate resistance on cells.

### Example 7. Cloning the EPSP synthase gene from Fusobacterium nucleatum subsp. nucleatum

The published amino acid sequence of the *Fusobacterium nucleatum* EPSP synthase (SEQ ID NO:7) was obtained from GENBANK® and designed synthetically by backtranslation and synthesized *in vitro* using DNA 2.0. The resultant DNA sequences were designed to include flanking *BamH* I and *Asc* I sites to facilitate subcloning. The synthetic gene was excised from DNA2.0's donor vector using *BamH* I and *Asc* I, gel purified, ligated into the same sites of a modified pUC18 which had been digested with *BamH* I and *Asc* I, then transformed into DH5α cells. A positive clone containing the EPSP synthase insert was identified by restriction digest and DNA sequencing, and named pAX723 (synFusoII). The encoded EPSP synthase was named *grg21* (SEQ ID NO:8).

### Example 8. Cloning the EPSP synthase gene from Methanopyrus kandleri

The published amino acid sequence of the *Methanopyrus kandleri* EPSP synthase was obtained from GENBANK® and designed synthetically by backtranslation and synthesized *in vitro* using DNA 2.0. The resultant DNA sequence (SEQ ID NO:9) was designed to include flanking *BamH* I *and Asc* I sites to facilitate subcloning. The synthetic gene was excised from DNA2.0's donor vector using *BamH* I and *Asc* I, gel purified, ligated into the same sites of a modified pUC18 which had been digested with *BamH* I and *Asc* I, then transformed into DH5α cells. A positive clone containing the EPSP synthase insert was identified by restriction digest and DNA sequencing, and named pAX724 (synMethII). The encoded EPSP synthase was named *grg22* (SEQ ID NO:10).

### Example 9. Testing grg21 and grg22 for resistance to glyphosate.

Plasmids pAX723 and pAX724, containing *grg21* and *grg22,* respectively, were transformed into *E. coli* cells and streaked onto M63 agar medium containing IPTG, carbenicillin, and various concentrations of glyphosate. Colonies of pAX701 (containing the wild-type *E. coli aroA* gene) were used as glyphosate-sensitive controls. The results are presented in the table below. Expression of *grg21* or *grg22* confers resistance to high levels of glyphosate.

| Growth of *E. coli* expressing *grg21* or *grg22* in the presence of glyphosate. | | | | | |
|---|---|---|---|---|---|
| | | Glyphosate Concentration | | | |
| Plasmid | Gene | 0 mM | 20 mM | 50 mM | 100 mM |
| pAX701 | *E. coli aroA* | ++ | - | - | - |
| pAX723 | *grg21* | ++ | +++ | +++ | +++ |
| pAX724 | *grg22* | ++ | +++ | +++ | +++ |

### Example 10: GRG23 contains a glyphosate-resistant EPSP synthase domain.

GRG23 (US Patent Application No. 60/741,166, filed December 1, 2005 and SEQ ID NO:14) was isolated from a bacterial strain exhibiting strong glyphosate resistance. GRG23 comprises an EPSP synthase domain that has an increased polarity in the Q-loop region relative to EPSP synthase enzymes not containing a domain referred to herein. This enzyme confers glyphosate tolerance to an organism transformed with an expression construct expressing GRG23.

### Example 11: Potential for proteins with combinations of domains.

The domains referred to herein do not overlap with respect to the previously defined Class II (US Patent No. 5,627,061) or Class III (US Patent Application No. 60/695,193, filed June 29, 2005) EPSP synthase domains. Thus, it is conceivable that a protein may exist in nature that would contain all or some elements of both the domains referred to herein and Class II or Class III domains (for example, the EPSP synthase derived from *Clostridium tetani* (Swissprot accession number Q894D2 and SEQ ID NO:28) contains both Class II and domains referred to herein

The presence of a domain referred to herein in an EPSP synthase enzyme is predictive of glyphosate resistance.

The presence of all or part of that domain is associated with an increase or enhancement in enzyme activity or function.

The domains identified herein may be engineered or recombined with the amino acid sequences of other enzymes, for example, by replacement of a Class I EPSP synthase motif of the *E. coli aroA* gene with a polypeptide having a Q-loop region with increased polarity, or with all or part of a domain referred to herein.

Alternatively, one or more domain(s) referred to herein may be inserted in replace of a polypeptide that does not comprise a domain referred to herein (including Class I and Class II EPSP synthase polypeptides), which may or may not comprise or result in improved properties.

### Example 12. Identification of additional novel EPSP synthase enzymes.

One can identify further glyphosate resistant EPSP synthases by searching databases containing EPSP synthase enzymes, and/or by alignment of the amino acid sequence of EPSP synthase enzymes and analysis for proteins containing a Q-loop region with increased polarity or domains referred to herein. It is understood that some modification of this Q-loop region or these domains is tolerated in nature without disrupting the glyphosate resistance conferring nature of these regions, and are therefore equivalent to the domains listed herein. Therefore, it is recognized that enzymes having about 90%, about 91%, about 92%, about 93%, about 94%, about 95%, about 96%, about 97%, about 98%, about 99% or greater homology to a domain could confer glyphosate tolerance.

It is now possible to generate further EPSP synthase enzymes with alterations in the Q-loop region that confer glyphosate resistance, in some instances without generating primary amino acid similarity to the specific domain residues described herein. For example, one may in general increase the polarity in the Q-loop region, and/or increase the bulkiness of the residues in this region, and achieve a similar glyphosate resistant EPSP synthase. Some of these alterations generated are likely to improve the glyphosate tolerance of the resulting protein, and are incorporated herein. Thus, the modification of EPSP synthase amino acid sequences to increase polarity, bulkiness, or to contain a domain listed herein is also referred to.

The domains identified herein may be engineered or recombined with the amino acid sequences of other EPSP synthase enzymes. For example, one of more of the domain sequences described herein may be inserted into an EPSP synthase sequence not containing a domain referred to herein.

The resulting proteins may have altered as well as improved properties.

### Example 13. Plant transformation by particle bombardment.

Maize ears are best collected 8-12 days after pollination. Embryos are isolated from the cars, and those embryos 0.8-1.5 mm in size are preferred for use in transformation. Embryos are plated scutellum side-up on a suitable incubation media, such as DN62A5S media (3.98 g/L N6 Salts; 1 ml/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 ml/L (of 1 mg/ml stock) 2,4-D). However, media and salts other than DN62A5S are suitable and are known in the art. Embryos are incubated overnight at 25°C in the dark. However, it is not necessary *per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to express EPSP synthase sequences having a Q-loop with an increased polarity or containing a domain referred to herein in plant cells are accelerated into plant tissue using an aerosol beam accelerator, using conditions essentially as described in PCT Publication No. WO/0138514. After beaming, embryos are incubated for about 30 min on osmotic media, and placed onto incubation media overnight at 25°C in the dark. To avoid unduly damaging beamed explants, they are incubated for at least 24 hours prior to transfer to recovery media. Embryos are then spread onto recovery period media, for about 5 days, 25°C in the dark, then transferred to a selection media. Explants are incubated in selection media for up to eight weeks, depending on the nature and characteristics of the particular selection utilized. After the selection period, the resulting callus is transferred to embryo maturation media until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants. The plants are assayed for improved resistance to glyphosate.

### Materials

| **DN62A5S Media** | | |
|---|---|---|
| **Components** | **per liter** | **Source** |
| Chu's N6 Basal Salt Mixture (Prod. No. C 416) | 3.98 g/L | Phytotechnology Labs |
| Chu's N6 Vitamin Solution (Prod. No. C 149) | 1 ml/L (of 1000x Stock) | Phytotechnology Labs |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 ml/L (of 1 mg/ml Stock) | Sigma |

Adjust the pH of the solution to pH 5.8 with 1N KOH/1N KCl, add Gelrite (Sigma) to 3g/L, and autoclave. After cooling to 50°C, add 2 ml/L of a 5 mg/ml stock solution of Silver Nitrate (Phytotechnology Labs). Recipe yields about 20 plates.

### Example 14. Transformation of Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors having a\n EPSP synthase enzyme with a Q-loop region with an increased polarity or a domain of the present invention for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art. The resulting shoots are allowed to root on rooting media, and the resulting plants are transferred to nursery pots and propagated as transgenic plants.

All publications and patent applications mentioned in the specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. A method of producing genetically transformed plants which are tolerant toward glyphosate herbicide, comprising the steps of:
a) inserting into the genome of a plant cell a polynucleotide encoding an EPSP synthase polypeptide having a Q-loop comprising D-C-X₁-X₂-S-G (SEQ ID NO:29), X₁ denoting glycine, serine, alanine or asparagine, X₂ denoting asparagine or glutamic acid, wherein said polypeptide has at least 95% sequence identity to SEQ ID NO:10, and wherein said polypeptide is resistant to glyphosate;
b) obtaining a transformed plant cell; and,
c) regenerating from the transformed plant cell a genetically transformed plant which has increased tolerance to glyphosate herbicide.

2. A glyphosate tolerant plant cell comprising a heterologous polynucleotide encoding an EPSP synthase polypeptide having a Q-loop comprising D-C-X₁-X₂-S-G (SEQ ID NO:29), X₁ denoting glycine, serine, alanine or asparagine, X₂ denoting asparagine or glutamic acid, wherein said polypeptide is resistant to glyphosate and wherein said polypeptide has at least 95% sequence identity to SEQ ID NO:10.

3. The glyphosate tolerant plant cell of claim 2 selected from the group consisting of corn, wheat, rice, barley, soybean, cotton, sugarbeet, oilseed rape, canola, flax, sunflower, potato, tobacco, tomato, alfalfa, poplar, pine, eucalyptus, apple, lettuce, peas, lentils, grape and turf grasses.

4. A glyphosate tolerant plant comprising the plant cell of claim 2-3.

5. A Transformed seed of the plant of claim 4, comprising the plant cell of claim 2-3.

6. The glyphosate tolerant plant of claim 4, wherein the plant is selected from the group consisting of corn, wheat, rice, barley, soybean, cotton, sugarbeet, oilseed rape, canola, flax, sunflower, potato, tobacco, tomato, alfalfa, poplar, pine, eucalyptus, apple, lettuce, peas, lentils, grape and turf grasses.

7. A method for selectively controlling weeds in a field containing a plant having planted seeds or plants comprising the steps of:
a) planting the seeds or plants which are glyphosate tolerant as a result of a polynucleotide encoding a glyphosate resistance EPSP synthase polypeptide having a Q loop comprising D-C-X₁-X₂-S-G (SEQ ID NO:29) being inserted into the seed or plant, X₁ denoting glycine, serine, alanine or asparagine, X₂ denoting asparagine or glutamic acid, wherein said polypeptide has at least 95% sequence identity to SEQ ID NO:10; and,
b) applying to the plants and weeds in a field an effective concentration of glyphosate herbicide to control weeds without significantly affecting the plants.

8. The glyphosate tolerant plant or plant cell of any of claims 2-6, or the method of claim 1 or 7 in which the polynucleotide encodes a fusion polypeptide comprising an amino terminal chloroplast transit peptide and the EPSP synthase enzyme.

## Patentansprüche

1. Verfahren zur Herstellung von genetisch transformierten Pflanzen, die gegen Glyphosat-Herbizid tolerant sind, umfassend die Schritte:
a) Inserieren in das Genom einer Pflanzenzelle ein Polynukleotid, das für ein EPSP-Synthase-Polypeptid codiert, das einen Q-Loop hat, der D-C-X₁-X₂-S-G (SEQ ID NO:29) umfasst, worin X₁ Glycin, Serin, Alanin oder Asparagin bezeichnet, X₂ Asparagin oder Glutaminsäure bezeichnet, wobei das Polypeptid wenigstens 95% Sequenzidentität zu SEQ ID NO:10 hat, und wobei das Polypeptid gegen Glyphosat resistent ist;
b) Erhalten einer transformierten Pflanzenzelle und
c) Regenerieren einer genetisch transformierten Pflanze, die erhöhte Toleranz gegen Glyphosat-Herbizid hat, aus der transformierten Pflanzenzelle.

2. Glyphosat-tolerante Pflanzenzelle, die ein heterologes Polynukleotid umfasst, das für ein EPSP-Synthase-Polypeptid codiert, welches einen Q-Loop hat, der D-C-X₁-X₂-S-G (SEQ ID NO:29) umfasst, worin X₁ Glycin, Serin, Alanin oder Asparagin bezeichnet, X₂ Asparagin oder Glutaminsäure bezeichnet, wobei das Polypeptid gegen Glyphosat resistent ist und wobei das Polypeptid wenigstens 95% Sequenzidentität zu SEQ ID NO:10 hat.

3. Glyphosat-tolerante Pflanzenzelle gemäß Anspruch 2, ausgewählt aus der Gruppe, bestehend aus Mais, Weizen, Reis, Gerste, Sojabohne, Baumwolle, Zuckerrübe, Ölsamenraps, Canola, Flachs, Sonnenblume, Kartoffel, Tabak, Tomate, Alfalfa, Pappel, Kiefer, Eukalyptus, Apfel, Salat, Erbsen, Linsen, Weintraube und Rasengräsern.

4. Glyphosat-tolerante Pflanze, die die Pflanzenzelle gemäß Anspruch 2-3 umfasst.

5. Transformierter Samen der Pflanze gemäß Anspruch 1, der die Pflanzenzelle gemäß Anspruch 2-3 umfasst.

6. Glyphosat-tolerante Pflanze gemäß Anspruch 4, wobei die Pflanze aus der Gruppe, bestehend aus Mais, Weizen, Reis Gerste, Sojabohne, Baumwolle, Zuckerrübe, Ölsamenraps, Canola, Flachs, Sonnenblume, Kartoffel, Tabak, Tomate, Alfalfa Pappel, Kiefer Eukalyptus, Apfel, Salat, Erbsen, Linsen, Weintraube und Rasengräsern, ausgewählt ist.

7. Verfahren zum selektiven Bekämpfen von Unkraut auf einem Feld, enthaltend eine Pflanze, aufweisend gepflanzte Samen oder Pflanzen, umfassend die Schritte:
a) Pflanzen der Samen oder Pflanzen, die Glyphosattolerant sind, und zwar als Resultat eines Polynukleotids, das für ein Glyphosatresistenz-EPSP-Synthase-Polypeptid codiert, das einen Q-Loop hat, der D-C-X₁-X₂-S-G (SEQ ID NO:29) umfasst, das in den Samen oder die Pflanze inseriert ist, wobei X₁ Glycin, Serin, Alanin oder Asparagin bezeichnet, X₂ Asparagin oder Glutaminsäure bezeichnet, wobei das Polypeptid wenigstens 95% Sequenzidentität zu SEQ ID NO:10 hat, und
b) Aufbringen auf die Pflanzen und Unkräuter auf dem Feld eine wirksame Konzentration an Glyphosat-Herbizid, um Unkräuter zu bekämpfen, ohne die Pflanzen merklich zu beeinträchtigen.

8. Glyphosat-tolerante Pflanze oder Pflanzenzelle gemäß einem der Ansprüche 2-6 oder Verfahren gemäß Anspruch 1 oder 7, wobei das Polynukleotid für ein Fusionspolypeptid codiert, das ein aminoterminales Chloroplastentransitpeptid und das EPSP-Synthaseenzym umfasst.

## Revendications

1. Procédé pour produire des plantes génétiquement transformées qui sont tolérantes à l'herbicide glyphosate, comprenant les étapes consistant à :
a) insérer dans le génome d'une cellule végétale un polynucléotide codant un polypeptide d'EPSP synthase ayant une boucle Q comprenant D-C-X₁-X₂-S-G (SEQ ID NO : 29), où X₁ désigne la glycine, la sérine, l'alanine ou l'asparagine, X₂ désigne l'asparagine ou l'acide glutamique, ledit polypeptide ayant une identité de séquence d'au moins 95 % avec la SEQ ID NO : 10, et ledit polypeptide étant résistant au glyphosate ;
b) obtenir une cellule végétale transformée ; et
c) régénérer à partir de la cellule végétale transformée une plante génétiquement transformée qui a une plus grande tolérance à l'herbicide glyphosate.

2. Cellule végétale tolérante au glyphosate comprenant un polynucléotide hétérologue codant un polypeptide d'EPSP synthase une boucle Q comprenant D-C-X₁-X₂-S-G (SEQ ID NO : 29), où X₁ désigne la glycine, la sérine, l'alanine ou l'asparagine, X₂ désigne l'asparagine ou l'acide glutamique, ledit polypeptide étant résistant au glyphosate et ledit polypeptide ayant une identité de séquence d'au moins 95 % avec la SEQ ID NO : 10.

3. Cellule végétale tolérante au glyphosate selon la revendication 2, choisie dans le groupe constitué par le maïs, le blé, le riz, l'orge, le soja, le coton, la betterave à sucre, le colza à huile, le canola, le lin, le tournesol, la pomme de terre, le tabac, la tomate, la luzerne, le peuplier, le pin, l'eucalyptus, le pommier, la laitue, les pois, les lentilles, le raisin et le gazon.

4. Plante tolérante au glyphosate comprenant la cellule végétale de la revendication 2 et 3.

5. Semence transformée de la plante selon la revendication 4, comprenant la cellule végétale de la revendication 2 et 3.

6. Plante tolérante au glyphosate selon la revendication 4, ladite plante étant choisie dans le groupe constitué par le maïs, le blé, le riz, l'orge, le soja, le coton, la betterave à sucre, le colza à huile, le canola, le lin, le tournesol, la pomme de terre, le tabac, la tomate, la luzerne, le peuplier, le pin, l'eucalyptus, le pommier, la laitue, les pois, les lentilles, le raisin et le gazon.

7. Procédé pour lutter sélectivement contre les mauvaises herbes dans un champ contenant une plante ayant des semences plantées ou des plantes, comprenant les étapes consistant à :
a) planter les semences ou plantes qui sont tolérantes au glyphosate en résultat d'un polynucléotide codant un polypeptide d'EPSP synthase de résistance au glyphosate ayant une boucle Q comprenant D-C-X₁-X₂-S-G (SEQ ID NO : 29) qui a été inséré dans la semence ou la plante, où X₁ désigne la glycine, la sérine, l'alanine ou l'asparagine, X₂ désigne l'asparagine ou l'acide glutamique, ledit polypeptide ayant une identité de séquence d'au moins 95 % avec la SEQ ID NO : 10 ; et
b) appliquer aux plantes et au mauvaises herbes dans un champ une concentration efficace d'herbicide glyphosate pour lutter contre les mauvaises herbes sans affecter significativement les plantes.

8. Plante ou cellule végétale tolérante au glyphosate selon l'une quelconque des revendications 2 à 6, ou procédé selon la revendication 1 ou 7, dans lequel le polynucléotide code un polypeptide de fusion comprenant un peptide de transit de chloroplaste amino-terminal et l'enzyme EPSP synthase.


### SEQUENCE LISTING

<110> Carr, Brian
   Hammer, Philip E.
   Hinson, Todd K.
   vande Berg, Brian
<120> EPSP Synthase Domains Conferring
   Glyphosate Resistance
<130> 45600/322326
<150> 60/758,320
   <151> 2006-01-12
<160> 52
<170> FastSEQ for windows Version 4.0
<210> 1
   <211> 1398
   <212> DNA
   <213> Enterobacteriaceae sp.
<220>
   <221> CDS
   <222> (103)...(1398)
<400> 1
   aaaaaaggaa atgaactatg tgttgctgga aaaagtaggg aagggagtgg tgaagagtat 60
   tccactggtt caattagaaa aaatcattca aggattacca aa gtg aaa gta aca    114
                                                  Val Lys Val Thr
                                                  1
   ata cag ccc gga gat ctg act gga att atc cag tca ccc gct tca aaa   162
   Ile Gln Pro Gly Asp Leu Thr Gly Ile Ile Gln Ser Pro Ala Ser Lys
   5                   10                  15                  20
   agt tcg atg cag cga gct tgt gct gct gca ctg gtt gca aaa gga ata   210
   Ser Ser Met Gln Arg Ala Cys Ala Ala Ala Leu Val Ala Lys Gly Ile
                   25                  30                  35
   agt gag atc att aat ccc ggt cat agc aat gat gat aaa gct gcc agg   258
   Ser Glu Ile Ile Asn Pro Gly His Ser Asn Asp Asp Lys Ala Ala Arg
               40                  45                  50
   gat att gta agc cgg ctt ggt gcc agg ctt gaa gat cag cct gat ggt   306
   Asp Ile Val Ser Arg Leu Gly Ala Arg Leu Glu Asp Gln Pro Asp Gly
           55                  60                  65
   tct ttg cag ata aca agt gaa ggc gta aaa cct gtc gct cct ttt att   354
   Ser Leu Gln Ile Thr Ser Glu Gly Val Lys Pro Val Ala Pro Phe Ile
       70                  75                  80
   gac tgc ggt gaa tct ggt tta agt atc cgg atg ttt act ccg att gtt   402
   Asp Cys Gly Glu Ser Gly Leu Ser Ile Arg Met Phe Thr Pro Ile Val
   85                  90                  95                  100
   gcg ttg agt aaa gaa gag gtg acg atc aaa gga tct gga agc ctt gtt   450
   Ala Leu Ser Lys Glu Glu Val Thr Ile Lys Gly Ser Gly Ser Leu Val
                   105                 110                 115
   aca aga cca atg gat ttc ttt gat gaa att ctt ccg cat ctc ggt gta   498
   Thr Arg Pro Met Asp Phe Phe Asp Glu Ile Leu Pro His Leu Gly Val
               120                 125                 130
   aaa gtt aaa tct aac cag ggt aaa ttg cct ctc gtt ata cag ggg cca   546
   Lys Val Lys Ser Asn Gln Gly Lys Leu Pro Leu Val Ile Gln Gly Pro
           135                 140                 145
   ttg aaa cca gca gac gtt acg gtt gat ggg tcc tta agc tct cag ttc     594
   Leu Lys Pro Ala Asp Val Thr Val Asp Gly Ser Leu Ser Ser Gln Phe
       150                 155                 160
   ctt aca ggt ttg ttg ctt gca tat gcg gcc gca gat gca agc gat gtt     642
   Leu Thr Gly Leu Leu Leu Ala Tyr Ala Ala Ala Asp Ala Ser Asp Val
   165                 170                 175                 180
   gcg ata aaa gta acg aat ctc aaa agc cgt ccg tat atc gat ctt aca     690
   Ala Ile Lys Val Thr Asn Leu Lys Ser Arg Pro Tyr Ile Asp Leu Thr
                   185                 190                 195
   ctg gat gtg atg aag cgg ttt ggt ttg aag act ccc gag aat cga aac     738
   Leu Asp Val Met Lys Arg Phe Gly Leu Lys Thr Pro Glu Asn Arg Asn
               200                 205                 210
   tat gaa gag ttt tat ttc aaa gcc ggg aat gta tat gat gaa acg aaa     786
   Tyr Glu Glu Phe Tyr Phe Lys Ala Gly Asn Val Tyr Asp Glu Thr Lys
           215                 220                 225
   atg caa cga tac acc gta gaa ggc gac tgg agc ggt ggt gct ttt tta     834
   Met Gln Arg Tyr Thr Val Glu Gly Asp Trp Ser Gly Gly Ala Phe Leu
       230                 235                 240
   ctg gta gcg ggg gct att gcc ggg ccg atc acg gta aga ggt ttg gat     882
   Leu Val Ala Gly Ala Ile Ala Gly Pro Ile Thr Val Arg Gly Leu Asp
   245                 250                 255                 260
   ata gct tcg acg cag gct gat aaa gcg atc gtt cag gct ttg atg agt     930
   Ile Ala Ser Thr Gln Ala Asp Lys Ala Ile Val Gln Ala Leu Met Ser
                   265                 270                 275
   gcg aac gca ggt att gcg att gat gca aaa gag atc aaa ctt cat cct     978
   Ala Asn Ala Gly Ile Ala Ile Asp Ala Lys Glu Ile Lys Leu His Pro
               280                 285                 290
   gct gat ctc aat gca ttt gaa ttt gat gct act gat tgc ccg gat ctt    1026
   Ala Asp Leu Asn Ala Phe Glu Phe Asp Ala Thr Asp Cys Pro Asp Leu
           295                 300                 305
   ttt ccg cca ttg gtt gct ttg gcg tct tat tgc aaa gga gaa aca aag    1074
   Phe Pro Pro Leu Val Ala Leu Ala Ser Tyr Cys Lys Gly Glu Thr Lys
       310                 315                 320
   atc aaa ggc gta agc agg ctg gcg cat aaa gaa agt gac aga gga ttg    1122
   Ile Lys Gly Val Ser Arg Leu Ala His Lys Glu Ser Asp Arg Gly Leu
   325                 330                 335                 340
   acg ctg cag gac gag ttc ggg aaa atg ggt gtt gaa atc cac ctt gag    1170
   Thr Leu Gln Asp Glu Phe Gly Lys Met Gly Val Glu Ile His Leu Glu
                   345                 350                 355
   gga gat ctg atg cgc gtg atc gga ggg aaa ggc gta aaa gga gct gaa    1218
   Gly Asp Leu Met Arg Val Ile Gly Gly Lys Gly Val Lys Gly Ala Glu
               360                 365                 370
   gtt agt tca agg cac gat cat cgc att gcg atg gct tgc gcg gtg gct    1266
   Val Ser Ser Arg His Asp His Arg Ile Ala Met Ala Cys Ala Val Ala
           375                 380                 385
   gct tta aaa gct gtg ggt gaa aca acc atc gaa cat gca gaa gcg gtg    1314
   Ala Leu Lys Ala Val Gly Glu Thr Thr Ile Glu His Ala Glu Ala Val
       390                 395                 400
   aat aaa tcc tac ccg gat ttt tac agc gat ctt aaa caa ctt ggc ggt    1362
   Asn Lys Ser Tyr Pro Asp Phe Tyr Ser Asp Leu Lys Gln Leu Gly Gly
   405                 410                 415                 420
   gtt gta tct tta aac cat caa ttt aat ttc tca tga                     1398
   Val Val Ser Leu Asn His Gln Phe Asn Phe Ser  *
                   425                 430
<210> 2
   <211> 431
   <212> PRT
   <213> Enterobacteriaceae sp.
<400> 2
   Val Lys Val Thr Ile Gln Pro Gly Asp Leu Thr Gly Ile Ile Gln Ser
   1 5 10 15
   Pro Ala Ser Lys Ser Ser Met Gln Arg Ala Cys Ala Ala Ala Leu Val
   20 25 30
   Ala Lys Gly Ile Ser Glu Ile Ile Asn Pro Gly His Ser Asn Asp Asp
   35 40 45
   Lys Ala Ala Arg Asp Ile Val Ser Arg Leu Gly Ala Arg Leu Glu Asp
   50 55 60
   Gln Pro Asp Gly Ser Leu Gln Ile Thr Ser Glu Gly Val Lys Pro Val
   65 70 75 80
   Ala Pro Phe Ile Asp Cys Gly Glu Ser Gly Leu Ser Ile Arg Met Phe
   85 90 95
   Thr Pro Ile Val Ala Leu Ser Lys Glu Glu Val Thr Ile Lys Gly Ser
   100 105 110
   Gly Ser Leu Val Thr Arg Pro Met Asp Phe Phe Asp Glu Ile Leu Pro
   115 120 125
   His Leu Gly Val Lys Val Lys Ser Asn Gln Gly Lys Leu Pro Leu Val
   130 135 140
   Ile Gln Gly Pro Leu Lys Pro Ala Asp Val Thr Val Asp Gly Ser Leu
   145 150 155 160
   Ser Ser Gln Phe Leu Thr Gly Leu Leu Leu Ala Tyr Ala Ala Ala Asp
   165 170 175
   Ala Ser Asp Val Ala Ile Lys Val Thr Asn Leu Lys Ser Arg Pro Tyr
   180 185 190
   Ile Asp Leu Thr Leu Asp Val Met Lys Arg Phe Gly Leu Lys Thr Pro
   195 200 205
   Glu Asn Arg Asn Tyr Glu Glu Phe Tyr Phe Lys Ala Gly Asn Val Tyr
   210 215 220
   Asp Glu Thr Lys Met Gln Arg Tyr Thr Val Glu Gly Asp Trp Ser Gly
   225 230 235 240
   Gly Ala Phe Leu Leu Val Ala Gly Ala Ile Ala Gly Pro Ile Thr Val
   245 250 255
   Arg Gly Leu Asp Ile Ala Ser Thr Gln Ala Asp Lys Ala Ile Val Gln
   260 265 270
   Ala Leu Met Ser Ala Asn Ala Gly Ile Ala Ile Asp Ala Lys Glu Ile
   275 280 285
   Lys Leu His Pro Ala Asp Leu Asn Ala Phe Glu Phe Asp Ala Thr Asp
   290 295 300
   Cys Pro Asp Leu Phe Pro Pro Leu Val Ala Leu Ala Ser Tyr Cys Lys
   305 310 315 320
   Gly Glu Thr Lys Ile Lys Gly Val Ser Arg Leu Ala His Lys Glu Ser
   325 330 335
   Asp Arg Gly Leu Thr Leu Gln Asp Glu Phe Gly Lys Met Gly Val Glu
   340 345 350
   Ile His Leu Glu Gly Asp Leu Met Arg Val Ile Gly Gly Lys Gly Val
   355 360 365
   Lys Gly Ala Glu Val Ser Ser Arg His Asp His Arg Ile Ala Met Ala
   370 375 380
   Cys Ala Val Ala Ala Leu Lys Ala Val Gly Glu Thr Thr Ile Glu His
   385 390 395 400
   Ala Glu Ala Val Asn Lys Ser Tyr Pro Asp Phe Tyr Ser Asp Leu Lys
   405 410 415
   Gln Leu Gly Gly Val Val Ser Leu Asn His Gln Phe Asn Phe Ser
   420 425 430
<210> 3
   <211> 1275
   <212> DNA
   <213> Clostridium perfringens
<400> 3
   gtgaaaaagg taattataac tcctagtaag ttaaggggaa gtgtaaaaat accaccttct 60
   aaaagtatgg ctcatagagc tattatttgt gcttctttaa gcaaaggaga aagtgttatt 120
   tctaacatag atttttcaga agatattatt gcaactatgg aaggtatgaa atctttagga 180
   gcaaatataa aagtagaaaa agataaacta attataaatg gagaaaatat tttaaaggat 240
   tctaattata aatttattga ttgtaatgaa tcaggttcca ctttaagatt tttagttcca 300
   atttccttaa taaaagataa tagagttaat tttatcggta gaggaaattt agggaaaaga 360
   ccattaaaaa cttattatga gatttttgag gagcaagaaa ttaagtattc ctatgaggaa 420
   gaaaatcttg atttgaatat agaaggaagc ttaaaaggtg gagaattcaa agttaaggga 480
   aatataagtt ctcaatttat aagtggttta ttatttactc ttcctttatt aaaagatgat 540
   tctaaaataa taataactac agaacttgaa tctaaaggat atatagattt aactttagac 600
   atgatagaaa agtttggagt tacaataaaa aataataatt atagagaatt tttaataaaa 660
   ggtaatcaaa gttataagcc tatgaattat aaggttgaag gtgattactc acaggctgct 720
   ttctattttt cagcaggggc cttaggctca gaaataaatt gtcttgattt agatttaagt 780
   tcttatcaag gagataagga atgcattgaa atattagagg gtatgggtgc taggcttata 840
   gaaagtcaag aaaggtcttt aagtataatt catggggatt taaatggaac aattatagat 900
   gcttcacaat gcccagatat aattcctgtt ttgacagtgg ttgctgcttt aagtaaggga 960
   gagactagga ttataaatgg agaaagactt agaataaaag aatgtgatag attaaatgct 1020
   atatgtacag agcttaataa actaggtgca gatataaagg aattaaaaga tggacttata 1080
   ataaatggag ttaaagattt aataggagga gaagtatata gccataaaga tcatagaata 1140
   gctatgagtt tggctattgc ttctacaaga tgcaagaaag aggttattat aaaagaacca 1200
   gattgtgtta aaaaatctta tccaggattt tgggaagatt ttaagagctt aggtggaatt 1260
   ttaagagaag aataa                                                  1275
<210> 4
   <211> 424
   <212> PRT
   <213> clostridium perfringens
<400> 4
   Met Lys Lys Val Ile Ile Thr Pro Ser Lys Leu Arg Gly Ser Val Lys
   1               5                   10                  15
   Ile Pro Pro Ser Lys Ser Met Ala His Arg Ala Ile Ile Cys Ala Ser
               20                  25                  30
   Leu Ser Lys Gly Glu Ser Val Ile Ser Asn Ile Asp Phe Ser Glu Asp
           35                  40                  45
   Ile Ile Ala Thr Met Glu Gly Met Lys Ser Leu Gly Ala Asn Ile Lys
       50                  55                  60
   Val Glu Lys Asp Lys Leu Ile Ile Asn Gly Glu Asn Ile Leu Lys Asp
   65                  70                  75                  80
   Ser Asn Tyr Lys Phe Ile Asp Cys Asn Glu Ser Gly Ser Thr Leu Arg
                   85                  90                  95
   Phe Leu Val Pro Ile Ser Leu Ile Lys Asp Asn Arg Val Asn Phe Ile
               100                 105                 110
   Gly Arg Gly Asn Leu Gly Lys Arg Pro Leu Lys Thr Tyr Tyr Glu Ile
           115                 120                 125
   Phe Glu Glu Gln Glu Ile Lys Tyr Ser Tyr Glu Glu Glu Asn Leu Asp
       130                 135                 140
   Leu Asn Ile Glu Gly Ser Leu Lys Gly Gly Glu Phe Lys Val Lys Gly
   145                 150                 155                 160
   Asn Ile Ser Ser Gln Phe Ile Ser Gly Leu Leu Phe Thr Leu Pro Leu
                   165                 170                 175
   Leu Lys Asp Asp Ser Lys Ile Ile Ile Thr Thr Glu Leu Glu Ser Lys
               180                 185                 190
   Gly Tyr Ile Asp Leu Thr Leu Asp Met Ile Glu Lys Phe Gly Val Thr
           195                 200                 205
   Ile Lys Asn Asn Asn Tyr Arg Glu Phe Leu Ile Lys Gly Asn Gln Ser
       210                 215                 220
   Tyr Lys Pro Met Asn Tyr Lys Val Glu Gly Asp Tyr Ser Gln Ala Ala
   225                 230                 235                 240
   Phe Tyr Phe Ser Ala Gly Ala Leu Gly Ser Glu Ile Asn Cys Leu Asp
                           245                     250                     255
   Leu Asp Leu Ser Ser Tyr Gln Gly Asp Lys Glu Cys Ile Glu Ile Leu
               260                     265                     270
   Glu Gly Met Gly Ala Arg Leu Ile Glu Ser Gln Glu Arg Ser Leu Ser
           275                     280                     285
   Ile Ile His Gly Asp Leu Asn Gly Thr Ile Ile Asp Ala Ser Gln Cys
       290                     295                     300
   Pro Asp Ile Ile Pro Val Leu Thr Val Val Ala Ala Leu Ser Lys Gly
   305                     310                     315                     320
   Glu Thr Arg Ile Ile Asn Gly Glu Arg Leu Arg Ile Lys Glu Cys Asp
                   325                     330                     335
   Arg Leu Asn Ala Ile Cys Thr Glu Leu Asn Lys Leu Gly Ala Asp Ile
               340                     345                     350
   Lys Glu Leu Lys Asp Gly Leu Ile Ile Asn Gly Val Lys Asp Leu Ile
           355                     360                     365
   Gly Gly Glu Val Tyr Ser His Lys Asp His Arg Ile Ala Met Ser Leu
       370                     375                     380
   Ala Ile Ala Ser Thr Arg Cys Lys Lys Glu Val Ile Ile Lys Glu Pro
   385                     390                     395                     400
   Asp Cys Val Lys Lys Ser Tyr Pro Gly Phe Trp Glu Asp Phe Lys Ser
                   405                     410                     415
   Leu Gly Gly Ile Leu Arg Glu Glu
               420
<210> 5
   <211> 1398
   <212> DNA
   <213> Clostridium acetobutylicum
<400> 5
   aaaaaaggaa atgaactatg tgttgctgga aaaagtaggg aagggagtgg tgaagagtat 60
   tccactggtt caattagaaa aaatcattca aggattacca aagtgaaagt aacaatacag 120
   cccggagatc tgactggaat tatccagtca cccgcttcaa aaagttcgat gcagcgagct 180
   tgtgctgctg cactggttgc aaaaggaata agtgagatca ttaatcccgg tcatagcaat 240
   gatgataaag ctgccaggga tattgtaagc cggcttggtg ccaggcttga agatcagcct 300
   gatggttctt tgcagataac aagtgaaggc gtaaaacctg tcgctccttt tattgactgc 360
   ggtgaatctg gtttaagtat ccggatgttt actccgattg ttgcgttgag taaagaagag 420
   gtgacgatca aaggatctgg aagccttgtt acaagaccaa tggatttctt tgatgaaatt 480
   cttccgcatc tcggtgtaaa agttaaatct aaccagggta aattgcctct cgttatacag 540
   gggccattga aaccagcaga cgttacggtt gatgggtcct taagctctca gttccttaca 600
   ggtttgttgc ttgcatatgc ggccgcagat gcaagcgatg ttgcgataaa agtaacgaat 660
   ctcaaaagcc gtccgtatat cgatcttaca ctggatgtga tgaagcggtt tggtttgaag 720
   actcccgaga atcgaaacta tgaagagttt tatttcaaag ccgggaatgt atatgatgaa 780
   acgaaaatgc aacgatacac cgtagaaggc gactggagcg gtggtgcttt tttactggta 840
   gcgggggcta ttgccgggcc gatcacggta agaggtttgg atatagcttc gacgcaggct 900
   gataaagcga tcgttcaggc tttgatgagt gcgaacgcag gtattgcgat tgatgcaaaa 960
   gagatcaaac ttcatcctgc tgatctcaat gcatttgaat ttgatgctac tgattgcccg 1020
   gatctttttc cgccattggt tgctttggcg tcttattgca aaggagaaac aaagatcaaa 1080
   ggcgtaagca ggctggcgca taaagaaagt gacagaggat tgacgctgca ggacgagttc 1140
   gggaaaatgg gtgttgaaat ccaccttgag ggagatctga tgcgcgtgat cggagggaaa 1200
   ggcgtaaaag gagctgaagt tagttcaagg cacgatcatc gcattgcgat ggcttgcgcg 1260
   gtggctgctt taaaagctgt gggtgaaaca accatcgaac atgcagaagc ggtgaataaa 1320
   tcctacccgg atttttacag cgatcttaaa caacttggcg gtgttgtatc tttaaaccat 1380
   caatttaatt tctcatga                                                1398
<210> 6
   <211> 428
   <212> PRT
   <213> clostridium acetobutylicum
<400> 6
   Met Asn Cys Val Lys Ile Asn Pro Cys Cys Leu Lys Gly Asp Ile Lys
   1 5 10 15
   Ile Pro Pro Ser Lys Ser Leu Gly His Arg Ala Ile Ile Cys Ala Ala
   20 25 30
   Leu Ser Glu Glu Glu Ser Thr Ile Glu Asn Ile Ser Tyr Ser Lys Asp
   35 40 45
   Ile Lys Ala Thr Cys Ile Gly Met Ser Lys Leu Gly Ala Leu Ile Ile
   50 55 60
   Glu Asp Ala Lys Asp Asn Ser Thr Leu Lys Ile Lys Lys Gln Lys Leu
   65 70 75 80
   Val Ser Lys Glu Lys Val Tyr Ile Asp Cys Ser Glu Ser Gly Ser Thr
   85 90 95
   Val Arg Phe Leu Ile Pro Ile Ser Leu Ile Glu Glu Arg Asn Val Val
   100 105 110
   Phe Asp Gly Gln Gly Lys Leu Ser Tyr Arg Pro Leu Asp Ser Tyr Phe
   115 120 125
   Asn Ile Phe Asp Glu Lys Glu Ile Ala Tyr Ser His Pro Glu Gly Lys
   130 135 140
   Val Leu Pro Leu Gln Ile Lys Gly Arg Leu Lys Ala Gly Met Phe Asn
   145 150 155 160
   Leu Pro Gly Asn Ile Ser Ser Gln Phe Ile Ser Gly Leu Met Phe Ser
   165 170 175
   Leu Pro Phe Leu Glu Gly Asp Ser Ile Ile Asn Ile Thr Thr Asn Leu
   180 185 190
   Glu Ser Val Gly Tyr Val Asp Met Thr Ile Asp Met Leu Lys Lys Phe
   195 200 205
   Gly Ile Glu Ile Glu Asn Lys Ala Tyr Lys Ser Phe Phe Ile Lys Gly
   210 215 220
   Asn Gln Lys Cys Lys Gly Thr Lys Tyr Lys Val Glu Gly Asp Phe Ser
   225 230 235 240
   Gln Ala Ala Phe Trp Leu Ser Ala Gly Ile Leu Asn Gly Asn Ile Asn
   245 250 255
   Cys Lys Asp Leu Asn Ile Ser Ser Leu Gln Gly Asp Lys Val Ile Leu
   260 265 270
   Asp Ile Leu Lys Lys Met Gly Gly Ala Ile Asp Glu Lys Ser Phe Ser
   275 280 285
   Ser Lys Lys Ser His Thr His Gly Ile Val Ile Asp Ala Ser Gln Cys
   290 295 300
   Pro Asp Leu Val Pro Ile Leu Ser Val Val Ala Ala Leu Ser Glu Gly
   305 310 315 320
   Thr Thr Lys Ile Val Asn Ala Ala Arg Leu Arg Ile Lys Glu Ser Asp
   325 330 335
   Arg Leu Lys Ala Met Ala Thr Glu Leu Asn Lys Leu Gly Ala Glu Val
   340 345 350
   Val Glu Leu Glu Asp Gly Leu Leu Ile Glu Gly Lys Glu Lys Leu Lys
   355 360 365
   Gly Gly Glu Val Glu Ser Trp Asn Asp His Arg Ile Ala Met Ala Leu
   370 375 380
   Gly Ile Ala Ala Leu Arg Cys Glu Glu Ser Val Thr Ile Asn Gly Ser
   385 390 395 400
   Glu Cys Val Ser Lys Ser Tyr Pro Gln Phe Trp Ser Asp Leu Lys Gln
   405 410 415
   Leu Gly Gly Asp Val His Glu Trp Ser Leu Gly Glu
   420 425
<210> 7
   <211> 1309
   <212> DNA
   <213> Artificial Sequence
<220>
   <223> Backtranslated from a protein isolated from
   Fusobacterium nucleatum
<221> CDS
   <222> (10)...(1284)
<221> misc_feature
   <222> (0)...(0)
   <223> synFuso II
<400> 7
   ggatccggc atg agg aac atg aac aag aag atc atc aag gcg gat aag ctc 51
                   Met Arg Asn Met Asn Lys Lys Ile Ile Lys Ala Asp Lys Leu
                    1               5                   10
   gtc ggc gag gtc acc ccc ccc ccc agc aag tca gtc ctg cat cgt tac     99
   Val Gly Glu Val Thr Pro Pro Pro Ser Lys Ser Val Leu His Arg Tyr
    15                  20                  25                  30
   atc atc gcc tcc agc ctg gcg aag ggt atc tcc aag atc gag aac atc    147
   Ile Ile Ala Ser Ser Leu Ala Lys Gly Ile Ser Lys Ile Glu Asn Ile
                    35                  40                  45
   agc tac tcc gat gat atc atc gcc acc atc gag gcg atg aag aag ctg    195
   Ser Tyr Ser Asp Asp Ile Ile Ala Thr Ile Glu Ala Met Lys Lys Leu
                50                  55                  60
   ggc gcc aac atc gag aag aag gat aac tac ctc ctg atc gat ggc agc    243
   Gly Ala Asn Ile Glu Lys Lys Asp Asn Tyr Leu Leu Ile Asp Gly Ser
            65                  70                  75
   aag acc ttc gat aag gag tac ctc aac aac gat tca gag atc gat tgc    291
   Lys Thr Phe Asp Lys Glu Tyr Leu Asn Asn Asp Ser Glu Ile Asp Cys
        80                  85                  90
   aac gag tcc ggc agc acc ctg cgc ttc ctc ttc ccc ctg agc atc gtc    339
   Asn Glu Ser Gly Ser Thr Leu Arg Phe Leu Phe Pro Leu Ser Ile Val
    95                 100                 105                 110
   aag gag aac aag atc ctg ttc aag ggc aag ggc aag ctg ttc aag cgc    387
   Lys Glu Asn Lys Ile Leu Phe Lys Gly Lys Gly Lys Leu Phe Lys Arg
                   115                 120                 125
   ccc ctc tcc ccc tac ttc gag aac ttc gat aag tac cag atc aag tgc    435
   Pro Leu Ser Pro Tyr Phe Glu Asn Phe Asp Lys Tyr Gln Ile Lys Cys
               130                 135                 140
   agc agc atc aac gag aac aag atc ctc ctg gat ggc gag ctc aag tca    483
   Ser Ser Ile Asn Glu Asn Lys Ile Leu Leu Asp Gly Glu Leu Lys Ser
           145                 150                 155
   ggc gtc tac gag atc gat ggc aac atc agc agc cag ttc atc acc ggc    531
   Gly Val Tyr Glu Ile Asp Gly Asn Ile Ser Ser Gln Phe Ile Thr Gly
       160                 165                 170
   ctg ctc ttc agc ctc ccc ctg ctc aac ggc aac tcc aag atc atc atc    579
   Leu Leu Phe Ser Leu Pro Leu Leu Asn Gly Asn Ser Lys Ile Ile Ile
   175                 180                 185                 190
   aag ggc aag ctc gag agc agc tcc tac atc gat atc acc ctt gat tgc    627
   Lys Gly Lys Leu Glu Ser Ser Ser Tyr Ile Asp Ile Thr Leu Asp Cys
                   195                 200                 205
   ctg aac aag ttc ggc atc aac atc atc aac aac tca tac aag gag ttc    675
   Leu Asn Lys Phe Gly Ile Asn Ile Ile Asn Asn Ser Tyr Lys Glu Phe
               210                 215                 220
   atc atc gag ggc aac cag acc tac aag tcc ggc aac tac cag gtc gag    723
   Ile Ile Glu Gly Asn Gln Thr Tyr Lys Ser Gly Asn Tyr Gln Val Glu
           225                 230                 235
   gcg gat tac agc cag gtc gcc ttc ttc ctg gtc gcc aac tcc atc ggc    771
   Ala Asp Tyr Ser Gln Val Ala Phe Phe Leu Val Ala Asn Ser Ile Gly
       240                 245                 250
   tcc aac atc aag atc aac ggc ctc aac gtc aac tcc ctc cag ggc gat    819
   Ser Asn Ile Lys Ile Asn Gly Leu Asn Val Asn Ser Leu Gln Gly Asp
   255                 260                 265                 270
   aag aag atc atc gat ttc atc tca gag atc gat aac tgg acc aag aac    867
   Lys Lys Ile Ile Asp Phe Ile Ser Glu Ile Asp Asn Trp Thr Lys Asn
                   275                 280                 285
   gag aag ctg atc ctc gat ggc agc gag acc ccc gat atc atc ccc atc    915
   Glu Lys Leu Ile Leu Asp Gly Ser Glu Thr Pro Asp Ile Ile Pro Ile
               290                 295                 300
   ctg agc ctc aag gcg tgc atc agc aag aag gag atc gag atc gtc aac    963
   Leu Ser Leu Lys Ala Cys Ile Ser Lys Lys Glu Ile Glu Ile Val Asn
           305                 310                 315
   atc gcc cgc ctc cgc atc aag gag tcc gat cgc ctg tca gcg acc gtt   1011
   Ile Ala Arg Leu Arg Ile Lys Glu Ser Asp Arg Leu Ser Ala Thr Val
       320                 325                 330
   caa gag ctc tcc aag ctc ggc ttc gat ctg atc gag aag gag gat tcc   1059
   Gln Glu Leu Ser Lys Leu Gly Phe Asp Leu Ile Glu Lys Glu Asp Ser
   335                 340                 345                 350
   atc ctg atc aac tcc cgc aag aac ttc aac gag atc agc aac aac tcc   1107
   Ile Leu Ile Asn Ser Arg Lys Asn Phe Asn Glu Ile Ser Asn Asn Ser
                   355                 360                 365
   ccc atc agc ctc agc tca cat agc gat cat cgt atc gcc atg acc gtc   1155
   Pro Ile Ser Leu Ser Ser His Ser Asp His Arg Ile Ala Met Thr Val
               370                 375                 380
   gcc atc gcg tcc acc tgc tac gag ggc gag atc atc ctg gat aac ctc   1203
   Ala Ile Ala Ser Thr Cys Tyr Glu Gly Glu Ile Ile Leu Asp Asn Leu
           385                 390                 395
   gat tgc gtc aag aag agc tac cct aac ttc tgg gag gtt ttc ctc agc   1251
   Asp Cys Val Lys Lys Ser Tyr Pro Asn Phe Trp Glu Val Phe Leu Ser
       400                 405                 410
   ctg ggc ggc aag atc tac gag tac ctc ggc tga ggcgcgcctg caggtcgaca 1304
   Leu Gly Gly Lys Ile Tyr Glu Tyr Leu Gly  *
   415                 420
   agctt                                                             1309
<210> 8
   <211> 424
   <212> PRT
   <213> Fusobacterium nucleatum
<400> 8
   Met Arg Asn Met Asn Lys Lys Ile Ile Lys Ala Asp Lys Leu Val Gly
    1               5                  10                  15
   Glu Val Thr Pro Pro Pro Ser Lys Ser Val Leu His Arg Tyr Ile Ile
               20                  25                  30
   Ala Ser Ser Leu Ala Lys Gly Ile Ser Lys Ile Glu Asn Ile Ser Tyr
           35                  40                  45
   Ser Asp Asp Ile Ile Ala Thr Ile Glu Ala Met Lys Lys Leu Gly Ala
       50                  55                  60
   Asn Ile Glu Lys Lys Asp Asn Tyr Leu Leu Ile Asp Gly Ser Lys Thr
   65                  70                  75                  80
   Phe Asp Lys Glu Tyr Leu Asn Asn Asp Ser Glu Ile Asp Cys Asn Glu
                   85                  90                  95
   Ser Gly Ser Thr Leu Arg Phe Leu Phe Pro Leu Ser Ile Val Lys Glu
               100                 105                 110
   Asn Lys Ile Leu Phe Lys Gly Lys Gly Lys Leu Phe Lys Arg Pro Leu
           115                 120                 125
   Ser Pro Tyr Phe Glu Asn Phe Asp Lys Tyr Gln Ile Lys Cys Ser Ser
       130                 135                 140
   Ile Asn Glu Asn Lys Ile Leu Leu Asp Gly Glu Leu Lys Ser Gly Val
   145                 150                 155                 160
   Tyr Glu Ile Asp Gly Asn Ile Ser Ser Gln Phe Ile Thr Gly Leu Leu
                   165                 170                 175
   Phe Ser Leu Pro Leu Leu Asn Gly Asn Ser Lys Ile Ile Ile Lys Gly
               180                 185                 190
   Lys Leu Glu Ser Ser Ser Tyr Ile Asp Ile Thr Leu Asp Cys Leu Asn
           195                 200                 205
   Lys Phe Gly Ile Asn Ile Ile Asn Asn Ser Tyr Lys Glu Phe Ile Ile
       210                 215                 220
   Glu Gly Asn Gln Thr Tyr Lys Ser Gly Asn Tyr Gln Val Glu Ala Asp
   225                 230                 235                 240
   Tyr Ser Gln Val Ala Phe Phe Leu Val Ala Asn Ser Ile Gly Ser Asn
                   245                 250                 255
   Ile Lys Ile Asn Gly Leu Asn Val Asn Ser Leu Gln Gly Asp Lys Lys
               260                 265                 270
   Ile Ile Asp Phe Ile Ser Glu Ile Asp Asn Trp Thr Lys Asn Glu Lys
           275                 280                 285
   Leu Ile Leu Asp Gly Ser Glu Thr Pro Asp Ile Ile Pro Ile Leu Ser
       290                 295                 300
   Leu Lys Ala Cys Ile Ser Lys Lys Glu Ile Glu Ile Val Asn Ile Ala
   305                 310                 315                 320
   Arg Leu Arg Ile Lys Glu Ser Asp Arg Leu Ser Ala Thr Val Gln Glu
                   325                 330                 335
   Leu Ser Lys Leu Gly Phe Asp Leu Ile Glu Lys Glu Asp Ser Ile Leu
               340                 345                 350
   Ile Asn Ser Arg Lys Asn Phe Asn Glu Ile Ser Asn Asn Ser Pro Ile
           355                 360                 365
   Ser Leu Ser Ser His Ser Asp His Arg Ile Ala Met Thr Val Ala Ile
       370                 375                 380
   Ala Ser Thr Cys Tyr Glu Gly Glu Ile Ile Leu Asp Asn Leu Asp Cys
   385                 390                 395                 400
   Val Lys Lys Ser Tyr Pro Asn Phe Trp Glu Val Phe Leu Ser Leu Gly
                   405                 410                 415
   Gly Lys Ile Tyr Glu Tyr Leu Gly
               420
<210> 9
   <211> 1321
   <212> DNA
   <213> Artificial sequence
<220>
   <223> Backtranslated from a protein isolated from
   Methanopyrus kandleri
<221> CDS
   <222> (10)...(1296)
<221> misc_feature
   <222> (0)...(0)
   <223> synMeth II
<400> 9
   ggatccggc atg aag cgc gtc gag ttg gag ggt atc cct gag gtc cgt ggt 51
             Met Lys Arg Val Glu Leu Glu Gly Ile Pro Glu Val Arg Gly
              1               5                  10
   act gtc tgc cct cct cct tct aag agc ggt tct cat cgc gcc ttg atc   99
   Thr Val Cys Pro Pro Pro Ser Lys Ser Gly Ser His Arg Ala Leu Ile
    15                  20                  25                  30
   gcc gcg tct ttg tgc gat ggt tca acc gag ttg tgg aac gtc ctg gat   147
   Ala Ala Ser Leu Cys Asp Gly Ser Thr Glu Leu Trp Asn Val Leu Asp
                    35                  40                  45
   gcg gag gat gtc cgt gcg act ttg cgt ctg tgc cgt atg ttg ggt gcc   195
   Ala Glu Asp Val Arg Ala Thr Leu Arg Leu Cys Arg Met Leu Gly Ala
                50                  55                  60
   gag gtt gat gtc gat ggt gag gag cgt ttg gag gcg act gtt tcc ggt 243
   Glu Val Asp Val Asp Gly Glu Glu Arg Leu Glu Ala Thr Val Ser Gly
   65 70 75
   ttc ggt gat tcc ccc cgt gcg cct gag gat gtt gtt gat tgc ggc aac 291
   Phe Gly Asp Ser Pro Arg Ala Pro Glu Asp Val Val Asp Cys Gly Asn
   80 85 90
   agc ggt acc acc ttg agg ctc ggt tgc ggt ttg gcg gcc ttg gtt gag 339
   Ser Gly Thr Thr Leu Arg Leu Gly Cys Gly Leu Ala Ala Leu Val Glu
   95 100 105 110
   ggt act act atc ctc acc ggt gat gat agc ctc cgt tcc agg cct gtt 387
   Gly Thr Thr Ile Leu Thr Gly Asp Asp Ser Leu Arg Ser Arg Pro Val
   115 120 125
   ggt gat ctg ctg gcc gcc ttg cgt tca ttg ggt gtt gat gcc cgt ggt 435
   Gly Asp Leu Leu Ala Ala Leu Arg Ser Leu Gly Val Asp Ala Arg Gly
   130 135 140
   cgt gtt gtt cgt ggt gag gag tac cct cct gtt gtc atc agc ggt agg 483
   Arg Val Val Arg Gly Glu Glu Tyr Pro Pro Val Val Ile Ser Gly Arg
   145 150 155
   cct ctg agg gag agg gtt gcg gtt tac ggt gat gtc tcc tct cag ttc 531
   Pro Leu Arg Glu Arg Val Ala Val Tyr Gly Asp Val Ser Ser Gln Phe
   160 165 170
   gtc agc gcc ttg ctg ttc ctg ggt gcg ggt ttg ggt gcc ttg agg gtt 579
   Val Ser Ala Leu Leu Phe Leu Gly Ala Gly Leu Gly Ala Leu Arg Val
   175 180 185 190
   gat gtt gtc ggt gat ctg cgt tcc cgt cct tac gtt gat atg acc gtc 627
   Asp Val Val Gly Asp Leu Arg Ser Arg Pro Tyr Val Asp Met Thr Val
   195 200 205
   gag acc ctc gag agg ttc ggt gtc agc gtc gtt agg gag ggt tcc tct 675
   Glu Thr Leu Glu Arg Phe Gly Val Ser Val Val Arg Glu Gly Ser Ser
   210 215 220
   ttc gag gtc gag ggt cgt cct cgt tca cct ggt aag ctg agg gtc gag 723
   Phe Glu Val Glu Gly Arg Pro Arg Ser Pro Gly Lys Leu Arg Val Glu
   225 230 235
   aac gat tgg tcc tcc gcc ggt tac ttc gtt gcg ttg ggt gcg atc ggt 771
   Asn Asp Trp Ser Ser Ala Gly Tyr Phe Val Ala Leu Gly Ala Ile Gly
   240 245 250
   ggt gag atg cgt atc gag ggt gtt gat ctg gat agc agc cat ccc gat 819
   Gly Glu Met Arg Ile Glu Gly Val Asp Leu Asp Ser Ser His Pro Asp
   255 260 265 270
   cgt agg atc gtc gag atc acc cgc gag atg ggt gcc gag gtt cgt cgt 867
   Arg Arg Ile Val Glu Ile Thr Arg Glu Met Gly Ala Glu Val Arg Arg
   275 280 285
   atc gat ggt ggt atc gtc gtc cgt tca acc ggt cgt ttg gag ggt gtt 915
   Ile Asp Gly Gly Ile Val Val Arg Ser Thr Gly Arg Leu Glu Gly Val
   290 295 300
   gag gtc gat ctg agc gat tcc cct gat ctg gtc cct acc gtc gcg gcc 963
   Glu Val Asp Leu Ser Asp Ser Pro Asp Leu Val Pro Thr Val Ala Ala
   305 310 315
   atg gcc tgc ttc gcc gag ggt gtt act cgt atc gag aac gtt ggt cat 1011
   Met Ala Cys Phe Ala Glu Gly Val Thr Arg Ile Glu Asn Val Gly His
   320 325 330
   ttg agg tac aag gag gtc gat cgc ctg cgt gcg ttg gcc gcg gag ttg      1059
   Leu Arg Tyr Lys Glu Val Asp Arg Leu Arg Ala Leu Ala Ala Glu Leu
   335                 340                 345                 350
   cct aag ttc ggt gtt gag gtt agg gag ggt aag gat tgg ttg gag ata      1107
   Pro Lys Phe Gly Val Glu Val Arg Glu Gly Lys Asp Trp Leu Glu Ile
                   355                 360                 365
   gtc ggt ggt gag cct gtt ggt gcc agg gtt gat tca agg ggt gat cat      1155
   Val Gly Gly Glu Pro Val Gly Ala Arg Val Asp Ser Arg Gly Asp His
               370                 375                 380
   agg atg gcg atg gcg ctg gcg gtt gtt ggt gcg ttc gcc agg ggt aag      1203
   Arg Met Ala Met Ala Leu Ala Val Val Gly Ala Phe Ala Arg Gly Lys
           385                 390                 395
   acc gtt gtt gag cgt gcc gat gcg gtt tca atc tct tac ccc agg ttc      1251
   Thr Val Val Glu Arg Ala Asp Ala Val Ser Ile Ser Tyr Pro Arg Phe
       400                 405                 410
   tgg gag gat ctc gcc tct gtt ggt gtc cct gtt cat tcc gtt tga          1296
   Trp Glu Asp Leu Ala Ser Val Gly Val Pro Val His Ser Val  *
   415                 420                 425
   ggcgcgcctg caggtcgaca agctt                                          1321
<210> 10
   <211> 428
   <212> PRT
   <213> Methanopyrus kanleri
<400> 10
   Met Lys Arg Val Glu Leu Glu Gly Ile Pro Glu Val Arg Gly Thr Val
    1               5                  10                  15
   Cys Pro Pro Pro Ser Lys Ser Gly Ser His Arg Ala Leu Ile Ala Ala
               20                  25                  30
   Ser Leu Cys Asp Gly Ser Thr Glu Leu Trp Asn Val Leu Asp Ala Glu
           35                  40                  45
   Asp Val Arg Ala Thr Leu Arg Leu Cys Arg Met Leu Gly Ala Glu Val
       50                  55                  60
   Asp Val Asp Gly Glu Glu Arg Leu Glu Ala Thr Val Ser Gly Phe Gly
   65                  70                  75                  80
   Asp Ser Pro Arg Ala Pro Glu Asp Val Val Asp Cys Gly Asn Ser Gly
                   85                  90                  95
   Thr Thr Leu Arg Leu Gly Cys Gly Leu Ala Ala Leu Val Glu Gly Thr
               100                 105                 110
   Thr Ile Leu Thr Gly Asp Asp Ser Leu Arg Ser Arg Pro Val Gly Asp
           115                 120                 125
   Leu Leu Ala Ala Leu Arg Ser Leu Gly Val Asp Ala Arg Gly Arg Val
       130                 135                 140
   Val Arg Gly Glu Glu Tyr Pro Pro Val Val Ile Ser Gly Arg Pro Leu
   145                 150                 155                 160
   Arg Glu Arg Val Ala Val Tyr Gly Asp Val Ser Ser Gln Phe Val Ser
                   165                 170                 175
   Ala Leu Leu Phe Leu Gly Ala Gly Leu Gly Ala Leu Arg Val Asp Val
               180                 185                 190
   Val Gly Asp Leu Arg Ser Arg Pro Tyr Val Asp Met Thr Val Glu Thr
           195                 200                 205
   Leu Glu Arg Phe Gly Val Ser Val Val Arg Glu Gly Ser Ser Phe Glu
       210                 215                 220
   Val Glu Gly Arg Pro Arg Ser Pro Gly Lys Leu Arg Val Glu Asn Asp
   225                 230                 235                 240
   Trp Ser Ser Ala Gly Tyr Phe Val Ala Leu Gly Ala Ile Gly Gly Glu
                   245                 250                 255
   Met Arg Ile Glu Gly Val Asp Leu Asp Ser Ser His Pro Asp Arg Arg
               260                 265                 270
   Ile Val Glu Ile Thr Arg Glu Met Gly Ala Glu Val Arg Arg Ile Asp
           275                 280                 285
   Gly Gly Ile Val Val Arg Ser Thr Gly Arg Leu Glu Gly Val Glu Val
       290                 295                 300
   Asp Leu Ser Asp Ser Pro Asp Leu Val Pro Thr Val Ala Ala Met Ala
   305                 310                 315                 320
   Cys Phe Ala Glu Gly Val Thr Arg Ile Glu Asn Val Gly His Leu Arg
                   325                 330                 335
   Tyr Lys Glu Val Asp Arg Leu Arg Ala Leu Ala Ala Glu Leu Pro Lys
               340                 345                 350
   Phe Gly Val Glu Val Arg Glu Gly Lys Asp Trp Leu Glu Ile Val Gly
           355                 360                 365
   Gly Glu Pro Val Gly Ala Arg Val Asp Ser Arg Gly Asp His Arg Met
       370                 375                 380
   Ala Met Ala Leu Ala Val Val Gly Ala Phe Ala Arg Gly Lys Thr Val
   385                 390                 395                 400
   Val Glu Arg Ala Asp Ala Val Ser Ile Ser Tyr Pro Arg Phe Trp Glu
                   405                 410                 415
   Asp Leu Ala Ser Val Gly Val Pro Val His Ser Val
               420                 425
<210> 11
   <211> 13066
   <212> DNA
   <213> sulfolobus solfataricus
<220>
   <221> misc_feature
   <222> (0)...(0)
   <223> aroA coding sequence: 10,578 - 11,822
<400> 11
   ctgcagtatt tctaactgtc attaaatcgt ttggttttgt aactctcatc tttattacaa 60
   tctcaaactc tttgccttct actttgcttt caattgacat tccttctggc aaatttacat 120
   catcaacttt taaacttctt ataaatagtt ctgcatcctt atattccgat gagattctta 180
   actctatcat caattttaat caatccttct ttctttactg acggaataaa aaatctacta 240
   gtgaaaagtc tatcattgtc tctgattact atcattttat ctctccttat tttttgtaac 300
   tgtaacaata ttatttgtgc taagagagga gaagtgagat tagatgtctc aacaatataa 360
   tatgactgtt tattttcaat agaattgatt gagaatccct tggagagagt ttgcctaaat 420
   ctactaatta atgcctctgc ataagaggat gacaggacaa actgtaagat ctcattactg 480
   ccaatagtat caaacgaata aattaaagag aatgccaact ctaatatatc ataatcaaga 540
   taaaaaaatc tatccgtaat tatatcatcc ctagtgaatt ttggattttc tttcacaata 600
   gtagaaatta ttctgaaaag taatgtattt agctgactct cattcaaatc ttctaatttc 660
   gatatctcac tagcgtttac ctctcttatg agacttatgg cattttctct attgccagat 720
   atgccaggta tataaggatc tagagaaaac attaacgata aaaaggctgg taaagtttta 780
   tatgcaggaa ttttcagatt tttttcgata gtcatatcaa atttctccat ttcccttatt 840
   gtctctaatt cccattcgga aagtgatctt ctgtctaata ttgtagacgt tataatacaa 900
   ctaacaattg gcaagacatc ttcagcagtt ataggtaaat atgaacaaaa ggaattctga 960
   cccaagaaat attttttctt attatctatt atccatttcc cgttagcgtc aagtctaatt 1020
   tgtgtttcac attcttctgg aatgaacgaa agagtgaaag aattcttagt atttttggaa 1080
   ataataaatg aaaataggat aggctctata gaatagtacg cagataaaca caaattatcc 1140
   ttagaataca cattcctaac tacttccctt atttcttgat tattcggctc tcctaagaat 1200
   acttccatta ctatctcttt agaatttctt ttgaccattt tttaagctct tcaaagctca 1260
   tatccttaaa gaaaatagat ccaagcaagt ttgatataat atagttcaat cttgtagcaa 1320
   agacgtaaga gggatttaaa aatattgata acgctaattc tcccactatt gctgcagatg 1380
   gtatcggtat taatatagat acttggtata aaaaagagat aaaaattgac aagaaaatat 1440
   tatgtgttac ataataaaaa taacctccat ataaggcaaa actaaatata tcaatcatga 1500
   tataaattac aaaatccttt gtggttcctt gcttaaggta atatttaaat tccatataat 1560
   ctgtaacaat tcgttccatt ttaactatct taaagataaa ttgttcaatt ttatttactt 1620
   tatcatgttg caagtaaaaa tacgacaatg ctcctatcca tccagctatg ttaaataata 1680
   cgactagaat gaatacaaat tctaatgggt taaaaaagaa tggcagcagt aaaatatagc 1740
   ccaaacatat tgcgagcaca tctatggatc ctataagaat agagtagcta aacgcttgcc 1800
   ttaagtttac cccatatttg ttgtaaacta aacttctaac caattcttgt ccagcccatc 1860
   caggtactaa taaacctacg aagttaccta gtaatcttgc tcttaacgtt atatctactc 1920
   tacgcttaat tagtaatgaa tccttaaatg atgagacgaa attctgggca gtatacgtta 1980
   aaagaaaaat cagaaagaat ttgggatctt cttgtaaaac atatattata ttaatcttaa 2040
   atatgtatgc atagactatg atcactataa acggaagaaa aatagctgca atgtatttct 2100
   tatccattat cttccccttt tctcaaaaca tccaatacta ggatagcata aagttacatt 2160
   cccaagattt ttaaacactt tgggctcatc taactcagga attttcatat tagttctaaa 2220
   tattccacat ttaggtttac ttatcttttg atatagaaaa ggatactcat ttagaacgca 2280
   tgtcttacat tgtgagttct tctcgatgtt aattctctca atttttaatt ctctagaatc 2340
   tatatagaat aatgaataat ccggattgcc tctcaagtga ttaagcatta agttaacttg 2400
   aagtgtagct gttaattcta ctattagtgg agtagttcca ataacatcac atgaattccc 2460
   aatttcgtct tgatctgaat agtcaataaa acaagataaa caggaggttt gactagggtc 2520
   tatcagttta gcagaaccgt attcaccatt aattcctcca tatattagga tttttcctaa 2580
   ttttactata gcatcattta acaatagctt gtagtacaag ctgtctaatg catcgaacac 2640
   gtaatcctta tcagaaatta gcctctcgac gttctcctcg tcgagtatat ctataatata 2700
   attaattttg attgaagaat ttataaggga tattttttta gcgcaaactt cagctttagg 2760
   tttgcctaca tcattttcat caaataaatg gaccctatgt agattagtta tatctaccac 2820
   atctgcatct actatagtta actctttaac tcctagccta gctagcaact ctgcaacggc 2880
   agtacctaaa gctccacagc ctgcaattaa tatctttaac tcatttaacc tctgttgaat 2940
   tcctaatcct aaaactatta gttgcctaga atatctttcc acaagattat aatgtagaat 3000
   aatctttaaa aataagtgtt gcctactaaa agtggggatt gatattgttc caatggaaaa 3060
   aggtggaggg agtgatggaa ctccaatatc aatagaggaa ttggacaagt taagacaagt 3120
   agctgaaaag gcaagaagaa atgtaataaa aatgctattt tatgatcaaa caatacatgt 3180
   gggatcgtcc ctaagtagca tagagatatt aactacgtta atattcaagc atataaggac 3240
   ggattcaagc ttagtgaata aagactggct tattttaagt aaaggccatg cagcgccagc 3300
   tctttatgcc gttttagctg aaaaaggtta cataaaagaa gaggaacttt ggagaataca 3360
   agatataacg ggattattac aagggcatcc agaaactttt attcccggtg tagatatgtc 3420
   gactggtagt ttagggcaag gtttgagctt tggaataggt gttgctactg gtataaagat 3480
   ggccaacggc actggaagag tatatgtcat aatgggtgat ggtgaacagg atgagggaga 3540
   aatatgggag gctatgacgc atgcagtagt tagaaatctt gataacttaa ttgcatttat 3600
   agagatgaac aatttccagc ttgatggttc aacagatgag ataaaaccaa agaacttctt 3660
   acctaaggta tgggaagcag taggttggaa agtattaaac tgcgatgggc atgatttcat 3720
   tagtattact aatgcagtta acgaggcata taaggcaagc aagcccgtag taatattcgc 3780
   taagactgta agaggaaagg ggtttcctcc aatagaaaat acccataaac agaggtccag 3840
   tccagatgat gcaaggaaat atttactcaa tgcgtgaaac cttcggaagg ctattagcag 3900
   acctagggga taagaacaag gatctagtcg tgataactgc agatgtagga gactctacca 3960
   gagcgctata ctttagagag aagtttaagg atagatactt taatgtaggc atagcagagc 4020
   aagatatggt gaattttgct gctggcttgg ctgctgtagg aaaaaagccc gctatagtta 4080
   actttggaat gttcttaatg agagcgtggg agcagataag aaatagtata gctagaatga 4140
   atctagacgt caagatgttt gtaacacaca ccggatacag tgaccacggt gatggttcga 4200
   gtcatcaagt tctcgaagat atagcgctaa tgcgtgtatt accaaacatg aaagtagtag 4260
   taccagcaga tcctaaggat attgaaagaa gcttaccagt tataattaat gaggaaaggg 4320
   gtccattgta ttataggata ggtagagaat attcaccacc aatcactata ggacaagaat 4380
   acgaattcaa gattggtaaa gcttatgtga ttaaggatgg gagtgactta gccataatag 4440
   gagcaggcgt tgttttgtgg gatgcactaa aggcggctga agaattagag aaattaggaa 4500
   ttagcgtagc agttataaat ttattctcaa taaagcctat tgacgaaaat acaatagaat 4560
   attatgctag aaaggctggt aagataatta ctattgagga acatagcata tatggaggta 4620
   ttggttctgc cgttgcagag gttacggcta ggcgttatcc agtacccata agatttgtag 4680
   gtgctacgac ttttggaaga tctgctagaa gccaaaggga tctactagat tactataata 4740
   taaactataa aacaattata agggaggcaa ttgatttatt gaagtagatg actgaagaaa 4800
   taacacggct gagggaagaa atagataagg tagacgagca gttagtaaag ttactctcat 4860
   atagattaga attatctaga aaaataggga aagctaagtc gaattctaat ataagtgtta 4920
   ctgacgagaa tagggaaatg aaagttagag aaaaatggat tgctaacgca aaaaagtata 4980
   atattccaaa tagtctggtt gaatctatat tgcctttgat tttttcttat tctaaactag 5040
   ttcagattaa cccaggagag aaagaaagag tagtaatata tggatatggc ggaatggcta 5100
   aatcgatcgt ttctattctt tcattagctg ggcatgaagt atcgattact ggaagagatt 5160
   taagtaaagc ggagatgtta gctaatcaat ttaaatgtgt aagtatgtcc ttattaaaag 5220
   caatagattg gggggatata attatatttg caatacctcc tagtgcaata ttaaataatt 5280
   ccgatgaatt attttcaaag gcacttaaag ataagattgt tatggatatt agttcttcta 5340
   aatttaaaat atttggcttc ctagaagaat tatctaggaa actagagttt aggtatattt 5400
   ctacacatcc acttttcggt cctattgaat accctattgg agagagagtt gtaattatac 5460
   cttccaaaac tagttctaat gatgatgtca tgaaggtgga gaatttctgg aggaaaagtg 5520
   gtttagtacc cgtcataact gatgttgaaa ctcatgaaaa agcaatggct attgttcaag 5580
   ttctaacgca ttattatctt ctgggtttat caaacgcaat tgatacttta tcgttagagt 5640
   taggtgtaga ttacagtaat ttccatacta caaactttag agaattaaac aagattttaa 5700
   agcgggttaa agatctaaaa aatgtaatta ctgaaataca aaatcaaaac ccttattctt 5760
   ataaagttag aaatataggt ttagaggagc ttaaaaaaat taaagaagaa ttagaaggag 5820
   gtaaatagaa tgatcttata tgtccttaag gatagagctg attactctat actaatagaa 5880
   aagctaaatg aaaactcagc atctttcaag atattaaacc tatatggtaa aaacttaata 5940
   ttagcatggc cagatcagaa cgtgaaaggt atcattgata atagtataga aatggctgtg 6000
   gaagtaaaga aaagctatgt attagctggt aatgattgga aaaagcaacc aacagtggta 6060
   aatgtaaaag atgtagaaat tggaagcaaa aaggtaatag tagctgcagg tccttgtgca 6120
   gtagaaaatg aagaacaagt ccttactact gctaaggctg taaaaagggc tggagcatca 6180
   ttacttagag gaggggctta caaacctagg acaagtccat attccttcca aggtctcgga 6240
   gaagaagggg tgaaaatctt gaggagagta ggagatgaag taggcttacc tattgtcaca 6300
   gaaataatgg atacaagaga ttccaatata tttagccaat atgttgatat gatacagatt 6360
   ggagccagaa acgcacagaa cttctcttta ttgaaggaag ttggaaagtt aggtaaacca 6420
   gtactactta agcgaggtat gggaaataca gtagaggaat ggcttcaagc tgcagagtac 6480
   attttactag agggaaatgg caatacagta ttatgcgaaa gaggaataag aacatttgaa 6540
   aagtcaacta ggtttacgtt agatataggt gggatggtag ctgctaaact aatgacacat 6600
   ttgcccatct gtgctgatcc aagtcatcct gcgggaaaaa gagaattggt acactcttta 6660
   gcactagctg cagtcgctgc tggtgcggat atgttattaa ttgaagttca tccacatcca 6720
   gaaaaggcat taagtgattc agagcaacaa cttacaccgg aatcattcga agttctaatg 6780
   aatcgaatta gaacgctagc tagagcttta gggagagatg catgagggaa atcttagaag 6840
   atatttgttg ctctgaagta agagtagtag taggagaggg atcactttca aaattatcta 6900
   agattaaaga caataacgct gcagttatct attcaagaaa aattagtata gcagataaaa 6960
   ttaataaata tttaccaaat gcatacttca tcccaattaa tgatggtgaa agtactaaag 7020
   aattatctag tgtaatatct ttagtagaaa agctatttga aaagaatttc gatagggggg 7080
   attatattat aggtgttggt ggtggaacgg taactgatgt agctggtttc ttagcatcta 7140
   tatatttaag aggattaaat ctgataaacg taccaacgac cttcttaggc atggtcgatg 7200
   cagcaatagg gggtaagaat ggagtaaatt tcaataatat aaagaactta attggaacat 7260
   tctatcaacc aagtatgata atttccgatt tagaattttt ggaaactcta ccaatagaag 7320
   aactaaagaa gggattagct gaagtaatta aatatggctt aactttagat aaagaattat 7380
   atgattactt gtctttaaat aaggagaaga tactaaataa agataaacaa gcattagaag 7440
   atataatctt tagatctaca cttgataaac taagtattgt aaaagaagat gagagagaga 7500
   ctaaaggaat acgaatagtt ctaaatttcg gccatacgat aggtcatgct atagaagctg 7560
   gatcctcttt taatgttcca catggctacg ctatctctgt aggaatggtt tgtgaggcta 7620
   agatggcgga agagttaggt tatgcagagg aaggagtagt agaagatgtg ttatggctat 7680
   tacagattta tggtttacct tacgatatat ctcaaataga tgccccagta gatcttaaac 7740
   tagcattaaa tgctattaat atggataaaa aacataggaa agatgtaatt ttgataccgt 7800
   ttcctactag aataggtagc tggaaaaaag ttgaagttcc tctagatacc gtaaaggggt 7860
   ttgccgaaca atgcttgaag aaataaatta tgatactaag ttattcggtc taataggtaa 7920
   aaacataaag tacacgctat ccccttatat tcataatttc tcatttagaa cactaggaat 7980
   aaatgcagtt tatctagttt ttgatctcga cgaaatgaaa ttcaagcgta gtattagtgg 8040
   gatattggaa attgcagaag gacttaatgt tacgataccg tataaggatg aagtaatgaa 8100
   atatttggat aatactgata cgcactccac gagaattcaa gctgtaaata caatatataa 8160
   aaaaagtggt tataacactg attatttagc aataaaaaat cttgtaagaa agaagattaa 8220
   gaatgtatct ggctacgaat gttacatata tggggctgga ggtgcagcaa aagcagcagc 8280
   ttttgcgtta tctgaattag gatgctctag tattagtatt gtgaatagaa caaaatcaag 8340
   ggcttatgag ttagctgaat tattaaataa gaacggttat aacgcgtcaa ttaaagagaa 8400
   ttgcaacatt acaaataata tacttattgt caatagcact cctaattctt ctgtagtccc 8460
   agaggactgt gttaaaaaat ctgatcttgt tatagaattt gtttatagac cagttgagac 8520
   tgagttaatt aaaaatgcta aaaaatatgg tatacaatat ataaacggtc tagaaatttt 8580
   agtgaatcaa gctgtagaag cggagaagat atggtttaat aagagtgtgg cagatgaaaa 8640
   gattatagag tatctttatg ccagggaact cgtttggtaa actatttaga ataaccactt 8700
   ttggagagag ccatggtcct gcagtaggtg tagtcataga cggtgttcct gccggtttac 8760
   cattaactgt tgaagatata aagttcgaat tagaatttag aagaccaggt agactatacg 8820
   tttctggaag gagagaaaaa gatgagccgg aaatattaag tgggatcttt aataatagaa 8880
   ctaccggatc tccaatagca gttatagtac gaaatactga tgtaatatca agtttttatg 8940
   acgagattaa atataaacca agaccaggac atgcagacct tccatttata atgaaatatg 9000
   gatatgaaaa ttgggattat aggggaggtg gaagagcaag tgctagagaa actgtaagta 9060
   gagttatagc tggtgcagta gctaagaagt tacttatgct aacagatact tggatagctg 9120
   gccatcttag aagtttaggc ccagaagagt tgagtgaaga ggtaacattt gaggaggttc 9180
   tatgctcaaa atatagccca gtaagagcta gtaaaaaaga ccttgaggaa aaatatgaag 9240
   cattaataaa gaaagctact caagaagggg atagctatgg cggaatagct gaagtaatag 9300
   ccaagaatcc accaataggt ttaggagaac cagtctttga taagatgaaa gctgaattgg 9360
   ctaaagcaat aatgtcgatc cctgctgtga tgggcttcga gtatggttta ggttttattg 9420
   ctagtaaaat gaaaggaagt gaggctaatg acgagattat aagaaagaat aatagaattg 9480
   gctggaagta caattacgct ggaggcattt taggtggttt aacaaatggt gaagatctta 9540
   tagtgagatg tgcatttaaa cctactagct cgattagaaa gcctcaaaag accatagatt 9600
   taaggaactt agaggagagt tatatttcag taattggcag acacgaccca gctgtagcaa 9660
   ttaggggagt tactgttgta gaatcaatgg tagcgttaac catagtagac catgcaatgc 9720
   gtgcaggagc tattccacta gttaaactta cagaggacca agctaataca atacagcaac 9780
   gttgggagag gtatgtgaaa tcatgcaagc ctatggagga gtctcaatcg taaacgcact 9840
   accatcttgg tatggctcat ctatggcaat caatttgaag gtaaaagtag aaattagaga 9900
   aggtaagaga gtttattctc aagagagtga actaattaag accattctta attactttaa 9960
   agaaaaatat tcaataccgg atattgaagt tgatattgaa tctgaacttc cacaaaagag 10020
   tggactaaaa agcagtagtg cagtttctgt agccctaata gcggagattg caaagcaata 10080
   tgatctaagg aatattaacc ctccaatatt atctgcgata ctttcactga aagctggagt 10140
   gtcatatacc ggggcacttg atgatgcagt tgcatcatat tgtggaggaa tagcattcac 10200
   ttataataag atgtttagaa tagtaaagtt agagaatctt gaggataatt tatcgatcct 10260
   catattagct aagggaggga gacaaaaacc tgttaatcta aacgagctaa gaaaatatag 10320
   tcacgtcttt gaagaaattt ttaagatagc acttaaggat tacttgactg ctatgaagat 10380
   gaatggaata ttgattgcta atattttagg ctattcatta gaaccaatag aaattgcact 10440
   gaaaaaagga gcgttagctg ccgggattag tggaaatggg ccttcatatt ttgcagtttc 10500
   taagaatgga gaagaaggtc cgatatacga aagtcttaag aaatttggag atgttattat 10560
   agttaggcct gtaagtcttg attgtaaaga tttatccatc aaagattagt ggaataataa 10620
   aagctcctca atcaaaaagt ctagctatta ggttaatttt tctttcactt ttcactagag 10680
   tatatcttca taacttagtt ctatcggaag atgttataga cgctataaaa tcagtaagag 10740
   cattaggagt aaaggtaaaa aacaattctg aatttatacc tccagagaaa ttagaaatta 10800
   aggagaggtt tataaaatta aaaggttccg ctactactct tagaatgctt attccaatat 10860
   tagccgcaat aggcggagaa gtgacaattg atgcagatga gagtttaaga aggagacctt 10920
   taaacagaat cgtacaagca ttaagtaact acggtatatc cttttcttct tacagtttgc 10980
   ctttgactat cacgggaaag ttaagtagta atgagataaa gatttctggt gatgagagta 11040
   gtcaatatat ttctggctta atatacgcac ttcatattct aaatggcggt agtattgaaa 11100
   tattgccccc catttcatct aaaagttata ttctgcttac aatagattta tttaagagat 11160
   ttggttctga tgttaagttt tatggtagta agattcatgt taatcccaat aatttggttg 11220
   aatttcaagg cgaagtggcg ggagattatg gtttagcctc gttttacgcg ctttctgcat 11280
   tagttagtgg tggaggaatt acaataacta atttgtggga gccgaaggaa tattttggtg 11340
   atcatagtat tgttaaaata tttagtgaga tgggcgcttc cagtgaatat aaagacggta 11400
   gatggtttgt caaggctaaa gataaatatt ctcccataaa aattgatata gatgacgcac 11460
   ctgacctggc tatgacaatt gcgggattat ctgcaatagc ggagggaaca agtgaaatta 11520
   tagggatcga aagattgagg attaaggaaa gtgatagaat tgaaagtata aggaaaatct 11580
   taggattata tggtgtaggt agtgaagtaa agtataattc tattctgata ttcggaatta 11640
   acaagggtat gttaaactct ccagttacag actgtttgaa tgatcacaga gttgctatga 11700
   tgtcgtcagc cttagcttta gtgaatggtg gggtaattac atcagctgaa tgtgtaggta 11760
   aaagtaatcc taattactgg caagatttat tatcactaaa tgcgaagatt tctattgaat 11820
   gagaccatta attgtagctt cattaccaat taaaaagata gaagacttaa aacttataga 11880
   aaatttttta gatgcagatc taatagaact aagacttgat tatctaagag aaagagaagt 11940
   cagtttgata tctgactatt atgaattttt agataaatat aaaaagaagt taatagtaac 12000
   gttaagagat aaaggggagg gaggaataaa tcaattagcg gatgaattaa agataaaaat 12060
   tttaaatgaa ctctacgaga gacaatatct gtatgatata gaggtttcat ttcttcaaaa 12120
   atatgatata ccatacgata ataggatagt ttctgtccac tattttaatt atcttccaac 12180
   tctagagaag ataaaggaaa ttgttagcaa gttttccgaa aaagcgttca gcgttaagat 12240
   tgcagttcct agtctaaaag gatataagga ggtactctta cctcttcttg aatatgaaaa 12300
   cgtaaccgta attccaatga gtaataattc tttagagagg atcgcagtgg gtctactggg 12360
   ctcaaagtta gtttattcgt acgcaattga acctttagca caagggcaac tttactataa 12420
   aaaagttatc cagattttta attatattaa cgatataaca acttcatctt tagttacttg 12480
   aactctgtat acttttatag gctttttgga agctcccttt ttaggttctc cactatatat 12540
   agaaaaatga gaaaagtgac attgacatac taattcttct tttatgataa cgccatattt 12600
   tgaaagatca caacctaaat gcgggcattt attatcaaaa acaaaaaatc tatctactcc 12660
   tagataaaaa acgactaatt cacgtccatt aggcagtata atttttctct tttcaccagt 12720
   cttaaaatca gttctactta tccttatata ctccacgact ttaattatga gctaacggag 12780
   aaattaaagc aaactatgtg ttaagcataa aaataagact cttatataat aacataactt 12840
   taatagagtt attgctctaa aaggtaatgc caaaattctt ccttataggt catttaatct 12900
   tgataatcaa atggcaagat ttaaacataa taggtgtaga ataataaaaa ctgcttaaaa 12960
   gattatgaac aaacaattta taagattggg agcaaaataa gtagattaga ggaaatcgaa 13020
   gatgttagaa ataagtgagg atcttaaagc aaagcttgat tataga                13066
<210> 12
   <211> 414
   <212> PRT
   <213> solfolobus solfataricus
<400> 12
   Met Ile Val Lys Ile Tyr Pro Ser Lys Ile Ser Gly Ile Ile Lys Ala
   1 5 10 15
   Pro Gln Ser Lys Ser Leu Ala Ile Arg Leu Ile Phe Leu Ser Leu Phe
   20 25 30
   Thr Arg Val Tyr Leu His Asn Leu Val Leu Ser Glu Asp Val Ile Asp
   35 40 45
   Ala Ile Lys Ser Val Arg Ala Leu Gly Val Lys Val Lys Asn Asn Ser
   50 55 60
   Glu Phe Ile Pro Pro Glu Lys Leu Glu Ile Lys Glu Arg Phe Ile Lys
   65 70 75 80
   Leu Lys Gly Ser Ala Thr Thr Leu Arg Met Leu Ile Pro Ile Leu Ala
   85 90 95
   Ala Ile Gly Gly Glu Val Thr Ile Asp Ala Asp Glu Ser Leu Arg Arg
   100 105 110
   Arg Pro Leu Asn Arg Ile Val Gln Ala Leu Ser Asn Tyr Gly Ile Ser
   115 120 125
   Phe Ser Ser Tyr Ser Leu Pro Leu Thr Ile Thr Gly Lys Leu Ser Ser
   130 135 140
   Asn Glu Ile Lys Ile Ser Gly Asp Glu Ser Ser Gln Tyr Ile Ser Gly
   145 150 155 160
   Leu Ile Tyr Ala Leu His Ile Leu Asn Gly Gly Ser Ile Glu Ile Leu
   165 170 175
   Pro Pro Ile Ser Ser Lys Ser Tyr Ile Leu Leu Thr Ile Asp Leu Phe
   180 185 190
   Lys Arg Phe Gly Ser Asp Val Lys Phe Tyr Gly Ser Lys Ile His Val
   195 200 205
   Asn Pro Asn Asn Leu Val Glu Phe Gln Gly Glu Val Ala Gly Asp Tyr
   210 215 220
   Gly Leu Ala Ser Phe Tyr Ala Leu Ser Ala Leu Val Ser Gly Gly Gly
   225 230 235 240
   Ile Thr Ile Thr Asn Leu Trp Glu Pro Lys Glu Tyr Phe Gly Asp His
   245 250 255
   Ser Ile Val Lys Ile Phe Ser Glu Met Gly Ala Ser Ser Glu Tyr Lys
   260 265 270
   Asp Gly Arg Trp Phe Val Lys Ala Lys Asp Lys Tyr Ser Pro Ile Lys
   275 280 285
   Ile Asp Ile Asp Asp Ala Pro Asp Leu Ala Met Thr Ile Ala Gly Leu
   290 295 300
   Ser Ala Ile Ala Glu Gly Thr Ser Glu Ile Ile Gly Ile Glu Arg Leu
   305 310 315 320
   Arg Ile Lys Glu Ser Asp Arg Ile Glu Ser Ile Arg Lys Ile Leu Gly
   325 330 335
   Leu Tyr Gly Val Gly Ser Glu Val Lys Tyr Asn Ser Ile Leu Ile Phe
   340 345 350
   Gly Ile Asn Lys Gly Met Leu Asn Ser Pro Val Thr Asp Cys Leu Asn
   355 360 365
   Asp His Arg Val Ala Met Met Ser Ser Ala Leu Ala Leu Val Asn Gly
   370 375 380
   Gly Val Ile Thr Ser Ala Glu Cys Val Gly Lys Ser Asn Pro Asn Tyr
   385 390 395 400
   Trp Gln Asp Leu Leu Ser Leu Asn Ala Lys Ile Ser Ile Glu
   405 410
<210> 13
   <211> 1892
   <212> DNA
   <213> Arthrobacter globiformis
<220>
   <221> CDS
   <222> (109)...(1417)
   <223> Strain ATX21308
<221> misc_feature
   <222> 1801
   <223> n= A, T, C or G
<400> 13
   gggaccacat gctgctcctg atttcagggc tgctgccggt atggaccagg gtttagagag 60
   ggacggcacg catccgggcc cttatcggac caacgccaac agcggtcg gtg gcc ttg  117
                                                        Val Ala Leu
                                                        1
   gag cgg ggc cag cac ggc cga tca cgt aga ctc ttt gga gct tcg ctc   165
   Glu Arg Gly Gln His Gly Arg Ser Arg Arg Leu Phe Gly Ala Ser Leu
       5                   10                  15
   gaa agg atc acc atg gaa act gat cga cta gtg atc cca gga tcg aaa   213
   Glu Arg Ile Thr Met Glu Thr Asp Arg Leu Val Ile Pro Gly Ser Lys
    20                 25                  30                  35
   agc atc acc aac cgg gct ttg ctt ttg gct gcc gca gcg aag ggc acg      261
   Ser Ile Thr Asn Arg Ala Leu Leu Leu Ala Ala Ala Ala Lys Gly Thr
                   40                  45                  50
   tcg gtc ctg gtg aga cca ttg gtc agc gcc gat acc tca gca ttc aaa      309
   Ser Val Leu Val Arg Pro Leu Val Ser Ala Asp Thr Ser Ala Phe Lys
               55                  60                  65
   act gca att cag gcc ctc ggt gcc aac gtc tca gcc gac ggt gac aat      357
   Thr Ala Ile Gln Ala Leu Gly Ala Asn Val Ser Ala Asp Gly Asp Asn
           70                  75                  80
   tgg gtc gtt gaa ggc ctg ggt cag gca ccc cac ctc gac gcc gac atc      405
   Trp Val Val Glu Gly Leu Gly Gln Ala Pro His Leu Asp Ala Asp Ile
       85                  90                  95
   tgg tgc gag gat gca ggt acc gtg gcc cgg ttc ctc cct cca ttc gtc      453
   Trp Cys Glu Asp Ala Gly Thr Val Ala Arg Phe Leu Pro Pro Phe Val
   100                 105                 110                 115
   gcc gca gga cag ggg aag ttc acc gtc gac gga agc gag cag ctg cgg      501
   Ala Ala Gly Gln Gly Lys Phe Thr Val Asp Gly Ser Glu Gln Leu Arg
                   120                 125                 130
   cgg cgc ccg ctt cgg ccc ctg gtc gac ggc atc cgc cac ctg ggc gcc      549
   Arg Arg Pro Leu Arg Pro Leu Val Asp Gly Ile Arg His Leu Gly Ala
               135                 140                 145
   cgc gtc tcc tcc gag cag ctg ccc cta aca att gaa gcg agc ggg ctg      597
   Arg Val Ser Ser Glu Gln Leu Pro Leu Thr Ile Glu Ala Ser Gly Leu
           150                 155                 160
   gca ggc ggg gag tac gaa att gaa gcc cat cag agc agc cag ttc gcc      645
   Ala Gly Gly Glu Tyr Glu Ile Glu Ala His Gln Ser Ser Gln Phe Ala
       165                 170                 175
   tcc ggc ctg atc atg gcc gcc ccg tac gcg cga caa ggc ctg cgt gtg      693
   Ser Gly Leu Ile Met Ala Ala Pro Tyr Ala Arg Gln Gly Leu Arg Val
   180                 185                 190                 195
   cgg ata cca aat ccc gtg agc cag ccc tac ctc acg atg aca ctg cgg      741
   Arg Ile Pro Asn Pro Val Ser Gln Pro Tyr Leu Thr Met Thr Leu Arg
                   200                 205                 210
   atg atg agg gac ttc ggc ctt gag acc agc acc gac gga gcc acc gtc      789
   Met Met Arg Asp Phe Gly Leu Glu Thr Ser Thr Asp Gly Ala Thr Val
               215                 220                 225
   agc gtc cct ccc ggg cgc tac aca gcc cgg cgg tat gaa att gaa ccg      837
   Ser Val Pro Pro Gly Arg Tyr Thr Ala Arg Arg Tyr Glu Ile Glu Pro
           230                 235                 240
   gac gcg tca act gcg tcg tac ttc gcc gcc gct tcc gcc gtc tct ggc      885
   Asp Ala Ser Thr Ala Ser Tyr Phe Ala Ala Ala Ser Ala Val Ser Gly
       245                 250                 255
   cga agc ttc gaa ttc cag ggc ctt ggc aca gac agc atc caa ggc gac      933
   Arg Ser Phe Glu Phe Gln Gly Leu Gly Thr Asp Ser Ile Gln Gly Asp
   260                 265                 270                 275
   acg tca ttc ttc aat gta ctt ggg cgg ctc ggt gca gag gtc cac tgg      981
   Thr Ser Phe Phe Asn Val Leu Gly Arg Leu Gly Ala Glu Val His Trp
                   280                 285                 290
   gca ccc aac tcg gtc acc ata tcc gga ccg gaa agg ctg aac ggc gac      1029
   Ala Pro Asn Ser Val Thr Ile Ser Gly Pro Glu Arg Leu Asn Gly Asp
               295                 300                 305
   att gaa gtg gat atg ggc gag ata tcg gac acc ttc atg aca ctc gcg 1077
   Ile Glu Val Asp Met Gly Glu Ile Ser Asp Thr Phe Met Thr Leu Ala
   310 315 320
   gcg att gcc cct cta gcc gat gga ccc atc acg ata acc aac att ggc 1125
   Ala Ile Ala Pro Leu Ala Asp Gly Pro Ile Thr Ile Thr Asn Ile Gly
   325 330 335
   cat gca cgg ttg aag gaa tcc gac cgc atc tcg gcg atg gaa acc aac 1173
   His Ala Arg Leu Lys Glu Ser Asp Arg Ile Ser Ala Met Glu Thr Asn
   340 345 350 355
   ctg cga acg ctc ggt gta caa acc gac gtc gga cac gac tgg atg cga 1221
   Leu Arg Thr Leu Gly Val Gln Thr Asp Val Gly His Asp Trp Met Arg
   360 365 370
   atc tac ccc tct acc ccg cac ggc ggc aga gtc aat tgc cac cgg gac 1269
   Ile Tyr Pro Ser Thr Pro His Gly Gly Arg Val Asn Cys His Arg Asp
   375 380 385
   cac agg atc gcc atg gcg ttt tca atc ctg gga ctg cga gtg gac ggg 1317
   His Arg Ile Ala Met Ala Phe Ser Ile Leu Gly Leu Arg Val Asp Gly
   390 395 400
   att acc ctc gac gac cct caa tgt gtc ggg aag acc ttt cct ggc ttc 1365
   Ile Thr Leu Asp Asp Pro Gln Cys Val Gly Lys Thr Phe Pro Gly Phe
   405 410 415
   ttc gac tac ctt gga cgc ctt ttc ccc gaa aag gcg ctt acg ctc ccc 1413
   Phe Asp Tyr Leu Gly Arg Leu Phe Pro Glu Lys Ala Leu Thr Leu Pro
   420 425 430 435
   ggc t agtgacttcc tctccggcgg acgctaggca tcggaaaacg aatcctgaca 1467
   Gly
   tgaccgacct cctcgcgtca cggcgtgtct gccggtaccc aagcattctg ccttagccgc 1527
   ttccgcggcc ccttatgctt tctggttgtc cagattttca tccgggatgt tgcctgacct 1587
   tgagcagggc aatcagctgt tcagcactgt caatggtgtg ggccctgaag gcggcttcga 1647
   tggctgccac gtcggcggct ctcatcgctg tcacgacacg cagatgcgct tcataggcac 1707
   gttcaggatc cgccctcgtc gcctgatcct gagccaaggc aatagttaga tgtgcctccg 1767
   ttggcggcca gagccgaagc aataaggagt tttncgaggc cacccagatt ccccgggtgg 1827
   aaggcgatat gggcttcatg ctgaactatg gggtccggat ggaagtgact tttcaactct 1887
   gccca 1892
<210> 14
   <211> 436
   <212> PRT
   <213> Arthrobacter globiformis
<220>
   <221> VARIANT
   <222> (0)...(0)
   <223> Strain ATX21308
<400> 14
   Met Ala Leu Glu Arg Gly Gln His Gly Arg Ser Arg Arg Leu Phe Gly
   1 5 10 15
   Ala Ser Leu Glu Arg Ile Thr Met Glu Thr Asp Arg Leu Val Ile Pro
   20 25 30
   Gly Ser Lys Ser Ile Thr Asn Arg Ala Leu Leu Leu Ala Ala Ala Ala
   35 40 45
   Lys Gly Thr Ser Val Leu Val Arg Pro Leu Val Ser Ala Asp Thr Ser
   50 55 60
   Ala Phe Lys Thr Ala Ile Gln Ala Leu Gly Ala Asn Val Ser Ala Asp
   65 70 75 80
   Gly Asp Asn Trp Val Val Glu Gly Leu Gly Gln Ala Pro His Leu Asp
   85 90 95
   Ala Asp Ile Trp Cys Glu Asp Ala Gly Thr Val Ala Arg Phe Leu Pro
   100 105 110
   Pro Phe Val Ala Ala Gly Gln Gly Lys Phe Thr Val Asp Gly Ser Glu
   115 120 125
   Gln Leu Arg Arg Arg Pro Leu Arg Pro Leu Val Asp Gly Ile Arg His
   130 135 140
   Leu Gly Ala Arg Val Ser Ser Glu Gln Leu Pro Leu Thr Ile Glu Ala
   145 150 155 160
   Ser Gly Leu Ala Gly Gly Glu Tyr Glu Ile Glu Ala His Gln Ser Ser
   165 170 175
   Gln Phe Ala Ser Gly Leu Ile Met Ala Ala Pro Tyr Ala Arg Gln Gly
   180 185 190
   Leu Arg Val Arg Ile Pro Asn Pro Val Ser Gln Pro Tyr Leu Thr Met
   195 200 205
   Thr Leu Arg Met Met Arg Asp Phe Gly Leu Glu Thr Ser Thr Asp Gly
   210 215 220
   Ala Thr Val Ser Val Pro Pro Gly Arg Tyr Thr Ala Arg Arg Tyr Glu
   225 230 235 240
   Ile Glu Pro Asp Ala Ser Thr Ala Ser Tyr Phe Ala Ala Ala Ser Ala
   245 250 255
   Val Ser Gly Arg Ser Phe Glu Phe Gln Gly Leu Gly Thr Asp Ser Ile
   260 265 270
   Gln Gly Asp Thr Ser Phe Phe Asn Val Leu Gly Arg Leu Gly Ala Glu
   275 280 285
   Val His Trp Ala Pro Asn Ser Val Thr Ile Ser Gly Pro Glu Arg Leu
   290 295 300
   Asn Gly Asp Ile Glu Val Asp Met Gly Glu Ile Ser Asp Thr Phe Met
   305 310 315 320
   Thr Leu Ala Ala Ile Ala Pro Leu Ala Asp Gly Pro Ile Thr Ile Thr
   325 330 335
   Asn Ile Gly His Ala Arg Leu Lys Glu Ser Asp Arg Ile Ser Ala Met
   340 345 350
   Glu Thr Asn Leu Arg Thr Leu Gly Val Gln Thr Asp Val Gly His Asp
   355 360 365
   Trp Met Arg Ile Tyr Pro Ser Thr Pro His Gly Gly Arg Val Asn Cys
   370 375 380
   His Arg Asp His Arg Ile Ala Met Ala Phe Ser Ile Leu Gly Leu Arg
   385 390 395 400
   Val Asp Gly Ile Thr Leu Asp Asp Pro Gln Cys Val Gly Lys Thr Phe
   405 410 415
   Pro Gly Phe Phe Asp Tyr Leu Gly Arg Leu Phe Pro Glu Lys Ala Leu
   420 425 430
   Thr Leu Pro Gly
   435
<210> 15
   <211> 425
   <212> PRT
   <213> Agrobacterium tumifaciens
<400> 15
   Met Ile Glu Leu Thr Ile Thr Pro Pro Gly His Pro Leu Ser Gly Lys
    1               5                  10                  15
   Val Glu Pro Pro Gly Ser Lys Ser Ile Thr Asn Arg Ala Leu Leu Leu
               20                  25                  30
   Ala Gly Leu Ala Lys Gly Lys Ser Arg Leu Thr Gly Ala Leu Lys Ser
           35                  40                  45
   Asp Asp Thr Leu Tyr Met Ala Glu Ala Leu Arg Glu Met Gly Val Lys
       50                  55                  60
   Val Thr Glu Pro Asp Ala Thr Thr Phe Val Val Glu Ser Ser Gly Gly
   65                  70                  75                  80
   Leu His Gln Pro Glu Lys Pro Leu Phe Leu Gly Asn Ala Gly Thr Ala
                   85                  90                  95
   Thr Arg Phe Leu Thr Ala Ala Ala Ala Leu Val Asp Gly Ala Val Ile
               100                 105                 110
   Ile Asp Gly Asp Glu His Met Arg Lys Arg Pro Ile Met Pro Leu Val
           115                 120                 125
   Glu Ala Leu Arg Ser Leu Gly Val Glu Ala Glu Ala Pro Thr Gly Cys
       130                 135                 140
   Pro Pro Val Thr Val Cys Gly Lys Gly Thr Gly Phe Pro Lys Gly Ser
   145                 150                 155                 160
   Val Thr Ile Asp Ala Asn Leu Ser Ser Gln Tyr Val Ser Ala Leu Leu
                   165                 170                 175
   Met Ala Ala Ala Cys Gly Asp Lys Pro Val Asp Ile Ile Leu Lys Gly
               180                 185                 190
   Glu Glu Ile Gly Ala Lys Gly Tyr Ile Asp Leu Thr Thr Ser Ala Met
           195                 200                 205
   Glu Ala Phe Gly Ala Lys Val Glu Arg Val Ser Asn Ala Ile Trp Arg
       210                 215                 220
   Val His Pro Thr Gly Tyr Thr Ala Thr Asp Phe His Ile Glu Pro Asp
   225                 230                 235                 240
   Ala Ser Ala Ala Thr Tyr Leu Trp Gly Ala Glu Leu Leu Thr Gly Gly
                   245                 250                 255
   Ala Ile Asp Ile Gly Thr Pro Ala Asp Lys Phe Thr Gln Pro Asp Ala
               260                 265                 270
   Lys Ala His Glu Val Met Ala Gln Phe Pro His Leu Pro Ala Glu Ile
           275                 280                 285
   Asp Gly Ser Gln Met Gln Asp Ala Ile Pro Thr Ile Ala Val Leu Ala
       290                 295                 300
   Ala Phe Asn Glu Thr Pro Val Arg Phe Val Gly Ile Ala Asn Leu Arg
   305                 310                 315                 320
   Val Lys Glu Cys Asp Arg Ile Arg Ala Val Ser Leu Gly Leu Asn Glu
                   325                 330                 335
   Ile Arg Asp Gly Leu Ala His Glu Glu Gly Asp Asp Leu Ile Val His
               340                 345                 350
   Ser Asp Pro Ser Leu Ala Gly Gln Thr Val Asn Ala Ser Ile Asp Thr
           355                 360                 365
   Phe Ala Asp His Arg Ile Ala Met Ser Phe Ala Leu Ala Ala Leu Lys
       370                 375                 380
   Ile Gly Gly Ile Ala Ile Gln Asn Pro Ala Cys Val Gly Lys Thr Tyr
   385                 390                 395                 400
   Pro Gly Tyr Trp Lys Ala Leu Ala Ser Leu Gly Val Glu Tyr Ser Glu
                   405                 410                 415
   Lys Glu Thr Ala Ala Glu Pro Gln His
               420                 425
<210> 16
   <211> 418
   <212> PRT
   <213> Pseudomonas syringae
<220>
   <221> VARIANT
   <222> (0)...(0)
   <223> pv phaseolicolla strain 1448a
<400> 16
   Met Arg Pro Gln Ala Thr Leu Thr Val Leu Pro Val Glu Arg Pro Leu
   1               5                   10                  15
   Val Gly Arg Val Ser Pro Pro Gly Ser Lys Ser Ile Thr Asn Arg Ala
               20                  25                  30
   Leu Leu Leu Ala Gly Leu Ala Lys Gly Thr Ser Arg Leu Thr Gly Ala
           35                  40                  45
   Leu Lys Ser Asp Asp Thr Arg Val Met Ser Glu Ala Leu Arg Leu Met
       50                  55                  60
   Gly Val Gln Val Asp Glu Pro Asp Asp Ser Thr Phe Val Val Thr Ser
   65                  70                  75                  80
   Ser Gly His Trp Gln Ala Pro Gln Gln Ala Leu Phe Leu Gly Asn Ala
                   85                  90                  95
   Gly Thr Ala Thr Arg Phe Leu Thr Ala Ala Leu Ala Asn Phe Glu Gly
               100                 105                 110
   Asp Phe Val Val Asp Gly Asp Glu Tyr Met Arg Lys Arg Pro Ile Gly
           115                 120                 125
   Pro Leu Val Asp Ala Leu Gln Arg Met Gly Val Glu Val Ser Ala Pro
       130                 135                 140
   Ser Gly Cys Pro Pro Val Ala Ile Lys Gly Lys Gly Gly Leu Glu Ala
   145                 150                 155                 160
   Gly Arg Ile Glu Ile Asp Gly Asn Leu Ser Ser Gln Tyr Val Ser Ala
                   165                 170                 175
   Leu Leu Met Ala Gly Ala Cys Gly Lys Gly Pro Val Glu Val Ala Leu
               180                 185                 190
   Thr Gly Ser Glu Ile Gly Ala Arg Gly Tyr Val Asp Leu Thr Leu Ala
           195                 200                 205
   Ala Met Gln Ala Phe Gly Ala Glu Val Gln Ala Ile Gly Glu Thr Ala
       210                 215                 220
   Trp Lys Val Ser Ala Thr Gly Tyr Arg Ala Thr Asp Phe His Ile Glu
   225                 230                 235                 240
   Pro Asp Ala Ser Ala Ala Thr Tyr Leu Trp Ala Ala Gln Ala Leu Thr
                   245                 250                 255
   Glu Gly Asp Ile Asp Leu Gly Val Ala Ser Asp Ala Phe Thr Gln Pro
               260                 265                 270
   Asp Ala Leu Ala Ser Gln Ile Ile Ala Ser Phe Pro Asn Met Pro Ala
           275                 280                 285
   Val Ile Asp Gly Ser Gln Met Gln Asp Ala Ile Pro Thr Leu Ala Val
       290                 295                 300
   Leu Ala Ala Phe Asn Arg Gln Pro Val Arg Phe Val Gly Ile Ala Asn
   305                 310                 315                 320
   Leu Arg Val Lys Glu Cys Asp Arg Ile Ser Ala Leu Ser His Gly Leu
                   325                 330                 335
   Cys Ala Ile Ala Pro Gly Leu Ala Val Glu Glu Gly Asp Asp Leu Leu
               340                 345                 350
   Val His Ala Asn Pro Ala Leu Ala Gly Thr Thr Val Asp Ala Leu Ile
           355                 360                 365
   Asp Thr His Ser Asp His Arg Ile Ala Met Cys Phe Ala Leu Ala Gly
       370                 375                 380
   Leu Lys Ile Ala Gly Ile Arg Ile Leu Asp Pro Asp Cys Val Gly Lys
   385                 390                 395                 400
   Thr Tyr Pro Gly Tyr Trp Asp Ala Leu Ala Ser Leu Gly Val Arg Val
                   405                 410                 415
   Gln Arg
<210> 17
   <211> 444
   <212> PRT
   <213> Ochrobactrum/Brucella
<400> 17
   Met Ala Cys Leu Pro Asp Asp Ser Gly Pro His Val Gly His Ser Thr
    1               5                  10                  15
   Pro Pro Cys Leu Asp Gln Glu Pro Cys Thr Leu Ser Ser Gln Lys Thr
               20                  25                  30
   Val Thr Val Thr Pro Pro Asn Phe Pro Leu Thr Gly Lys Val Ala Pro
           35                  40                  45
   Pro Gly Ser Lys Ser Ile Thr Asn Arg Ala Leu Leu Leu Ala Ala Leu
       50                  55                  60
   Ala Lys Gly Thr Ser Arg Leu Ser Gly Ala Leu Lys Ser Asp Asp Thr
   65                  70                  75                  80
   Arg His Met Ser Val Ala Leu Arg Gln Met Gly Val Thr Ile Asp Glu
                   85                  90                  95
   Pro Asp Asp Thr Thr Phe Val Val Thr Ser Gln Gly Ser Leu Gln Leu
               100                 105                 110
   Pro Ala Gln Pro Leu Phe Leu Gly Asn Ala Gly Thr Ala Met Arg Phe
           115                 120                 125
   Leu Thr Ala Ala Val Ala Thr Val Gln Gly Thr Val Val Leu Asp Gly
       130                 135                 140
   Asp Glu Tyr Met Gln Lys Arg Pro Ile Gly Pro Leu Leu Ala Thr Leu
   145                 150                 155                 160
   Gly Gln Asn Gly Ile Gln Val Asp Ser Pro Thr Gly Cys Pro Pro Val
                   165                 170                 175
   Thr Val His Gly Ala Gly Lys Val Gln Ala Arg Arg Phe Glu Ile Asp
               180                 185                 190
   Gly Gly Leu Ser Ser Gln Tyr Val Ser Ala Leu Leu Met Leu Ala Ala
           195                 200                 205
   Cys Gly Glu Ala Pro Ile Glu Val Ala Leu Thr Gly Lys Asp Ile Gly
       210                 215                 220
   Ala Arg Gly Tyr Val Asp Leu Thr Leu Asp Cys Met Arg Ala Phe Gly
   225                 230                 235                 240
   Ala Gln Val Asp Ile Val Asp Asp Thr Thr Trp Arg Val Ala Pro Thr
                   245                 250                 255
   Gly Tyr Thr Ala His Asp Tyr Leu Ile Glu Pro Asp Ala Ser Ala Ala
               260                 265                 270
   Thr Tyr Leu Trp Ala Ala Glu Val Leu Thr Gly Gly Arg Ile Asp Ile
           275                 280                 285
   Gly Val Ala Ala Gln Asp Phe Thr Gln Pro Asp Ala Lys Ala Gln Ala
       290                 295                 300
   Val Ile Ala Gln Phe Pro Asn Met Gln Ala Thr Val Val Gly Ser Gln
   305                 310                 315                 320
   Met Gln Asp Ala Ile Pro Thr Leu Ala Val Leu Ala Ala Phe Asn Asn
                   325                 330                 335
   Thr Pro Val Arg Phe Thr Glu Leu Ala Asn Leu Arg Val Lys Glu Cys
               340                 345                 350
   Asp Arg Val Gln Ala Leu His Asp Gly Leu Asn Glu Ile Arg Pro Gly
           355                 360                 365
   Leu Ala Thr Ile Glu Gly Asp Asp Leu Leu Val Ala Ser Asp Pro Ala
       370                 375                 380
   Leu Ala Gly Thr Ala Cys Thr Ala Leu Ile Asp Thr His Ala Asp His
   385                 390                 395                 400
   Arg Ile Ala Met Cys Phe Ala Leu Ala Gly Leu Lys Val Ser Gly Ile
                   405                 410                 415
   Arg Ile Gln Asp Pro Asp Cys Val Ala Lys Thr Tyr Pro Asp Tyr Trp
               420                 425                 430
   Lys Ala Leu Ala Ser Leu Gly Val His Leu Ser Tyr
           435                 440
<210> 18
   <211> 418
   <212> PRT
   <213> Pseudomonas syringae
<220>
   <221> VARIANT
   <222> (0)...(0)
   <223> Strain Dc3000 EPSPS Gene
<400> 18
   Met Arg Pro Gln Ala Thr Leu Thr Val Met Pro Val Glu Arg Pro Leu
    1               5                  10                  15
   Val Gly Arg Val Ser Pro Pro Gly Ser Lys Ser Ile Thr Asn Arg Ala
               20                  25                  30
   Leu Leu Leu Ala Gly Leu Ala Lys Gly Thr Ser Arg Leu Thr Gly Ala
           35                  40                  45
   Leu Lys Ser Asp Asp Thr Arg Val Met Ser Glu Ala Leu Arg Leu Met
       50                  55                  60
   Gly Val Gln Val Asp Glu Pro Asp Asp Ser Thr Phe Val Val Thr Ser
   65                  70                  75                  80
   Ser Gly His Trp Gln Ala Pro Gln Gln Ala Leu Phe Leu Gly Asn Ala
                   85                  90                  95
   Gly Thr Ala Thr Arg Phe Leu Thr Ala Ala Leu Ala Asn Phe Glu Gly
               100                 105                 110
   Asp Phe Val Val Asp Gly Asp Glu Tyr Met Arg Lys Arg Pro Ile Gly
           115                 120                 125
   Pro Leu Val Asp Ala Leu Gln Arg Met Gly Val Glu Ile Ser Ala Pro
       130                 135                 140
   Ser Gly Cys Pro Pro Val Ala Ile Lys Gly Lys Gly Gly Leu Gln Ala
   145                 150                 155                 160
   Gly Arg Ile Glu Ile Asp Gly Asn Leu Ser Ser Gln Tyr Val Ser Ala
                   165                 170                 175
   Leu Leu Met Ala Gly Ala Cys Gly Lys Gly Ser Leu Glu Val Ala Leu
               180                 185                 190
   Thr Gly Ser Glu Ile Gly Ala Arg Gly Tyr Val Asp Leu Thr Leu Ala
           195                 200                 205
   Ala Met Gln Ala Phe Gly Ala Glu Val Gln Ala Ile Gly Asp Ala Ala
       210                 215                 220
   Trp Lys Val Ser Ala Thr Gly Tyr His Ala Thr Asp Phe His Ile Glu
   225                 230                 235                 240
   Pro Asp Ala Ser Ala Ala Thr Tyr Leu Trp Ala Ala Gln Ala Leu Thr
                   245                 250                 255
   Glu Gly Asn Ile Asp Leu Gly Val Ala Ser Asp Ala Phe Thr Gln Pro
               260                 265                 270
   Asp Ala Leu Ala Ser Gln Ile Ile Asp Ser Phe Pro Asn Met Pro Ala
           275                 280                 285
   Val Ile Asp Gly Ser Gln Met Gln Asp Ala Ile Pro Thr Leu Ala Val
       290                 295                 300
   Leu Ala Ala Phe Asn Arg Gln Pro Val Arg Phe Val Gly Ile Ala Asn
   305                 310                 315                 320
   Leu Arg Val Lys Glu Cys Asp Arg Ile Ser Ala Leu Cys Asp Gly Leu
                   325                 330                 335
   Cys Ala Ile Ala Pro Gly Leu Ala Val Glu Glu Gly Asp Asp Leu Ile
               340                 345                 350
   Val His Ala Asn Pro Ala Leu Ala Gly Thr Thr Val Asn Ala Leu Ile
           355                 360                 365
   Asp Thr His Ser Asp His Arg Ile Ala Met Cys Phe Ala Leu Ala Gly
       370                 375                 380
   Leu Lys Ile Lys Gly Ile His Ile Gln Asp Pro Asp Cys Val Ala Lys
   385                 390                 395                 400
   Thr Tyr Pro Gly Tyr Trp Asp Ala Leu Ala Ser Leu Gly Val Ser Val
                   405                 410                 415
   Gln Arg
<210> 19
   <211> 418
   <212> PRT
   <213> Pseudomonas syringae
<220>
   <221> VARIANT
   <222> (0)...(0)
   <223> pv syringae strain B728a
<400> 19
   Met Arg Pro Gln Ala Thr Leu Thr Val Leu Pro Val Glu Arg Pro Leu
   1 5 10 15
   Val Gly Arg Val Ser Pro Pro Gly Ser Lys Ser Ile Thr Asn Arg Ala
   20 25 30
   Leu Leu Leu Ala Gly Leu Ala Lys Gly Thr Ser Arg Leu Thr Gly Ala
   35 40 45
   Leu Lys Ser Asp Asp Thr Arg Val Met Ser Glu Ala Leu Arg Leu Met
   50 55 60
   Gly Val Gln Val Asp Glu Pro Asp Asp Ser Thr Phe Val Val Thr Ser
   65 70 75 80
   Ser Gly His Trp Gln Ala Pro Gln Gln Ala Leu Phe Leu Gly Asn Ala
   85 90 95
   Gly Thr Ala Thr Arg Phe Leu Thr Ala Ala Leu Ala Asn Phe Glu Gly
   100 105 110
   Asp Phe Val Val Asp Gly Asp Glu Tyr Met Arg Lys Arg Pro Ile Gly
   115 120 125
   Pro Leu Val Asp Ala Leu Gln Arg Met Gly Val Glu Val Ser Ala Pro
   130 135 140
   Ser Gly Cys Pro Pro Val Ala Ile Lys Gly Lys Gly Gly Leu Glu Ala
   145 150 155 160
   Gly Arg Ile Glu Ile Asp Gly Asn Leu Ser Ser Gln Tyr Val Ser Ala
   165 170 175
   Leu Leu Met Ala Gly Ala Cys Gly Lys Gly Pro Val Glu Val Ala Leu
   180 185 190
   Thr Gly Ser Glu Ile Gly Ala Arg Gly Tyr Leu Asp Leu Thr Leu Ala
   195 200 205
   Ala Met Arg Ala Phe Gly Ala Glu Val Gln Ala Ile Gly Asp Ala Ala
   210 215 220
   Trp Lys Val Ser Ala Thr Gly Tyr Arg Ala Thr Asp Phe His Ile Glu
   225 230 235 240
   Pro Asp Ala Ser Ala Ala Thr Tyr Leu Trp Ala Ala Gln Ala Leu Thr
   245 250 255
   Glu Gly Ala Ile Asp Leu Gly Val Ala Ser Asn Ala Phe Thr Gln Pro
   260 265 270
   Asp Ala Leu Ala Ser Gln Ile Ile Ala Ser Phe Pro Asn Met Pro Ala
   275 280 285
   Val Ile Asp Gly Ser Gln Met Gln Asp Ala Ile Pro Thr Leu Ala Val
   290 295 300
   Leu Ala Ala Phe Asn Arg Gln Pro Val Arg Phe Val Gly Ile Ala Asn
   305 310 315 320
   Leu Arg Val Lys Glu Cys Asp Arg Ile Ser Ala Leu Ser Asn Gly Leu
   325 330 335
   Cys Ala Ile Ala Pro Gly Leu Ala Val Glu Glu Gly Asp Asp Leu Ile
   340 345 350
   Val Thr Ala Asn Pro Thr Leu Ala Gly Thr Thr Val Asp Ala Leu Ile
   355 360 365
   Asp Thr His Ser Asp His Arg Ile Ala Met Cys Phe Ala Leu Ala Gly
   370 375 380
   Leu Lys Ile Ala Gly Ile Arg Ile Leu Asp Pro Asp Cys Val Ala Lys
   385 390 395 400
   Thr Tyr Pro Gly Tyr Trp Asp Ala Leu Ala Ser Leu Gly Val Ser Val
   405 410 415
   Gln Arg
<210> 20
   <211> 419
   <212> PRT
   <213> Brevundomonas vesicularis
<400> 20
   Met Met Met Gly Arg Ala Lys Leu Thr Ile Ile Pro Pro Gly Lys Pro
   1               5                   10                  15
   Leu Thr Gly Arg Ala Met Pro Pro Gly Ser Lys Ser Ile Thr Asn Arg
               20                  25                  30
   Ala Leu Leu Leu Ala Gly Leu Ala Lys Gly Thr Ser Arg Leu Thr Gly
           35                  40                  45
   Ala Leu Lys Ser Asp Asp Thr Arg Tyr Met Ala Glu Ala Leu Arg Ala
       50                  55                  60
   Met Gly Val Thr Ile Asp Glu Pro Asp Asp Thr Thr Phe Ile Val Lys
   65                  70                  75                  80
   Gly Ser Gly Lys Leu Gln Pro Pro Ala Ala Pro Leu Phe Leu Gly Asn
                   85                  90                  95
   Ala Gly Thr Ala Thr Arg Phe Leu Thr Ala Ala Ala Ala Leu Val Asp
               100                 105                 110
   Gly Lys Val Ile Val Asp Gly Asp Ala His Met Arg Lys Arg Pro Ile
           115                 120                 125
   Gly Pro Leu Val Asp Ala Leu Arg Ser Leu Gly Ile Asp Ala Ser Ala
       130                 135                 140
   Glu Thr Gly Cys Pro Pro Val Thr Ile Asn Gly Thr Gly Arg Phe Glu
   145                 150                 155                 160
   Ala Ser Arg Val Gln Ile Asp Gly Gly Leu Ser Ser Gln Tyr Val Ser
                   165                 170                 175
   Ala Leu Leu Met Met Ala Ala Gly Gly Asp Arg Ala Val Asp Val Glu
               180                 185                 190
   Leu Leu Gly Glu His Ile Gly Ala Leu Gly Tyr Ile Asp Leu Thr Val
           195                 200                 205
   Ala Ala Met Arg Ala Phe Gly Ala Lys Val Glu Arg Val Ser Pro Val
       210                 215                 220
   Ala Trp Arg Val Glu Pro Thr Gly Tyr His Ala Ala Asp Phe Val Ile
   225                 230                 235                 240
   Glu Pro Asp Ala Ser Ala Ala Thr Tyr Leu Trp Ala Ala Glu Val Leu
                   245                 250                 255
   Ser Gly Gly Lys Ile Asp Leu Gly Thr Pro Ala Glu Gln Phe Ser Gln
               260                 265                 270
   Pro Asp Ala Lys Ala Tyr Asp Leu Ile Ser Lys Phe Pro His Leu Pro
           275                 280                 285
   Ala Val Ile Asp Gly Ser Gln Met Gln Asp Ala Ile Pro Thr Leu Ala
       290                 295                 300
   Val Leu Ala Ala Phe Asn Glu Met Pro Val Arg Phe Val Gly Ile Glu
   305                 310                 315                 320
   Asn Leu Arg Val Lys Glu Cys Asp Arg Ile Arg Ala Leu Ser Ser Gly
                   325                 330                 335
   Leu Ser Arg Ile Val Pro Asn Leu Gly Thr Glu Glu Gly Asp Asp Leu
               340                 345                 350
   Ile Ile Ala Ser Asp Pro Ser Leu Ala Gly Lys Ile Leu Thr Ala Glu
           355                 360                 365
   Ile Asp Ser Phe Ala Asp His Arg Ile Ala Met Ser Phe Ala Leu Ala
       370                 375                 380
   Gly Leu Lys Ile Gly Gly Ile Thr Ile Leu Asp Pro Asp Cys Val Ala
   385                 390                 395                 400
   Lys Thr Phe Pro Ser Tyr Trp Asn Val Leu Ser Ser Leu Gly Val Ala
                   405                 410                 415
   Tyr Glu Asp
<210> 21
   <211> 425
   <212> PRT
   <213> Agroibacterium tumifaciens
<220>
   <221> VARIANT
   <222> (0)...(0)
   <223> Strain C58 EPSPS
<400> 21
   Met Ile Glu Leu Thr Ile Thr Pro Pro Gly His Pro Leu Ser Gly Lys
    1               5                  10                  15
   Val Glu Pro Pro Gly Ser Lys Ser Ile Thr Asn Arg Ala Leu Leu Leu
               20                  25                  30
   Ala Gly Leu Ala Lys Gly Lys Ser His Leu Ser Gly Ala Leu Lys Ser
           35                  40                  45
   Asp Asp Thr Leu Tyr Met Ala Glu Ala Leu Arg Glu Met Gly Val Lys
       50                  55                  60
   Val Thr Glu Pro Asp Ala Thr Thr Phe Val Val Glu Gly Thr Gly Val
   65                  70                  75                  80
   Leu Gln Gln Pro Glu Lys Pro Leu Phe Leu Gly Asn Ala Gly Thr Ala
                   85                  90                  95
   Thr Arg Phe Leu Thr Ala Ala Gly Ala Leu Val Asp Gly Ala Val Ile
               100                 105                 110
   Ile Asp Gly Asp Glu His Met Arg Lys Arg Pro Ile Leu Pro Leu Val
           115                 120                 125
   Gln Ala Leu Arg Ala Leu Gly Val Glu Ala Asp Ala Pro Thr Gly Cys
       130                 135                 140
   Pro Pro Val Thr Val Arg Gly Lys Gly Met Gly Phe Pro Lys Gly Ser
   145                 150                 155                 160
   Val Thr Ile Asp Ala Asn Leu Ser Ser Gln Tyr Val Ser Ala Leu Leu
                   165                 170                 175
   Met Ala Ala Ala Cys Gly Asp Lys Pro Val Asp Ile Ile Leu Lys Gly
               180                 185                 190
   Glu Glu Ile Gly Ala Lys Gly Tyr Ile Asp Leu Thr Thr Ser Ala Met
           195                 200                 205
   Glu Ala Phe Gly Ala Lys Val Glu Arg Val Ser Asn Ala Ile Trp Arg
       210                 215                 220
   Val His Pro Thr Gly Tyr Thr Ala Thr Asp Phe His Ile Glu Pro Asp
   225                 230                 235                 240
   Ala Ser Ala Ala Thr Tyr Leu Trp Gly Ala Glu Leu Leu Thr Gly Gly
                   245                 250                 255
   Ala Ile Asp Ile Gly Thr Pro Ala Asp Lys Phe Thr Gln Pro Asp Ala
               260                 265                 270
   Lys Ala Tyr Glu Val Met Ala Gln Phe Pro His Leu Pro Ala Glu Ile
           275                 280                 285
   Asp Gly Ser Gln Met Gln Asp Ala Ile Pro Thr Ile Ala Val Ile Ala
       290                 295                 300
   Ala Phe Asn Glu Thr Pro Val Arg Phe Val Gly Ile Ala Asn Leu Arg
   305                 310                 315                 320
   Val Lys Glu Cys Asp Arg Ile Arg Ala Val Ser Leu Gly Leu Asn Glu
                   325                 330                 335
   Ile Arg Glu Gly Leu Ala His Glu Glu Gly Asp Asp Leu Ile Val His
               340                 345                 350
   Ala Asp Pro Ser Leu Ala Gly Gln Thr Val Asp Ala Ser Ile Asp Thr
           355                 360                 365
   Phe Ala Asp His Arg Ile Ala Met Ser Phe Ala Leu Ala Ala Leu Lys
       370                 375                 380
   Ile Gly Gly Ile Ala Ile Gln Asn Pro Ala Cys Val Ala Lys Thr Tyr
   385                 390                 395                 400
   Pro Gly Tyr Trp Lys Ala Leu Ala Ser Leu Gly Val Asp Tyr Thr Glu
                   405                 410                 415
   Lys Glu Ser Ala Ala Glu Pro Gln His
               420                 425
<210> 22
   <211> 427
   <212> PRT
   <213> Escherichia coli
<400> 22
   Met Glu Ser Leu Thr Leu Gln Pro Ile Ala Arg Val Asp Gly Thr Ile
   1 5 10 15
   Asn Leu Pro Gly Ser Lys Ser Val Ser Asn Arg Ala Leu Leu Leu Ala
   20 25 30
   Ala Leu Ala His Gly Lys Thr Val Leu Thr Asn Leu Leu Asp Ser Asp
   35 40 45
   Asp Val Arg His Met Leu Asn Ala Leu Thr Ala Leu Gly Val Ser Tyr
   50 55 60
   Thr Leu Ser Ala Asp Arg Thr Arg Cys Glu Ile Ile Gly Asn Gly Gly
   65 70 75 80
   Pro Leu His Ala Glu Gly Ala Leu Glu Leu Phe Leu Gly Asn Ala Gly
   85 90 95
   Thr Ala Met Arg Pro Leu Ala Ala Ala Leu Cys Leu Gly Ser Asn Asp
   100 105 110
   Ile Val Leu Thr Gly Glu Pro Arg Met Lys Glu Arg Pro Ile Gly His
   115 120 125
   Leu Val Asp Ala Leu Arg Leu Gly Gly Ala Lys Ile Thr Tyr Leu Glu
   130 135 140
   Gln Glu Asn Tyr Pro Pro Leu Arg Leu Gln Gly Gly Phe Thr Gly Gly
   145 150 155 160
   Asn Val Asp Val Asp Gly Ser Val Ser Ser Gln Phe Leu Thr Ala Leu
   165 170 175
   Leu Met Thr Ala Pro Leu Ala Pro Glu Asp Thr Val Ile Arg Ile Lys
   180 185 190
   Gly Asp Leu Val Ser Lys Pro Tyr Ile Asp Ile Thr Leu Asn Leu Met
   195 200 205
   Lys Thr Phe Gly Val Glu Ile Glu Asn Gln His Tyr Gln Gln Phe Val
   210 215 220
   Val Lys Gly Gly Gln Ser Tyr Gln Ser Pro Gly Thr Tyr Leu Val Glu
   225 230 235 240
   Gly Asp Ala Ser Ser Ala Ser Tyr Phe Leu Ala Ala Ala Ala Ile Lys
   245 250 255
   Gly Gly Thr Val Lys Val Thr Gly Ile Gly Arg Asn Ser Met Gln Gly
   260 265 270
   Asp Ile Arg Phe Ala Asp Val Leu Glu Lys Met Gly Ala Thr Ile Cys
   275 280 285
   Trp Gly Asp Asp Tyr Ile Ser Cys Thr Arg Gly Glu Leu Asn Ala Ile
   290 295 300
   Asp Met Asp Met Asn His Ile Pro Asp Ala Ala Met Thr Ile Ala Thr
   305 310 315 320
   Ala Ala Leu Phe Ala Lys Gly Thr Thr Thr Leu Arg Asn Ile Tyr Asn
   325 330 335
   Trp Arg Val Lys Glu Thr Asp Arg Leu Phe Ala Met Ala Thr Glu Leu
   340 345 350
   Arg Lys Val Gly Ala Glu Val Glu Glu Gly His Asp Tyr Ile Arg Ile
   355 360 365
   Thr Pro Pro Glu Lys Leu Asn Phe Ala Glu Ile Ala Thr Tyr Asn Asp
   370 375 380
   His Arg Met Ala Met Cys Phe Ser Leu Val Ala Leu Ser Asp Thr Pro
   385 390 395 400
   Val Thr Ile Leu Asp Pro Lys Cys Thr Ala Lys Thr Phe Pro Asp Tyr
   405 410 415
   Phe Glu Gln Leu Ala Arg Ile Ser Gln Ala Ala
   420 425
<210> 23
   <211> 423
   <212> PRT
   <213> Salmonella typhimurium
<400> 23
   Met Glu Ser Leu Thr Leu Gln Pro Ile Ala Arg Val Asp Gly Ala Ile
   1               5                   10                  15
   Asn Leu Pro Gly Ser Lys Ser Val Ser Asn Arg Ala Leu Leu Leu Ala
               20                  25                  30
   Ala Leu Ala Cys Gly Lys Thr Ala Leu Thr Asn Leu Leu Asp Ser Asp
           35                  40                  45
   Asp Val Arg His Met Leu Asn Ala Leu Ser Ala Leu Gly Ile Asn Tyr
       50                  55                  60
   Thr Leu Ser Ala Asp Arg Thr Arg Cys Asp Ile Thr Gly Asn Gly Gly
   65                  70                  75                  80
   Ala Leu Arg Ala Pro Gly Ala Leu Glu Leu Phe Leu Gly Asn Ala Gly
                   85                  90                  95
   Thr Ala Met Arg Pro Leu Ala Ala Ala Leu Cys Leu Gly Gln Asn Glu
               100                 105                 110
   Ile Val Leu Thr Gly Glu Pro Arg Met Lys Glu Arg Pro Ile Gly His
           115                 120                 125
   Leu Val Asp Ser Leu Arg Gln Gly Gly Ala Asn Ile Asp Tyr Leu Glu
       130                 135                 140
   Gln Glu Asn Tyr Pro Pro Leu Arg Leu Arg Gly Gly Phe Gly Gly Asp
   145                 150                 155                 160
   Ile Glu Val Asp Gly Ser Val Ser Ser Gln Phe Leu Thr Ala Leu Leu
                   165                 170                 175
   Met Thr Ala Pro Leu Ala Pro Lys Asp Thr Ile Ile Arg Val Lys Gly
               180                 185                 190
   Glu Leu Val Ser Lys Pro Tyr Ile Asp Ile Thr Leu Asn Leu Met Lys
           195                 200                 205
   Thr Phe Gly Val Glu Ile Ala Asn His His Tyr Gln Gln Phe Val Val
       210                 215                 220
   Lys Gly Gly Gln Gln Tyr His Ser Gly Arg Tyr Leu Val Glu Gly Asp
   225                 230                 235                 240
   Ala Ser Ser Ala Ser Tyr Phe Leu Ala Ala Gly Ala Ile Lys Gly Gly
                   245                 250                 255
   Thr Val Lys Val Thr Gly Ile Gly Arg Lys Ser Met Gln Gly Asp Ile
               260                 265                 270
   Arg Phe Ala Asp Val Leu Glu Lys Met Gly Ala Thr Ile Thr Trp Gly
           275                 280                 285
   Asp Asp Phe Ile Ala Cys Thr Arg Gly Glu Leu His Ala Ile Asp Met
       290                 295                 300
   Asp Met Asn His Ile Pro Asp Ala Ala Met Thr Ile Ala Thr Thr Ala
   305                 310                 315                 320
   Leu Phe Ala Lys Gly Thr Thr Thr Leu Arg Asn Ile Tyr Asn Trp Arg
                   325                 330                 335
   Val Lys Glu Thr Asp Arg Leu Phe Ala Met Ala Thr Glu Leu Arg Lys
               340                 345                 350
   Val Gly Ala Glu Val Glu Glu Gly His Asp Tyr Ile Arg Ile Thr Pro
           355                 360                 365
   Pro Ala Lys Leu Gln His Ala Asp Ile Gly Asn Asp His Arg Met Ala
       370                 375                 380
   Met Cys Phe Ser Leu Val Ala Leu Ser Asp Thr Pro Val Thr Ile Leu
   385                 390                 395                 400
   Asp Pro Lys Cys Thr Ala Lys Thr Phe Pro Asp Tyr Phe Glu Gln Leu
                   405                 410                 415
   Ala Arg Met Ser Thr Pro Ala
               420
<210> 24
   <211> 444
   <212> PRT
   <213> zea mays
<400> 24
   Ala Gly Ala Glu Glu Ile Val Leu Gln Pro Ile Lys Glu Ile Ser Gly
   1 5 10 15
   Thr Val Lys Leu Pro Gly Ser Lys Ser Leu Ser Asn Arg Ile Leu Leu
   20 25 30
   Leu Ala Ala Leu Ser Glu Gly Thr Thr Val Val Asp Asn Leu Leu Asn
   35 40 45
   Ser Glu Asp Val His Tyr Met Leu Gly Ala Leu Arg Thr Leu Gly Leu
   50 55 60
   Ser Val Glu Ala Asp Lys Ala Ala Lys Arg Ala Val Val Val Gly Cys
   65 70 75 80
   Gly Gly Lys Phe Pro Val Glu Asp Ala Lys Glu Glu Val Gln Leu Phe
   85 90 95
   Leu Gly Asn Ala Gly Thr Ala Met Arg Pro Leu Thr Ala Ala Val Thr
   100 105 110
   Ala Ala Gly Gly Asn Ala Thr Tyr Val Leu Asp Gly Val Pro Arg Met
   115 120 125
   Arg Glu Arg Pro Ile Gly Asp Leu Val Val Gly Leu Lys Gln Leu Gly
   130 135 140
   Ala Asp Val Asp Cys Phe Leu Gly Thr Asp Cys Pro Pro Val Arg Val
   145 150 155 160
   Asn Gly Ile Gly Gly Leu Pro Gly Gly Lys Val Lys Leu Ser Gly Ser
   165 170 175
   Ile Ser Ser Gln Tyr Leu Ser Ala Leu Leu Met Ala Ala Pro Leu Ala
   180 185 190
   Leu Gly Asp Val Glu Ile Glu Ile Ile Asp Lys Leu Ile Ser Ile Pro
   195 200 205
   Tyr Val Glu Met Thr Leu Arg Leu Met Glu Arg Phe Gly Val Lys Ala
   210 215 220
   Glu His Ser Asp Ser Trp Asp Arg Phe Tyr Ile Lys Gly Gly Gln Lys
   225 230 235 240
   Tyr Lys Ser Pro Lys Asn Ala Tyr Val Glu Gly Asp Ala Ser Ser Ala
   245 250 255
   Ser Tyr Phe Leu Ala Gly Ala Ala Ile Thr Gly Gly Thr Val Thr Val
   260 265 270
   Glu Gly Cys Gly Thr Thr Ser Leu Gln Gly Asp Val Lys Phe Ala Glu
   275 280 285
   Val Leu Glu Met Met Gly Ala Lys Val Thr Trp Thr Glu Thr Ser Val
   290 295 300
   Thr Val Thr Gly Pro Pro Arg Glu Pro Phe Gly Arg Lys His Leu Lys
   305 310 315 320
   Ala Ile Asp Val Asn Met Asn Lys Met Pro Asp Val Ala Met Thr Leu
   325 330 335
   Ala Val Val Ala Leu Phe Ala Asp Gly Pro Thr Ala Ile Arg Asp Val
   340 345 350
   Ala Ser Trp Arg Val Lys Glu Thr Glu Arg Met Val Ala Ile Arg Thr
   355 360 365
   Glu Leu Thr Lys Leu Gly Ala Ser Val Glu Glu Gly Pro Asp Tyr Cys
   370 375 380
   Ile Ile Thr Pro Pro Glu Lys Leu Asn Val Thr Ala Ile Asp Thr Tyr
   385 390 395 400
   Asp Asp His Arg Met Ala Met Ala Phe Ser Leu Ala Ala Cys Ala Glu
   405 410 415
   Val Pro Val Thr Ile Arg Asp Pro Gly Cys Thr Arg Lys Thr Phe Pro
   420 425 430
   Asp Tyr Phe Asp Val Leu Ser Thr Phe Val Lys Asn
   435 440
<210> 25
   <211> 455
   <212> PRT
   <213> Agrobacterium sp. CP4
<400> 25
   Met Ser His Gly Ala Ser Ser Arg Pro Ala Thr Ala Arg Lys Ser Ser
   1 5 10 15
   Gly Leu Ser Gly Thr Val Arg Ile Pro Gly Asp Lys Ser Ile Ser His
   20 25 30
   Arg Ser Phe Met Phe Gly Gly Leu Ala Ser Gly Glu Thr Arg Ile Thr
   35 40 45
   Gly Leu Leu Glu Gly Glu Asp Val Ile Asn Thr Gly Lys Ala Met Gln
   50 55 60
   Ala Met Gly Ala Arg Ile Arg Lys Glu Gly Asp Thr Trp Ile Ile Asp
   65 70 75 80
   Gly Val Gly Asn Gly Gly Leu Leu Ala Pro Glu Ala Pro Leu Asp Phe
   85 90 95
   Gly Asn Ala Ala Thr Gly Cys Arg Leu Thr Met Gly Leu Val Gly Val
   100 105 110
   Tyr Asp Phe Asp Ser Thr Phe Ile Gly Asp Ala Ser Leu Thr Lys Arg
   115 120 125
   Pro Met Gly Arg Val Leu Asn Pro Leu Arg Glu Met Gly Val Gln Val
   130 135 140
   Lys Ser Glu Asp Gly Asp Arg Leu Pro Val Thr Leu Arg Gly Pro Lys
   145 150 155 160
   Thr Pro Thr Pro Ile Thr Tyr Arg Val Pro Met Ala Ser Ala Gln Val
   165 170 175
   Lys Ser Ala Val Leu Leu Ala Gly Leu Asn Thr Pro Gly Ile Thr Thr
   180 185 190
   Val Ile Glu Pro Ile Met Thr Arg Asp His Thr Glu Lys Met Leu Gln
   195 200 205
   Gly Phe Gly Ala Asn Leu Thr Val Glu Thr Asp Ala Asp Gly Val Arg
   210 215 220
   Thr Ile Arg Leu Glu Gly Arg Gly Lys Leu Thr Gly Gln Val Ile Asp
   225 230 235 240
   Val Pro Gly Asp Pro Ser Ser Thr Ala Phe Pro Leu Val Ala Ala Leu
   245 250 255
   Leu Val Pro Gly Ser Asp Val Thr Ile Leu Asn Val Leu Met Asn Pro
   260 265 270
   Thr Arg Thr Gly Leu Ile Leu Thr Leu Gln Glu Met Gly Ala Asp Ile
   275 280 285
   Glu Val Ile Asn Pro Arg Leu Ala Gly Gly Glu Asp Val Ala Asp Leu
   290 295 300
   Arg Val Arg Ser Ser Thr Leu Lys Gly Val Thr Val Pro Glu Asp Arg
   305 310 315 320
   Ala Pro Ser Met Ile Asp Glu Tyr Pro Ile Leu Ala Val Ala Ala Ala
   325 330 335
   Phe Ala Glu Gly Ala Thr Val Met Asn Gly Leu Glu Glu Leu Arg Val
   340 345 350
   Lys Glu Ser Asp Arg Leu Ser Ala Val Ala Asn Gly Leu Lys Leu Asn
   355 360 365
   Gly Val Asp Cys Asp Glu Gly Glu Thr Ser Leu Val Val Arg Gly Arg
   370 375 380
   Pro Asp Gly Lys Gly Leu Gly Asn Ala Ser Gly Ala Ala Val Ala Thr
   385 390 395 400
   His Leu Asp His Arg Ile Ala Met Ser Phe Leu Val Met Gly Leu Val
   405 410 415
   Ser Glu Asn Pro Val Thr Val Asp Asp Ala Thr Met Ile Ala Thr Ser
   420 425 430
   Phe Pro Glu Phe Met Asp Leu Met Ala Gly Leu Gly Ala Lys Ile Glu
   435 440 445
   Leu Ser Asp Thr Lys Ala Ala
   450 455
<210> 26
   <211> 428
   <212> PRT
   <213> Bacillus subtilis
<400> 26
   Met Lys Arg Asp Lys Val Gln Thr Leu His Gly Glu Ile His Ile Pro
    1               5                  10                  15
   Gly Asp Lys Ser Ile Ser His Arg Ser Val Met Phe Gly Ala Leu Ala
               20                  25                  30
   Ala Gly Thr Thr Thr Val Lys Asn Phe Leu Pro Gly Ala Asp Cys Leu
           35                  40                  45
   Ser Thr Ile Asp Cys Phe Arg Lys Met Gly Val His Ile Glu Gln Ser
       50                  55                  60
   Ser Ser Asp Val Val Ile His Gly Lys Gly Ile Asp Ala Leu Lys Glu
   65                  70                  75                  80
   Pro Glu Ser Leu Leu Asp Val Gly Asn Ser Gly Thr Thr Ile Arg Leu
                   85                  90                  95
   Met Leu Gly Ile Leu Ala Gly Arg Pro Phe Tyr Ser Ala Val Ala Gly
               100                 105                 110
   Asp Glu Ser Ile Ala Lys Arg Pro Met Lys Arg Val Thr Glu Pro Leu
           115                 120                 125
   Lys Lys Met Gly Ala Lys Ile Asp Gly Arg Ala Gly Gly Glu Phe Thr
       130                 135                 140
   Pro Leu Ser Val Ser Gly Ala Ser Leu Lys Gly Ile Asp Tyr Val Ser
   145                 150                 155                 160
   Pro Val Ala Ser Ala Gln Ile Lys Ser Ala Val Leu Leu Ala Gly Leu
                   165                 170                 175
   Gln Ala Glu Gly Thr Thr Thr Val Thr Glu Pro His Lys Ser Arg Asp
               180                 185                 190
   His Thr Glu Arg Met Leu Ser Ala Phe Gly Val Lys Leu Ser Glu Asp
           195                 200                 205
   Gln Thr Ser Val Ser Ile Ala Gly Gly Gln Lys Leu Thr Ala Ala Asp
       210                 215                 220
   Ile Phe Val Pro Gly Asp Ile Ser Ser Ala Ala Phe Phe Leu Ala Ala
   225                 230                 235                 240
   Gly Ala Met Val Pro Asn Ser Arg Ile Val Leu Lys Asn Val Gly Leu
                   245                 250                 255
   Asn Pro Thr Arg Thr Gly Ile Ile Asp Val Leu Gln Asn Met Gly Ala
               260                 265                 270
   Lys Leu Glu Ile Lys Pro Ser Ala Asp Ser Gly Ala Glu Pro Tyr Gly
           275                 280                 285
   Asp Leu Ile Ile Glu Thr Ser Ser Leu Lys Ala Val Glu Ile Gly Gly
       290                 295                 300
   Asp Ile Ile Pro Arg Leu Ile Asp Glu Ile Pro Ile Ile Ala Leu Leu
   305                 310                 315                 320
   Ala Thr Gln Ala Glu Gly Thr Thr Val Ile Lys Asp Ala Ala Glu Leu
                   325                 330                 335
   Lys Val Lys Glu Thr Asn Arg Ile Asp Thr Val Val Ser Glu Leu Arg
               340                 345                 350
   Lys Leu Gly Ala Glu Ile Glu Pro Thr Ala Asp Gly Met Lys Val Tyr
           355                 360                 365
   Gly Lys Gln Thr Leu Lys Gly Gly Ala Ala Val Ser Ser His Gly Asp
       370                 375                 380
   His Arg Ile Gly Met Met Leu Gly Ile Ala Ser Cys Ile Thr Glu Glu
   385                 390                 395                 400
   Pro Ile Glu Ile Glu His Thr Asp Ala Ile His Val Ser Tyr Pro Thr
                   405                 410                 415
   Phe Phe Glu His Leu Asn Lys Leu Ser Lys Lys Ser
                       420                 425
<210> 27
   <211> 427
   <212> PRT
   <213> K. pneumoniae
<400> 27
   Met Glu Ser Leu Thr Leu Gln Pro Ile Ala Arg Val Asp Gly Thr Val
    1               5                  10                  15
   Asn Leu Pro Gly Ser Lys Ser Val Ser Asn Arg Ala Leu Leu Leu Ala
               20                  25                  30
   Ala Leu Ala Arg Gly Thr Thr Val Leu Thr Asn Leu Leu Asp Ser Asp
           35                  40                  45
   Asp Val Arg His Met Leu Asn Ala Leu Ser Ala Leu Gly Val His Tyr
       50                  55                  60
   Val Leu Ser Ser Asp Arg Thr Arg Cys Glu Val Thr Gly Thr Gly Gly
   65                  70                  75                  80
   Pro Leu Gln Ala Gly Ser Ala Leu Glu Leu Phe Leu Gly Asn Ala Gly
                   85                  90                  95
   Thr Ala Met Arg Pro Leu Ala Ala Ala Leu Cys Leu Gly Ser Asn Asp
               100                 105                 110
   Ile Val Leu Thr Gly Glu Pro Arg Met Lys Glu Arg Pro Ile Gly His
           115                 120                 125
   Leu Val Asp Ala Leu Arg Gln Gly Gly Ala Gln Ile Asp Tyr Leu Glu
       130                 135                 140
   Gln Glu Asn Tyr Pro Pro Leu Arg Leu Arg Gly Gly Phe Thr Gly Gly
   145                 150                 155                 160
   Asp Val Glu Val Asp Gly Ser Val Ser Ser Gln Phe Leu Thr Ala Leu
                   165                 170                 175
   Leu Met Ala Ser Pro Leu Ala Pro Gln Asp Thr Val Ile Ala Ile Lys
               180                 185                 190
   Gly Glu Leu Val Ser Arg Pro Tyr Ile Asp Ile Thr Leu His Leu Met
           195                 200                 205
   Lys Thr Phe Gly Val Glu Val Glu Asn Gln Ala Tyr Gln Arg Phe Ile
       210                 215                 220
   Val Arg Gly Asn Gln Gln Tyr Gln Ser Pro Gly Asp Tyr Leu Val Glu
   225                 230                 235                 240
   Gly Asp Ala Ser Ser Ala Ser Tyr Phe Leu Ala Ala Gly Ala Ile Lys
                   245                 250                 255
   Gly Gly Thr Val Lys Val Thr Gly Ile Gly Arg Asn Ser Val Gln Gly
               260                 265                 270
   Asp Ile Arg Phe Ala Asp Val Leu Glu Lys Met Gly Ala Thr Val Thr
           275                 280                 285
   Trp Gly Glu Asp Tyr Ile Ala Cys Thr Arg Gly Glu Leu Asn Ala Ile
       290                 295                 300
   Asp Met Asp Met Asn His Ile Pro Asp Ala Ala Met Thr Ile Ala Thr
   305                 310                 315                 320
   Ala Ala Leu Phe Ala Arg Gly Thr Thr Thr Leu Arg Asn Ile Tyr Asn
                   325                 330                 335
   Trp Arg Val Lys Glu Thr Asp Arg Leu Phe Ala Met Ala Thr Glu Leu
               340                 345                 350
   Arg Lys Val Gly Ala Glu Val Glu Glu Gly Glu Asp Tyr Ile Arg Ile
           355                 360                 365
   Thr Pro Pro Leu Thr Leu Gln Phe Ala Glu Ile Gly Thr Tyr Asn Asp
       370                 375                 380
   His Arg Met Ala Met Cys Phe Ser Leu Val Ala Leu Ser Asp Thr Pro
   385                 390                 395                 400
   Val Thr Ile Leu Asp Pro Lys Cys Thr Ala Lys Thr Phe Pro Asp Tyr
                   405                 410                 415
   Phe Gly Gln Leu Ala Arg Ile Ser Thr Leu Ala
               420                 425
<210> 28
   <211> 439
   <212> PRT
   <213> Clostridium tetani
<400> 28
   Met His Lys Glu Glu Thr Phe Asn Gln Cys Ala Leu Thr Ile Asn Gly
   1 5 10 15
   Tyr Lys Ser Glu Val Lys Lys Thr Tyr Glu Leu Pro Gly Asp Lys Ser
   20 25 30
   Val Gly His Arg Ser Leu Leu Ile Gly Ala Leu Pro Lys Gly Glu Tyr
   35 40 45
   Lys Ile Arg Asn Phe Pro Gln Ser Arg Asp Cys Leu Thr Thr Leu Lys
   50 55 60
   Ile Met Glu Glu Leu Gly Val Lys Val Lys Val Leu Lys Asp Tyr Ile
   65 70 75 80
   Leu Val Asn Ser Pro Gly Tyr Glu Asn Phe Lys Lys Lys Ile Asp Tyr
   85 90 95
   Ile Asp Cys Gly Asn Ser Gly Thr Thr Ser Arg Leu Ile Ala Gly Ile
   100 105 110
   Leu Ala Gly Val Gly Val Glu Thr Asn Leu Val Gly Asp Lys Ser Leu
   115 120 125
   Ser Ile Arg Pro Met Lys Arg Ile Val Asp Pro Leu Asn Ser Met Gly
   130 135 140
   Ala Asn Ile Glu Met Glu Lys Asp His Met Pro Leu Ile Phe Lys Gly
   145 150 155 160
   Asn Gly Glu Leu Lys Gly Ile Asp Tyr Thr Met Glu Ile Ala Ser Ala
   165 170 175
   Gln Val Lys Ser Cys Ile Leu Leu Ala Gly Phe Leu Ser Glu Gly Val
   180 185 190
   Thr Lys Val Arg Glu Leu Ser Pro Thr Arg Asp His Thr Glu Arg Met
   195 200 205
   Leu Lys Tyr Ile Glu Gly Asn Ile Lys Ile Glu Asn Lys Glu Ile Glu
   210 215 220
   Ile Glu Asn Ser Thr Ile Lys Ser Lys Asp Ile Tyr Val Pro Gly Asp
   225 230 235 240
   Ile Ser Ser Ala Ala Tyr Ile Ile Ala Cys Ala Ile Leu Gly Glu Asp
   245 250 255
   Cys Glu Ile Ile Leu Glu Asn Val Leu Leu Asn Glu Asn Arg Arg Lys
   260 265 270
   Tyr Leu Asp Leu Leu Lys Lys Met Gly Ala Asn Leu Lys Tyr Leu Glu
   275 280 285
   Lys Asn Gln Cys Asn Gly Glu His Val Gly Asn Ile Leu Val Lys Ser
   290 295 300
   Ser Phe Leu Lys Gly Ile Ser Ile Gly Lys Glu Ile Thr Pro Tyr Ile
   305 310 315 320
   Ile Asp Glu Ile Pro Ile Ile Ser Leu Ile Ala Ser Phe Ala Glu Gly
   325 330 335
   Lys Thr Ile Phe Glu Asn Val Glu Glu Leu Lys Tyr Lys Glu Ser Asp
   340 345 350
   Arg Ile Lys Ala Ile Met Val Asn Leu Lys Ser Leu Gly Val Lys Thr
   355 360 365
   Glu Leu Val Glu Asn Asn Leu Ile Ile Tyr Gly Gly Leu Ser Lys Ile
   370 375 380
   Asn Lys Glu Ile Asn Ile Arg Thr Phe Asn Asp His Arg Ile Ala Leu
   385 390 395 400
   Thr Phe Leu Cys Ser Ala Met Arg Asn Ser Glu Lys Thr Tyr Ile Asp
   405 410 415
   Asn Trp Asp Cys Val Ala Ile Ser Phe Pro Asn Ser Leu Asn Tyr Phe
   420 425 430
   Lys Asp Phe Phe Arg Ile Asn
   435
<210> 29
   <211> 6
   <212> PRT
   <213> Artificial Sequence
<220>
   <223> Conserved Domains
   <221> VARIANT
   <222> 3
   <223> Xaa= Gly, Ser, Ala or Asn
<221> VARIANT
   <222> 4
   <223> Xaa= Asn or Glu
<400> 29
   Asp Cys Xaa Xaa Ser Gly
   1               5
<210> 30
   <211> 6
   <212> PRT
   <213> Artificial Sequence
<220>
   <223> Conserved Domains
<221> VARIANT
   <222> 3
   <223> Xaa= Ala or Arg
<221> VARIANT
   <222> 4
   <223> Xaa= Asn or Glu
<400> 30
   Asp Ala Xaa Xaa Ser Gly
   1               5
<210> 31
   <211> 6
   <212> PRT
   <213> Artificial sequence
<220>
   <223> Conserved Domains
<221> VARIANT
   <222> 2
   <223> Xaa= Gly, Asn, or Glu
<400> 31
   Lys Leu Lys Xaa Ser Ala
   1               5
<210> 32
   <211> 6
   <212> PRT
   <213> Artificial sequence
<220>
   <223> Conserved Domains
<400> 32
   Trp Cys Glu Asp Ala Gly
   1               5
<210> 33
   <211> 10
   <212> PRT
   <213> Artificial Sequence
<220>
   <223> conserved Domains
<221> VARIANT
   <222> 3, 4, 7, 9
   <223> Xaa= Any amino acid
<221> VARIANT
   <222> 8
   <223> Xaa= Ser or Thr
<400> 33
   Asp Cys Xaa Xaa Ser Gly Xaa Xaa Xaa Arg
   1               5                   10
<210> 34
   <211> 10
   <212> PRT
   <213> Artificial Sequence
<220>
   <223> conserved Domains
<221> VARIANT
   <222> 1, 3, 4, 7, 9
   <223> Xaa= Any amino acid
<221> VARIANT
   <222> 8
   <223> Xaa= Ser or Thr
<400> 34
   Xaa Cys Xaa Xaa Ser Gly Xaa Xaa Xaa Arg
   1               5                   10
<210> 35
   <211> 2
   <212> PRT
   <213> Artificial sequence
<220>
   <223> Conserved Domains
<221> VARIANT
   <222> 2
   <223> Xaa= Ile or Leu
<400> 35
   Pro Xaa
   1
<210> 36
   <211> 10
   <212> PRT
   <213> Artificial Sequence
<220>
   <223> Conserved Domains
<221> VARIANT
   <222> 3
   <223> Xaa= Ser or Thr
<221> VARIANT
   <222> 4
   <223> xaa= Gln or Asp
<221> VARIANT
   <222> 8
   <223> Xaa= Ala, Leu, Met, Ile or Val
<221> VARIANT
   <222> 9
   <223> xaa= Phe, Ala, Leu, Met, Ile or Val
<400> 36
   Asp Ala Xaa Xaa Cys Pro Asp Xaa Xaa Pro
    1               5                  10
<210> 37
   <211> 2
   <212> PRT
   <213> Artificial sequence
<220>
   <223> conserved Domains
<400> 37
   Leu Lys
    1
<210> 38
   <211> 1320
   <212> DNA
   <213> Clostridium tetani
<220>
   <221> CDS
   <222> (1)...(1320)
<400> 38
   atg cat aag gaa gaa act ttt aac cag tgt gca ctt act att aat gga     48
   Met His Lys Glu Glu Thr Phe Asn Gln Cys Ala Leu Thr Ile Asn Gly
    1               5                  10                  15
   tac aag tcc gag gtt aaa aag acc tat gaa ctt cca ggt gat aaa tct     96
   Tyr Lys Ser Glu Val Lys Lys Thr Tyr Glu Leu Pro Gly Asp Lys Ser
               20                  25                  30
   gta ggt cat agg tct ctt tta att gga gcc ttg cca aaa gga gaa tat     144
   Val Gly His Arg Ser Leu Leu Ile Gly Ala Leu Pro Lys Gly Glu Tyr
           35                  40                  45
   aaa ata aga aat ttt cct caa agt aga gat tgt tta act act ttg aaa     192
   Lys Ile Arg Asn Phe Pro Gln Ser Arg Asp Cys Leu Thr Thr Leu Lys
       50                  55                  60
   ata atg gaa gag cta ggt gtg aaa gtt aaa gtt ctt aaa gat tat ata     240
   Ile Met Glu Glu Leu Gly Val Lys Val Lys Val Leu Lys Asp Tyr Ile
    65                  70                  75                  80
   tta gta aac tca ccg ggg tat gaa aat ttt aaa aag aaa att gat tat     288
   Leu Val Asn Ser Pro Gly Tyr Glu Asn Phe Lys Lys Lys Ile Asp Tyr
                    85                  90                  95
   ata gac tgt gga aat tct gga act act tca agg ctt ata gca ggt ata     336
   Ile Asp Cys Gly Asn Ser Gly Thr Thr Ser Arg Leu Ile Ala Gly Ile
               100                 105                 110
   tta gca ggt gta gga gtg gaa act aat tta gta ggt gat aaa tcc ctc      384
   Leu Ala Gly Val Gly Val Glu Thr Asn Leu Val Gly Asp Lys Ser Leu
           115                 120                 125
   tct ata aga cct atg aaa aga ata gta gac cct cta aat tct atg gga      432
   Ser Ile Arg Pro Met Lys Arg Ile Val Asp Pro Leu Asn Ser Met Gly
       130                 135                 140
   gct aat ata gag atg gaa aaa gat cat atg ccc tta att ttt aaa ggt      480
   Ala Asn Ile Glu Met Glu Lys Asp His Met Pro Leu Ile Phe Lys Gly
   145                 150                 155                 160
   aat gga gaa cta aag ggt att gat tat act atg gaa att gcc tct gcc      528
   Asn Gly Glu Leu Lys Gly Ile Asp Tyr Thr Met Glu Ile Ala Ser Ala
                   165                 170                 175
   cag gtg aaa tcc tgc att tta tta gct gga ttt tta tca gaa ggt gtt      576
   Gln Val Lys Ser Cys Ile Leu Leu Ala Gly Phe Leu Ser Glu Gly Val
               180                 185                 190
   aca aag gta aga gaa tta agt cct aca aga gat cac aca gaa aga atg      624
   Thr Lys Val Arg Glu Leu Ser Pro Thr Arg Asp His Thr Glu Arg Met
           195                 200                 205
   tta aaa tac ata gaa ggg aat ata aaa ata gaa aat aaa gaa ata gaa      672
   Leu Lys Tyr Ile Glu Gly Asn Ile Lys Ile Glu Asn Lys Glu Ile Glu
       210                 215                 220
   atc gaa aat tct acc ata aag agt aaa gat att tat gtt cca gga gat      720
   Ile Glu Asn Ser Thr Ile Lys Ser Lys Asp Ile Tyr Val Pro Gly Asp
   225                 230                 235                 240
   ata tct tca gca gca tat att ata gcc tgt gcc ata tta gga gaa gac      768
   Ile Ser Ser Ala Ala Tyr Ile Ile Ala Cys Ala Ile Leu Gly Glu Asp
                   245                 250                 255
   tgt gaa att att tta gaa aat gta ttg ttg aat gag aat aga aga aaa      816
   Cys Glu Ile Ile Leu Glu Asn Val Leu Leu Asn Glu Asn Arg Arg Lys
               260                 265                 270
   tac ttg gac tta tta aag aaa atg gga gct aac tta aag tac tta gag      864
   Tyr Leu Asp Leu Leu Lys Lys Met Gly Ala Asn Leu Lys Tyr Leu Glu
           275                 280                 285
   aaa aat cag tgt aat gga gaa cat gta ggt aat att tta gtt aag agt      912
   Lys Asn Gln Cys Asn Gly Glu His Val Gly Asn Ile Leu Val Lys Ser
       290                 295                 300
   agt ttt tta aag ggt ata agt ata gga aaa gaa att acg cct tat ata      960
   Ser Phe Leu Lys Gly Ile Ser Ile Gly Lys Glu Ile Thr Pro Tyr Ile
   305                 310                 315                 320
   ata gat gaa ata cct ata ata tcc ctt ata gcc tcc ttt gca gaa gga     1008
   Ile Asp Glu Ile Pro Ile Ile Ser Leu Ile Ala Ser Phe Ala Glu Gly
                   325                 330                 335
   aag acc ata ttt gaa aat gta gag gag tta aag tac aaa gaa agt gat     1056
   Lys Thr Ile Phe Glu Asn Val Glu Glu Leu Lys Tyr Lys Glu Ser Asp
               340                 345                 350
   aga ata aag gca att atg gtg aat tta aag tca ctt ggg gta aaa aca     1104
   Arg Ile Lys Ala Ile Met Val Asn Leu Lys Ser Leu Gly Val Lys Thr
           355                 360                 365
   gaa tta gta gaa aat aat tta att atc tat gga gga ctt tct aag ata     1152
   Glu Leu Val Glu Asn Asn Leu Ile Ile Tyr Gly Gly Leu Ser Lys Ile
       370                 375                 380
   aat aaa gaa att aat att aga acc ttt aat gat cac aga ata gca tta      1200
   Asn Lys Glu Ile Asn Ile Arg Thr Phe Asn Asp His Arg Ile Ala Leu
   385                 390                 395                 400
   act ttt ttg tgt tca gct atg aga aat agt gaa aaa act tat ata gat      1248
   Thr Phe Leu Cys Ser Ala Met Arg Asn Ser Glu Lys Thr Tyr Ile Asp
                   405                 410                 415
   aat tgg gat tgt gta gcc ata tcc ttt cca aat tct ttg aat tat ttt      1296
   Asn Trp Asp Cys Val Ala Ile Ser Phe Pro Asn Ser Leu Asn Tyr Phe
               420                 425                 430
   aag gat ttt ttc aga ata aat taa                                      1320
   Lys Asp Phe Phe Arg Ile Asn  *
           435
<210> 39
   <211> 439
   <212> PRT
   <213> Clostridium tetani
<400> 39
   Met His Lys Glu Glu Thr Phe Asn Gln Cys Ala Leu Thr Ile Asn Gly
   1 5 10 15
   Tyr Lys Ser Glu Val Lys Lys Thr Tyr Glu Leu Pro Gly Asp Lys Ser
   20 25 30
   Val Gly His Arg Ser Leu Leu Ile Gly Ala Leu Pro Lys Gly Glu Tyr
   35 40 45
   Lys Ile Arg Asn Phe Pro Gln Ser Arg Asp Cys Leu Thr Thr Leu Lys
   50 55 60
   Ile Met Glu Glu Leu Gly Val Lys Val Lys Val Leu Lys Asp Tyr Ile
   65 70 75 80
   Leu Val Asn Ser Pro Gly Tyr Glu Asn Phe Lys Lys Lys Ile Asp Tyr
   85 90 95
   Ile Asp Cys Gly Asn Ser Gly Thr Thr Ser Arg Leu Ile Ala Gly Ile
   100 105 110
   Leu Ala Gly Val Gly Val Glu Thr Asn Leu Val Gly Asp Lys Ser Leu
   115 120 125
   Ser Ile Arg Pro Met Lys Arg Ile Val Asp Pro Leu Asn Ser Met Gly
   130 135 140
   Ala Asn Ile Glu Met Glu Lys Asp His Met Pro Leu Ile Phe Lys Gly
   145 150 155 160
   Asn Gly Glu Leu Lys Gly Ile Asp Tyr Thr Met Glu Ile Ala Ser Ala
   165 170 175
   Gln Val Lys Ser Cys Ile Leu Leu Ala Gly Phe Leu Ser Glu Gly Val
   180 185 190
   Thr Lys Val Arg Glu Leu Ser Pro Thr Arg Asp His Thr Glu Arg Met
   195 200 205
   Leu Lys Tyr Ile Glu Gly Asn Ile Lys Ile Glu Asn Lys Glu Ile Glu
   210 215 220
   Ile Glu Asn Ser Thr Ile Lys Ser Lys Asp Ile Tyr Val Pro Gly Asp
   225 230 235 240
   Ile Ser Ser Ala Ala Tyr Ile Ile Ala Cys Ala Ile Leu Gly Glu Asp
   245 250 255
   Cys Glu Ile Ile Leu Glu Asn Val Leu Leu Asn Glu Asn Arg Arg Lys
   260 265 270
   Tyr Leu Asp Leu Leu Lys Lys Met Gly Ala Asn Leu Lys Tyr Leu Glu
   275 280 285
   Lys Asn Gln Cys Asn Gly Glu His Val Gly Asn Ile Leu Val Lys Ser
   290 295 300
   Ser Phe Leu Lys Gly Ile Ser Ile Gly Lys Glu Ile Thr Pro Tyr Ile
   305 310 315 320
   Ile Asp Glu Ile Pro Ile Ile Ser Leu Ile Ala Ser Phe Ala Glu Gly
   325 330 335
   Lys Thr Ile Phe Glu Asn Val Glu Glu Leu Lys Tyr Lys Glu Ser Asp
   340 345 350
   Arg Ile Lys Ala Ile Met Val Asn Leu Lys Ser Leu Gly Val Lys Thr
   355 360 365
   Glu Leu Val Glu Asn Asn Leu Ile Ile Tyr Gly Gly Leu Ser Lys Ile
   370 375 380
   Asn Lys Glu Ile Asn Ile Arg Thr Phe Asn Asp His Arg Ile Ala Leu
   385 390 395 400
   Thr Phe Leu Cys Ser Ala Met Arg Asn Ser Glu Lys Thr Tyr Ile Asp
   405 410 415
   Asn Trp Asp Cys Val Ala Ile Ser Phe Pro Asn Ser Leu Asn Tyr Phe
   420 425 430
   Lys Asp Phe Phe Arg Ile Asn
   435
<210> 40
   <211> 1293 <212> DNA
   <213> Methanosarcina mazei
<220>
   <221> CDS
   <222> (1)...(1293)
<400> 40
   atg cgc gcc tca att agc aaa tcc tca atc aaa ggg gag gtc ttt gcc     48
   Met Arg Ala Ser Ile Ser Lys Ser Ser Ile Lys Gly Glu Val Phe Ala
    1               5                  10                  15
   cct cct tca aag agt tac acc cac agg gct ata act ctc gca gcc ctt     96
   Pro Pro Ser Lys Ser Tyr Thr His Arg Ala Ile Thr Leu Ala Ala Leu
               20                  25                  30
   tca aaa gaa tcg atc att cac cgt ccc ctc ctt tcc gct gat act ctt    144
   Ser Lys Glu Ser Ile Ile His Arg Pro Leu Leu Ser Ala Asp Thr Leu
           35                  40                  45
   gct aca atc aga gct tct gag atg ttc gga gcc gcg gtt aga cgg gag    192
   Ala Thr Ile Arg Ala Ser Glu Met Phe Gly Ala Ala Val Arg Arg Glu
       50                  55                  60
   aaa gaa aat ctc atc atc cag gga tct aat gga aag ccc ggt att cct    240
   Lys Glu Asn Leu Ile Ile Gln Gly Ser Asn Gly Lys Pro Gly Ile Pro
    65                  70                  75                  80
   gat gat gta att gat gcc gca aat tca ggg aca acc ctc cgc ttt atg    288
   Asp Asp Val Ile Asp Ala Ala Asn Ser Gly Thr Thr Leu Arg Phe Met
                    85                  90                  95
   aca gca ata gca ggc tta act gac gga atc act gta ctt aca gga gac    336
   Thr Ala Ile Ala Gly Leu Thr Asp Gly Ile Thr Val Leu Thr Gly Asp
               100                 105                 110
   tca tct ctt cgc acg cgt cca aac gga cct ctt ctt gaa gtt ctc aac    384
   Ser Ser Leu Arg Thr Arg Pro Asn Gly Pro Leu Leu Glu Val Leu Asn
           115                 120                 125
   agg ctg gga gca aaa gcc tgt tct acg cga gga aac gaa aga gcg cct    432
   Arg Leu Gly Ala Lys Ala Cys Ser Thr Arg Gly Asn Glu Arg Ala Pro
       130                 135                 140
   att gtg gtc aaa gga gga att aag gga tct gaa gtg gaa ata agc ggc    480
   Ile Val Val Lys Gly Gly Ile Lys Gly Ser Glu Val Glu Ile Ser Gly
   145                 150                 155                 160
   tcg atc agc tcc cag ttt atc tct gct ctt ctt ata gcc tgc ccg ctt    528
   Ser Ile Ser Ser Gln Phe Ile Ser Ala Leu Leu Ile Ala Cys Pro Leu
                   165                 170                 175
   gct gaa aac agc acc act ctt tcc att ata gga aaa ctg aag tca aga    576
   Ala Glu Asn Ser Thr Thr Leu Ser Ile Ile Gly Lys Leu Lys Ser Arg
               180                 185                 190
   cct tat gtt gac gtg acc ata gaa atg ctc ggg ctg gca gga gtc aaa      624
   Pro Tyr Val Asp Val Thr Ile Glu Met Leu Gly Leu Ala Gly Val Lys
           195                 200                 205
   atc cat aca gat gat aat aac ggc acg aaa ttt atc atc ccc gga aaa      672
   Ile His Thr Asp Asp Asn Asn Gly Thr Lys Phe Ile Ile Pro Gly Lys
       210                 215                 220
   cag aaa tac gac ctg aaa caa tac acg gtt ccc gga gac ttt tct tct      720
   Gln Lys Tyr Asp Leu Lys Gln Tyr Thr Val Pro Gly Asp Phe Ser Ser
   225                 230                 235                 240
   gct tcc tac ctg cta gca gct gca gcc atg ctt gaa ggc tcc gaa atc      768
   Ala Ser Tyr Leu Leu Ala Ala Ala Ala Met Leu Glu Gly Ser Glu Ile
                   245                 250                 255
   aca gtc aaa aat cta ttc cct tca aaa cag gga gat aaa gtg att att      816
   Thr Val Lys Asn Leu Phe Pro Ser Lys Gln Gly Asp Lys Val Ile Ile
               260                 265                 270
   gat act ctc aaa cag atg gga gca gac ata aca tgg gac atg gaa gct      864
   Asp Thr Leu Lys Gln Met Gly Ala Asp Ile Thr Trp Asp Met Glu Ala
           275                 280                 285
   ggc att gtg acc gta aga gga gga aga aaa tta aaa gcc att acc ttt      912
   Gly Ile Val Thr Val Arg Gly Gly Arg Lys Leu Lys Ala Ile Thr Phe
       290                 295                 300
   gat gcc gga tca acc cct gac ctt gta ccg act gtt gcc gtc ctt gct      960
   Asp Ala Gly Ser Thr Pro Asp Leu Val Pro Thr Val Ala Val Leu Ala
   305                 310                 315                 320
   tca gtt gcc gaa ggg acc agc aga ata gaa aac gcc gag cat gtc cgc     1008
   Ser Val Ala Glu Gly Thr Ser Arg Ile Glu Asn Ala Glu His Val Arg
                   325                 330                 335
   tat aaa gaa aca gac cgg ctt cac gcc ctt gcg acc gag ctt ccg aaa     1056
   Tyr Lys Glu Thr Asp Arg Leu His Ala Leu Ala Thr Glu Leu Pro Lys
               340                 345                 350
   atg gga gtc tcc ctc aaa gaa gaa atg gac agc ctg aca atc acc gga     1104
   Met Gly Val Ser Leu Lys Glu Glu Met Asp Ser Leu Thr Ile Thr Gly
           355                 360                 365
   ggg act ctt gag gga gcc gaa gtc cac ggc tgg gac gac cac cgg att     1152
   Gly Thr Leu Glu Gly Ala Glu Val His Gly Trp Asp Asp His Arg Ile
       370                 375                 380
   gtg atg tct cta gct ata gca ggc atg gtt gca gga aac acg ata gtt     1200
   Val Met Ser Leu Ala Ile Ala Gly Met Val Ala Gly Asn Thr Ile Val
   385                 390                 395                 400
   gac acc act gag tct gta tcg ata tcc tat cct gat ttc ttt aaa gat     1248
   Asp Thr Thr Glu Ser Val Ser Ile Ser Tyr Pro Asp Phe Phe Lys Asp
                   405                 410                 415
   atg cga aac ctt gga gca aaa gtc aag gag att cct gaa gaa taa         1293
   Met Arg Asn Leu Gly Ala Lys Val Lys Glu Ile Pro Glu Glu  *
               420                 425                 430
<210> 41
   <211> 430
   <212> PRT
   <213> Methanosarcina mazei
<400> 41
   Met Arg Ala Ser Ile Ser Lys Ser Ser Ile Lys Gly Glu Val Phe Ala
    1               5                  10                  15
   Pro Pro Ser Lys Ser Tyr Thr His Arg Ala Ile Thr Leu Ala Ala Leu
               20                  25                  30
   Ser Lys Glu Ser Ile Ile His Arg Pro Leu Leu Ser Ala Asp Thr Leu
           35                  40                  45
   Ala Thr Ile Arg Ala Ser Glu Met Phe Gly Ala Ala Val Arg Arg Glu
       50                  55                  60
   Lys Glu Asn Leu Ile Ile Gln Gly Ser Asn Gly Lys Pro Gly Ile Pro
   65                  70                  75                  80
   Asp Asp Val Ile Asp Ala Ala Asn Ser Gly Thr Thr Leu Arg Phe Met
                   85                  90                  95
   Thr Ala Ile Ala Gly Leu Thr Asp Gly Ile Thr Val Leu Thr Gly Asp
               100                 105                 110
   Ser Ser Leu Arg Thr Arg Pro Asn Gly Pro Leu Leu Glu Val Leu Asn
           115                 120                 125
   Arg Leu Gly Ala Lys Ala Cys Ser Thr Arg Gly Asn Glu Arg Ala Pro
       130                 135                 140
   Ile Val Val Lys Gly Gly Ile Lys Gly Ser Glu Val Glu Ile Ser Gly
   145                 150                 155                 160
   Ser Ile Ser Ser Gln Phe Ile Ser Ala Leu Leu Ile Ala Cys Pro Leu
                   165                 170                 175
   Ala Glu Asn Ser Thr Thr Leu Ser Ile Ile Gly Lys Leu Lys Ser Arg
               180                 185                 190
   Pro Tyr Val Asp Val Thr Ile Glu Met Leu Gly Leu Ala Gly Val Lys
           195                 200                 205
   Ile His Thr Asp Asp Asn Asn Gly Thr Lys Phe Ile Ile Pro Gly Lys
       210                 215                 220
   Gln Lys Tyr Asp Leu Lys Gln Tyr Thr Val Pro Gly Asp Phe Ser Ser
   225                 230                 235                 240
   Ala Ser Tyr Leu Leu Ala Ala Ala Ala Met Leu Glu Gly Ser Glu Ile
                   245                 250                 255
   Thr Val Lys Asn Leu Phe Pro Ser Lys Gln Gly Asp Lys Val Ile Ile
               260                 265                 270
   Asp Thr Leu Lys Gln Met Gly Ala Asp Ile Thr Trp Asp Met Glu Ala
           275                 280                 285
   Gly Ile Val Thr Val Arg Gly Gly Arg Lys Leu Lys Ala Ile Thr Phe
       290                 295                 300
   Asp Ala Gly Ser Thr Pro Asp Leu Val Pro Thr Val Ala Val Leu Ala
   305                 310                 315                 320
   Ser Val Ala Glu Gly Thr Ser Arg Ile Glu Asn Ala Glu His Val Arg
                   325                 330                 335
   Tyr Lys Glu Thr Asp Arg Leu His Ala Leu Ala Thr Glu Leu Pro Lys
               340                 345                 350
   Met Gly Val Ser Leu Lys Glu Glu Met Asp Ser Leu Thr Ile Thr Gly
           355                 360                 365
   Gly Thr Leu Glu Gly Ala Glu Val His Gly Trp Asp Asp His Arg Ile
       370                 375                 380
   Val Met Ser Leu Ala Ile Ala Gly Met Val Ala Gly Asn Thr Ile Val
   385                 390                 395                 400
   Asp Thr Thr Glu Ser Val Ser Ile Ser Tyr Pro Asp Phe Phe Lys Asp
                   405                 410                 415
   Met Arg Asn Leu Gly Ala Lys Val Lys Glu Ile Pro Glu Glu
               420                 425                 430
<210> 42
   <211> 38
   <212> DNA
   <213> Artificial Sequence
<220>
   <223> oligonucleotide primer
<400> 42
   cagggatccg ccatgaattg tgttaaaata aatccatg 38
<210> 43
   <211> 32
   <212> DNA
   <213> Artificial Sequence
<220>
   <223> Oligonucleotide primer
<400> 43
   cagggcgcgc cttattcccc caaactccac tc 32
<210> 44
   <211> 35
   <212> DNA
   <213> Artificial Sequence
<220>
   <223> Oligonucleotide primer
<400> 44
   cagggatccg ccatgattgt aaagatttat ccatc 35
<210> 45
   <211> 36
   <212> DNA
   <213> Artificial Sequence
<220>
   <223> Oligonucleotide primer
<400> 45
   cagggcgcgc cggtctcatt caatagaaat cttcgc 36
<210> 46
   <211> 418
   <212> PRT
   <213> Rhodobacter sphaeroides
<400> 46
   Leu Lys Gly Arg Ala Glu Ile Pro Gly Asp Lys Ser Ile Ser His Arg
    1               5                  10                  15
   Ala Leu Ile Leu Gly Ala Met Ala Val Gly Glu Thr Arg Ile Thr Gly
               20                  25                  30
   Leu Leu Glu Gly Gln Asp Val Leu Asp Thr Ala Lys Ala Met Arg Ala
           35                  40                  45
   Phe Gly Ala Glu Val Ile Gln His Gly Pro Gly Ala Trp Ser Val His
       50                  55                  60
   Gly Val Gly Val Gly Gly Phe Thr Glu Pro Ala Glu Val Ile Asp Cys
   65                  70                  75                  80
   Gly Asn Ser Gly Thr Gly Val Arg Leu Val Met Gly Ala Met Ala Thr
                   85                  90                  95
   Ser Pro Leu Thr Ala Thr Phe Thr Gly Asp Ala Ser Leu Arg Lys Arg
               100                 105                 110
   Pro Met Gly Arg Val Thr Asp Pro Leu Ala Leu Phe Gly Thr Arg Ala
           115                 120                 125
   Tyr Gly Arg Lys Gly Gly Arg Leu Pro Met Thr Leu Val Gly Ala Ala
       130                 135                 140
   Asp Pro Val Pro Val Arg Tyr Thr Val Pro Val Pro Ser Ala Gln Val
   145                 150                 155                 160
   Lys Ser Ala Val Leu Leu Ala Gly Leu Asn Ala Pro Gly Gln Thr Val
                   165                 170                 175
   Val Ile Glu Arg Glu Ala Thr Arg Asp His Ser Glu Arg Met Leu Arg
               180                 185                 190
   Gly Phe Gly Ala Glu Leu Ser Val Glu Thr Gly Pro Glu Gly Gln Val
           195                 200                 205
   Ile Thr Leu Thr Gly Gln Pro Glu Leu Arg Pro Gln Thr Val Ala Val
               210                     215                     220
   Pro Arg Asp Pro Ser Ser Ala Ala Phe Pro Val Cys Ala Ala Leu Ile
   225                 230                 235                 240
   Val Glu Gly Ser Glu Ile Leu Val Pro Gly Val Ser Arg Asn Pro Thr
                   245                 250                 255
   Arg Asp Gly Leu Tyr Val Thr Leu Leu Glu Met Gly Ala Asp Ile Ala
               260                 265                 270
   Phe Glu Asn Glu Arg Glu Glu Gly Gly Glu Pro Val Ala Asp Leu Arg
           275                 280                 285
   Val Arg Ala Ser Ala Leu Lys Gly Val Glu Val Pro Pro Glu Arg Ala
       290                 295                 300
   Pro Ser Met Ile Asp Glu Tyr Pro Ile Leu Ser Val Val Ala Ala Phe
   305                 310                 315                 320
   Ala Glu Gly Leu Thr Ile Met Arg Gly Val Lys Glu Leu Arg Val Lys
                   325                 330                 335
   Glu Ser Asp Arg Ile Asp Ala Met Ala Arg Gly Leu Glu Ala Cys Gly
               340                 345                 350
   Val Arg Ile Glu Glu Asp Glu Asp Thr Leu Ile Val His Gly Met Gly
           355                 360                 365
   Arg Val Pro Gly Gly Ala Thr Cys Ala Thr His Leu Asp His Arg Ile
       370                 375                 380
   Ala Met Ser Phe Leu Val Leu Gly Met Ala Ala Glu Ala Pro Val Thr
   385                 390                 395                 400
   Val Asp Asp Gly Ser Pro Ile Ala Thr Ser Phe Pro Ala Phe Ile Asp
                   405                 410                 415
   Leu Met
<210> 47
   <211> 424
   <212> PRT
   <213> Chloroflexus aurantiacus
<400> 47
   Lys Arg Leu Arg Gly Val Ile Glu Val Pro Gly Asp Lys Ser Ile Ser
   1               5                   10                  15
   His Arg Ser Val Leu Phe Asn Ala Ile Ala Thr Gly Ser Ala His Ile
               20                  25                  30
   Thr His Phe Leu Pro Gly Ala Asp Cys Leu Ser Thr Val Ala Cys Ile
           35                  40                  45
   Arg Ala Leu Gly Val Thr Val Glu Gln Pro Ala Glu Arg Glu Leu Ile
       50                  55                  60
   Val His Gly Val Gly Leu Gly Gly Leu Arg Glu Pro Ala Asp Val Leu
   65                  70                  75                  80
   Asp Cys Gly Asn Ser Gly Thr Thr Leu Arg Leu Leu Ala Gly Leu Leu
                   85                  90                  95
   Ala Gly His Pro Phe Phe Ser Val Leu Thr Gly Asp Ala Ser Leu Arg
               100                 105                 110
   Ser Arg Pro Gln Arg Arg Ile Val Val Pro Leu Arg Ala Met Gly Ala
           115                 120                 125
   Gln Ile Asp Gly Arg Asp Asp Gly Asp Arg Ala Pro Leu Ala Ile Arg
       130                 135                 140
   Gly Asn Arg Leu Arg Gly Gly His Tyr Glu Leu Ser Ile Ala Ser Ala
   145                 150                 155                 160
   Gln Val Lys Ser Ala Leu Leu Leu Ala Ala Leu Asn Ala Glu Gln Pro
                   165                 170                 175
   Leu Thr Leu Thr Gly Arg Ile Asp Ser Arg Asp His Thr Glu Arg Met
               180                 185                 190
   Leu Ala Ala Met Gly Leu Glu Ile Thr Val Thr Ala Asp Gln Ile Thr
           195                 200                 205
   Ile Gln Pro Pro Ser Glu Ala Thr Ala Pro Thr Ala Leu Ser Leu Arg
       210                 215                 220
   Val Pro Gly Asp Pro Ser Ser Ala Ala Phe Trp Trp Val Ala Ala Ala
   225                 230                 235                 240
   Ile His Pro Asp Ala Glu Leu Val Thr Pro Gly Val Cys Leu Asn Pro
                   245                 250                 255
   Thr Arg Ile Gly Ala Ile Glu Val Leu Gln Ala Met Gly Ala Asp Leu
               260                 265                 270
   Thr Val Met Asn Glu Arg Leu Glu Gly Ser Glu Pro Val Gly Asp Val
           275                 280                 285
   Val Val Arg Ser Ser Ser Leu Arg Gly Thr Thr Ile Ala Gly Thr Leu
       290                 295                 300
   Ile Pro Arg Leu Ile Asp Glu Ile Pro Val Leu Ala Val Ala Ala Ala
   305                 310                 315                 320
   Cys Ala Ser Gly Glu Thr Val Ile Arg Asp Ala Gln Glu Leu Arg Ala
                   325                 330                 335
   Lys Glu Thr Asp Arg Ile Ala Thr Val Ala Ala Gly Leu Ser Ala Met
               340                 345                 350
   Gly Ala Val Val Glu Pro Thr Ala Asp Gly Met Val Ile Val Gly Gln
           355                 360                 365
   Pro Gly Gln Leu Gln Gly Thr Thr Leu Asn Ser Phe His Asp His Arg
       370                 375                 380
   Leu Ala Met Ala Trp Ala Ile Ala Ala Met Val Ala Arg Gly Glu Thr
   385                 390                 395                 400
   Thr Ile Leu Glu Pro Ala Ala Ala Ala Val Ser Tyr Pro Glu Phe Trp
                   405                 410                 415
   Gln Thr Leu Ala Met Val Gln Glu
               420
<210> 48
   <211> 424
   <212> PRT
   <213> Methanosarcina mazei
<400> 48
   Met Arg Val Ser Ile Asp Lys Ser Ser Ile Lys Gly Glu Val Phe Ala
    1               5                  10                  15
   Pro Pro Ser Lys Ser Tyr Thr His Arg Ala Val Thr Leu Ala Ala Leu
               20                  25                  30
   Ser Lys Glu Ser Thr Val Arg His Pro Leu Ile Ser Ala Asp Thr Leu
           35                  40                  45
   Ala Thr Val Arg Ala Ser Glu Met Phe Gly Ala Leu Val Glu Arg Glu
       50                  55                  60
   Glu Asp Arg Leu Ile Ile His Gly Ile Asn Gly Lys Pro Asn Val Pro
   65                  70                  75                  80
   Asp Asp Val Ile Asp Ala Ala Asn Ser Gly Thr Thr Leu Arg Phe Met
                   85                  90                  95
   Thr Ala Val Ala Ala Leu Thr Asp Gly Ile Thr Val Leu Thr Gly Asp
               100                 105                 110
   Ala Ser Leu Arg Thr Arg Pro Asn Gly Pro Leu Leu Glu Val Leu Asn
           115                 120                 125
   Arg Leu Gly Val Lys Ala Cys Ser Thr Arg Gly Asn Glu Arg Ala Pro
       130                 135                 140
   Leu Val Val Lys Gly Gly Leu Lys Gly Gln Asp Val Ser Ile Asp Gly
   145                 150                 155                 160
   Ser Ile Ser Ser Gln Phe Ile Ser Ala Leu Leu Ile Thr Cys Pro Leu
                   165                 170                 175
   Ala Glu Asn Ser Thr Ile Leu Ser Ile Thr Gly Lys Ile Lys Ser Arg
               180                 185                 190
   Pro Tyr Val Asp Ile Thr Leu Glu Met Leu Glu Leu Ala Gly Val Lys
           195                 200                 205
   Val His Ile Asp Asp Ser Asn Gly Thr Arg Phe Ile Ile Pro Gly Lys
       210                 215                 220
   Gln Lys Tyr Asp Phe Lys Asp Tyr Thr Val Pro Gly Asp Phe Ser Ser
   225                 230                 235                 240
   Ala Ser Tyr Leu Leu Ala Ala Ala Ala Met Thr Asp Gly Ser Glu Val
                   245                 250                 255
   Thr Val Lys Asn Leu Phe Pro Ser Lys Gln Gly Asp Lys Val Ile Ile
               260                 265                 270
   Glu Thr Leu Lys Gln Met Gly Ala Asp Ile Thr Trp Asp Lys Glu Ala
           275                 280                 285
   Gly Asn Val Thr Val Lys Gly Gly Arg Gln Leu Lys Ala Ile Thr Phe
       290                 295                 300
   Asp Ala Gly Ala Asn Pro Asp Leu Val Pro Thr Val Ala Val Leu Ala
   305                 310                 315                 320
   Ala Val Ala Lys Gly Thr Ser Arg Ile Glu Asn Ala Glu His Val Arg
                   325                 330                 335
   Tyr Lys Glu Thr Asp Arg Leu Arg Ala Leu Ala Thr Glu Leu Pro Lys
               340                 345                 350
   Leu Gly Val Asp Leu Lys Glu Glu Arg Asp Ser Leu Thr Ile Thr Gly
           355                 360                 365
   Gly Lys Leu His Gly Ala Ser Val His Gly Trp Asp Asp His Arg Ile
       370                 375                 380
   Val Met Ala Leu Ser Val Ala Gly Ile Val Ala Gly Asn Thr Lys Ile
   385                 390                 395                 400
   Asp Thr Thr Glu Ser Ala Ser Ile Ser Tyr Pro Glu Phe Phe Lys Asp
                   405                 410                 415
   Met Arg Ser Leu Gly Ala Lys Ile
               420
<210> 49
   <211> 439
   <212> PRT
   <213> Halobacterium sp. NRC-1
<400> 49
   Met Pro Trp Ala Ala Leu Leu Ala Gly Met His Ala Thr Val Ser Pro
   1 5 10 15
   Ser Arg Val Arg Gly Arg Ala Arg Ala Pro Pro Ser Lys Ser Tyr Thr
   20 25 30
   His Arg Ala Leu Leu Ala Ala Gly Tyr Ala Asp Gly Glu Thr Val Val
   35 40 45
   Arg Asp Pro Leu Val Ser Ala Asp Thr Arg Ala Thr Ala Arg Ala Val
   50 55 60
   Glu Leu Leu Gly Gly Ala Ala Ala Arg Glu Asn Gly Asp Trp Val Val
   65 70 75 80
   Thr Gly Phe Gly Ser Arg Pro Ala Ile Pro Asp Ala Val Ile Asp Cys
   85 90 95
   Ala Asn Ser Gly Thr Thr Met Arg Leu Val Thr Ala Ala Ala Ala Leu
   100 105 110
   Ala Asp Gly Thr Thr Val Leu Thr Gly Asp Glu Ser Leu Arg Ala Arg
   115 120 125
   Pro His Gly Pro Leu Leu Asp Ala Leu Ser Gly Leu Gly Gly Thr Ala
   130 135 140
   Arg Ser Thr Arg Gly Asn Gly Gln Ala Pro Leu Val Val Asp Gly Pro
   145 150 155 160
   Val Ser Gly Gly Ser Val Ala Leu Pro Gly Asp Val Ser Ser Gln Phe
   165 170 175
   Val Thr Ala Leu Leu Met Ala Gly Ala Val Thr Glu Thr Gly Ile Glu
   180 185 190
   Thr Asp Leu Thr Thr Glu Leu Lys Ser Ala Pro Tyr Val Asp Ile Thr
   195 200 205
   Leu Asp Val Leu Asp Ala Phe Gly Val Gly Ala Ser Glu Thr Ala Ala
   210 215 220
   Gly Tyr Arg Val Arg Gly Gly Gln Ala Tyr Ala Pro Ser Gly Ala Glu
   225 230 235 240
   Tyr Ala Val Pro Gly Asp Phe Ser Ser Ala Ser Tyr Leu Leu Ala Ala
   245 250 255
   Gly Ala Leu Ala Ala Ala Asp Gly Ala Ala Val Val Val Glu Gly Met
   260 265 270
   His Pro Ser Ala Gln Gly Asp Ala Ala Ile Val Asp Val Leu Glu Arg
   275 280 285
   Met Gly Ala Asp Ile Asp Trp Asp Thr Glu Ser Gly Val Ile Thr Val
   290 295 300
   Gln Arg Ser Glu Leu Ser Gly Val Glu Val Gly Val Ala Asp Thr Pro
   305 310 315 320
   Asp Leu Leu Pro Thr Ile Ala Val Leu Gly Ala Ala Ala Asp Gly Thr
   325 330 335
   Thr Arg Ile Thr Asp Ala Glu His Val Arg Tyr Lys Glu Thr Asp Arg
   340 345 350
   Val Ala Ala Met Ala Glu Ser Leu Ser Lys Leu Gly Ala Ser Val Glu
   355 360 365
   Glu Arg Pro Asp Glu Leu Val Val Arg Gly Gly Asp Thr Glu Leu Ser
   370 375 380
   Gly Ala Ser Val Asp Gly Arg Gly Asp His Arg Leu Val Met Ala Leu
   385 390 395 400
   Ala Val Ala Gly Leu Val Ala Asp Gly Glu Thr Thr Ile Ala Gly Ser
   405 410 415
   Glu His Val Asp Val Ser Phe Pro Asp Phe Phe Glu Val Leu Ala Gly
   420 425 430
   Leu Gly Ala Asp Thr Asp Gly
   435
<210> 50
   <211> 424
   <212> PRT
   <213> Synechococcus SP. WH 8102
<400> 50
   Gly Gly Ser Leu Ser Gly His Val Lys Val Pro Gly Asp Lys Ser Ile
   1               5                   10                  15
   Ser His Arg Ser Leu Leu Phe Gly Ala Ile Ala Glu Gly Thr Thr Thr
               20                  25                  30
   Ile Asp Gly Leu Leu Pro Ala Glu Asp Pro Ile Ser Thr Ala Ala Cys
           35                  40                  45
   Leu Arg Ala Met Gly Val Leu Ile Ser Pro Ile Glu Ala Ala Gly Leu
       50                  55                  60
   Val Thr Val Glu Gly Val Gly Leu Asp Gly Leu Gln Glu Pro Ala Glu
   65                  70                  75                  80
   Ile Leu Asp Cys Gly Asn Ser Gly Thr Thr Met Arg Leu Met Leu Gly
                   85                  90                  95
   Leu Leu Ala Gly Arg Ala Gly Arg His Phe Val Leu Asp Gly Asp Ala
               100                 105                 110
   Ser Leu Arg Arg Arg Pro Met Arg Arg Val Gly Gln Pro Leu Ala Ser
           115                 120                 125
   Met Gly Ala Asp Val Arg Gly Arg Asp Gly Gly Asn Leu Ala Pro Leu
       130                 135                 140
   Ala Val Gln Gly Gln Ser Leu Arg Gly Thr Val Ile Gly Thr Pro Val
   145                 150                 155                 160
   Ala Ser Ala Gln Val Lys Ser Ala Leu Leu Leu Ala Ala Leu Thr Ala
                   165                 170                 175
   Asp Gly Thr Thr Thr Val Ile Glu Pro Ala Gln Ser Arg Asp His Ser
               180                 185                 190
   Glu Arg Met Leu Arg Ala Phe Gly Ala Asp Leu Gln Val Gly Gly Glu
           195                 200                 205
   Met Gly Arg His Ile Thr Val Arg Pro Gly Asn Thr Leu Lys Gly Gln
       210                 215                 220
   Gln Val Val Val Pro Gly Asp Ile Ser Ser Ala Ala Phe Trp Leu Val
   225                 230                 235                 240
   Ala Gly Ala Leu Val Pro Gly Ala Asp Leu Thr Ile Glu Asn Val Gly
                   245                 250                 255
   Leu Asn Pro Thr Arg Thr Gly Ile Leu Glu Val Leu Glu Gln Met Asn
               260                 265                 270
   Ala Gln Ile Glu Val Leu Asn Arg Arg Asp Val Ala Gly Glu Pro Val
           275                 280                 285
   Gly Asp Leu Arg Ile Thr His Gly Pro Leu Gln Pro Phe Ser Ile Gly
       290                 295                 300
   Glu Glu Ile Met Pro Arg Leu Val Asp Glu Val Pro Ile Leu Ser Val
   305                 310                 315                 320
   Ala Ala Cys Phe Cys Asp Gly Glu Ser Arg Ile Ser Gly Ala Ser Glu
                   325                 330                 335
   Leu Arg Val Lys Glu Thr Asp Arg Leu Ala Val Met Ala Arg Gln Leu
               340                 345                 350
   Lys Ala Met Gly Ala Glu Ile Glu Glu His Glu Asp Gly Met Thr Ile
           355                 360                 365
   His Gly Gly Arg Pro Leu Lys Gly Ala Ala Leu Asp Ser Glu Thr Asp
       370                 375                 380
   His Arg Val Ala Met Ser Leu Ala Val Ala Ser Leu Leu Ala Ser Gly
   385                 390                 395                 400
   Asp Ser Thr Leu Gln Arg Ser Asp Ala Ala Ala Val Ser Tyr Pro Ser
                   405                 410                 415
   Phe Trp Asp Asp Leu Asp Arg Leu
               420
<210> 51
   <211> 322
   <212> PRT
   <213> Archaeoglobus fulgidus
<400> 51
   Met Ala Glu Phe Pro Lys Val Arg Met Arg Arg Leu Arg Lys Ala Asn
    1               5                  10                  15
   Leu Arg Trp Met Phe Arg Glu Ala Arg Leu Ser Pro Glu Asn Leu Ile
               20                  25                  30
   Thr Pro Ile Phe Val Asp Glu Asn Ile Lys Glu Lys Lys Pro Ile Glu
           35                  40                  45
   Ser Met Pro Asp Tyr Phe Arg Ile Pro Leu Glu Met Val Asp Lys Glu
       50                  55                  60
   Val Glu Glu Cys Leu Glu Lys Asp Leu Arg Ser Phe Ile Leu Phe Gly
   65                  70                  75                  80
   Ile Pro Ser Tyr Lys Asp Glu Thr Gly Ser Ser Ala Tyr Asp Gln Asn
                   85                  90                  95
   Gly Val Ile Gln Lys Ala Val Arg Arg Ile Lys Ala Glu Phe Pro Asp
               100                 105                 110
   Ala Val Ile Val Thr Asp Val Cys Leu Cys Glu Tyr Thr Thr His Gly
           115                 120                 125
   His Cys Gly Val Val Lys Asp Gly Glu Ile Val Asn Asp Glu Thr Leu
       130                 135                 140
   Pro Ile Ile Gly Lys Thr Ala Val Ser His Ala Glu Ser Gly Ala Asp
   145                 150                 155                 160
   Ile Val Ala Pro Ser Gly Met Met Asp Gly Met Val Lys Ala Ile Arg
                   165                 170                 175
   Glu Ala Leu Asp Ala Ala Gly Phe Glu Ser Thr Pro Ile Met Ser Tyr
               180                 185                 190
   Ser Ala Lys Tyr Ala Ser Asn Phe Tyr Ser Pro Phe Arg Asp Ala Ala
           195                 200                 205
   Glu Ser Gly Phe Lys Phe Gly Asp Arg Arg Gly Tyr Gln Met Asp Ile
       210                 215                 220
   His Asn Ala Arg Glu Ala Met Arg Glu Ile Glu Leu Asp Val Lys Glu
   225                 230                 235                 240
   Gly Ala Asp Ile Ile Met Val Lys Pro Ala Leu Pro Tyr Leu Asp Ile
                   245                 250                 255
   Ile Arg Met Val Arg Glu Arg Phe Asp Leu Pro Leu Ala Ala Tyr Asn
               260                 265                 270
   Val Ser Gly Glu Tyr Ser Met Ile Lys Ala Ala Ile Lys Asn Gly Trp
           275                 280                 285
   Leu Ser Glu Glu Ala Ile Tyr Glu Val Leu Ile Ser Ile Lys Arg Ala
       290                 295                 300
   Gly Ala Asp Leu Ile Ile Thr Tyr His Ser Lys Glu Ile Ala Glu Lys
   305                 310                 315                 320
   Leu Gln
<210> 52
   <211> 410
   <212> PRT
   <213> Pyrococcus abyssi
<400> 52
   Met Phe Gly Pro Val Ser Val Glu Met Ile Ile Glu Arg Val Asp Glu
    1               5                  10                  15
   Val Arg Gly Lys Val Lys Ala Pro Pro Ser Lys Ser Tyr Thr His Arg
               20                  25                  30
   Ala Tyr Phe Leu Ser Leu Leu Ala Asp Ser Pro Ser Lys Val Met Asn
           35                      40                      45
   Pro Leu Ile Ser Glu Asp Thr Ile Ala Ser Leu Asp Ala Ile Ser Lys
       50                      55                      60
   Phe Gly Ala Gln Val Asn Gly Asn Lys Ile Ile Pro Pro Gln Glu Leu
   65                  70                      75                  80
   Thr Pro Gly Lys Ile Asp Ala Arg Glu Ser Gly Thr Thr Ala Arg Ile
                   85                      90                  95
   Ser Leu Ala Val Ala Ser Leu Ala Arg Gly Thr Ser Val Ile Thr Gly
               100                     105                 110
   Lys Gly Arg Leu Val Glu Arg Pro Phe Lys Pro Leu Val Asp Ala Leu
           115                     120                 125
   Arg Ser Leu Lys Val Lys Ile Ser Gly Glu Lys Leu Pro Ile Ala Val
       130                     135                 140
   Glu Gly Gly Asn Pro Val Gly Glu Tyr Val Lys Val Asp Cys Ser Leu
   145                 150                     155                 160
   Ser Ser Gln Phe Gly Thr Ala Met Leu Ile Leu Ala Ser Lys Ile Gly
                   165                     170                 175
   Leu Thr Val Glu Met Leu Asn Pro Val Ser Arg Pro Tyr Ile Glu Val
               180                     185                 190
   Thr Leu Lys Val Met Glu Ser Phe Gly Ile Glu Phe Glu Arg Asn Gly
           195                     200                 205
   Phe Lys Val Lys Val His Pro Gly Ile Arg Gly Ser Lys Phe His Val
       210                     215                 220
   Pro Gly Asp Tyr Ser Ser Ala Ser Phe Phe Leu Ala Ala Gly Ala Leu
   225                 230                     235                 240
   Tyr Gly Lys Val Lys Val Ser Asn Leu Val Lys Asp Asp Pro Gln Ala
                   245                     250                 255
   Asp Ala Arg Ile Ile Asp Ile Leu Glu Glu Phe Gly Ala Asp Val Lys
               260                     265                 270
   Val Gly Arg Lys Tyr Val Val Val Glu Arg Asn Glu Met Lys Pro Ile
           275                     280                 285
   Asn Val Asp Cys Ser Asn Phe Pro Asp Leu Phe Pro Ile Leu Ala Val
       290                     295                 300
   Leu Ala Ser Tyr Ala Glu Gly Lys Ser Val Ile Thr Gly Arg Gln Leu
   305                 310                     315                 320
   Arg Leu Lys Glu Ser Asp Arg Val Lys Ala Val Ala Val Asn Leu Arg
                   325                     330                 335
   Lys Ala Gly Ile Lys Val Lys Glu Leu Pro Asn Gly Leu Glu Ile Val
               340                     345                 350
   Gly Gly Lys Pro Arg Gly Phe Thr Val Glu Ser Phe Asn Asp His Arg
           355                     360                 365
   Ile Val Met Ala Met Ala Ile Leu Gly Leu Gly Ala Glu Gly Lys Thr
       370                     375                 380
   Ile Ile Lys Asp Pro His Val Val Ser Lys Ser Tyr Pro Ser Phe Phe
   385                 390                     395                 400
   Leu Asp Leu Arg Arg Val Leu Asn Glu Gly
                   405                     410